# EUROPEAN PATENT SPECIFICATION

(11) **EP 1 517 894 B1**
(45) Date of publication and mention of the grant of the patent: **06.09.2006**
(21) Application number: 03740413.4
(22) Date of filing: 02.07.2003
(51) Int. Cl.: C07D 211/14, C07D 211/38, C07D 207/20, C07D 211/70, C07D 207/06, C07D 265/30, C07D 213/16, A61K 31/40, A61K 31/445, A61K 31/44, A61K 31/535, A61P 25/00, A61P 29/00, A61P 1/08

(54) **N-BENZYL-3-PHENYL-3-HETEROCYCLYL-PROPIONAMIDE COMPOUNDS AS TACHYKININ ANTAGONISTS AND/OR SEROTONIN REUPTAKE INHIBITORS**
N-BENZYL-3-PHENYL-3-HETEROCYCLYL-PROPIONAMID-VERBINDUNGEN ALS TACHYKININ REZEPTOREN ANTAGONISTEN UND/ODER ALS SEROTONINWIEDERAUFNAHME INHIBITOREN
COMPOSES DE N-BENZYL-3-PHENYL-3-HETEROCYCLYL-PROPIONAMIDE EN TANT QU'INHIBITEURS DE RECAPTAGE DE LA TACHYKININE ET/OU DE LA SEROTONINE

(30) Priority: 03.07.2002 GB 0215392
(43) Date of publication of application: 30.03.2005
(73) Proprietor: GLAXO GROUP LIMITED, Greenford, Middlesex UB6 0NN (GB)
(72) Inventor: ALVARO, Giuseppe, GlaxoSmithKline, I-37135 Verona (IT); CARDULLO, Francesca, GlaxoSmithKline, I-37135 Verona (IT); D'ADAMO, Lucilla, GlaxoSmithKline, I-37135 Verona (IT); PIGA, Elisabetta, GlaxoSmithKline, I-37135 Verona (IT); SERI, Catia, GlaxoSmithKline, I-37135 Verona (IT)
(74) Representative: Giddings, Peter John
(86) International application number: PCT/EP2003/007126
(87) International publication number: WO 2004/005255

(56) References cited:
- WO-A-93/01169
- WO-A-94/04494
- JP-A- 4 089 478
- MULLER G W ET AL: "STRUCTURAL MODIFICATIONS OF THALIDOMIDE PRODUCE ANALOGS WITH ENHANCED TUMOR NECROSIS FACTOR INHIBITORY ACTIVITY" JOURNAL OF MEDICINAL CHEMISTRY, AMERICAN CHEMICAL SOCIETY. WASHINGTON, US, vol. 39, no. 17, 16 August 1996 (1996-08-16), pages 3238-3240, XP002064438 ISSN: 0022-2623
- MARK D.E.: J.MED.CHEMISTRY, vol. 36, no. 24, 1993, pages 3771-3783, XP002255002

## Description

The present invention relates to heterocyclic derivatives, to processes for their preparation, to pharmaceutical compositions containing them and to their medical use.

WO 94/04494 discloses aromatic compounds of formula (I) having activity as NK₁ receptor antagonists of general formula (A) 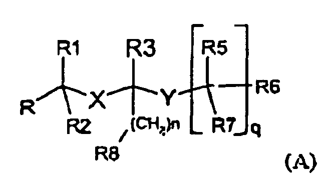 wherein R, R₈ and R₆ are inter alia optionally substituted phenyl, pyridine, thiophene, furan, naphthalene; R₁ and R₂ are each independently hydrogen or C₁₋₄alkyl; R and R₂, when joined by a bond, can form a ring; X is -OC(O)NR¹¹ wherein R¹¹ is hydrogen or C₁₋₃ alkyl;; R₃ is hydrogen or (CH₂)ₘR¹³ where m is an integer of from 1 to 6 and R¹³ is H, CN, NH₂. N(CH₃)₂, or NHCOCH₃ : n is an integer of from 1 to 2; Y is -C(O)NR⁴ wherein R⁴ is inter alia hydrogen or C₁₋₃alkyl, -CO₂-: R₅ and R₇ are each independently hydrogen or C₁₋₄alkyl; q is 0 or 1.

WO 93/01169 discloses tachykinin antagonists of general formula (B) 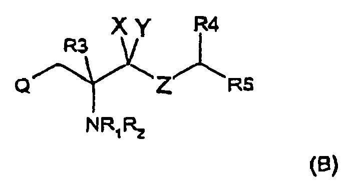 and salts and prodrugs thereof, wherein Q represents optionally substituted phenyl, naphthyl, indolyl, benzothiophenyl, benzofuranyl, benzyl or indazolyl; Z represents O, S or NR₈; X and Y are H or are together = O; R₁ and R₂ are H; optionally substituted C₁. ₆alkyl; optionally substituted phenyl(C₁₋₄alkyl), COR^{c}; CO₂R^{c}; CONR^{c}R^{d}; CONR^{c}COOR^{d}; or SO₂R^{c}: R₃ is H or C₁₋₆alkyl, R₄ is H, C₁₋₆alkyl or optionally substituted phenyl; and R₅ represents optionally substituted phenyl.

Neither compounds of formula A nor compounds of formula B disclose nor suggest a compound of formula (I) according to the present invention.

The present invention thus provides compounds of formula (I) 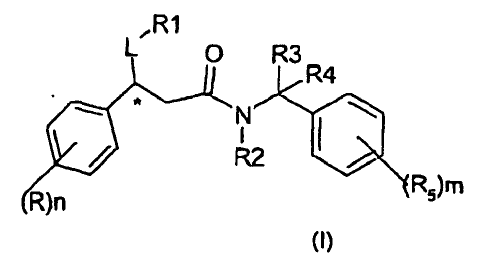 wherein
R represents halogen, C₁₋₄ alkyl, cyano, C₁₋₄ alkoxy, trifluoromethyl or trifluoromethoxy;
R₁ represents a 5 or 6 membered heteroaryl group, in which the 5-membered heteroaryl group contains at least one heteroatom selected from oxygen, sulphur or nitrogen and the 6-membered heteroaryl group contains from 1 to 3 nitrogen atoms, or R₁ represents a 4, 5 or 6 membered heterocyclic group, wherein saids 5 or 6 membered heteroaryl or the 4, 5 or 6 membered heterocyclic group may optionally be substituted by one to three substituents, which may be the same or different, selected from (CH₂)ₚR₆. wherein p is zero or an integer from 1 to 4 and R₆ is selected from:
   halogen,
   C₁₋₄alkoxy,
   C₁₋₄alkyl,
   C₃₋₇cycloalkyl,
   C₁₋₄ alkyl optionally substituted by halogen, cyano or C₁₋₄ alkoxy,
   hydroxy,
   cyano,
   nitro,
   trifluoromethyl,
   carboxy,
   NH(C₁₋₄ alkyl),
   N(C ₁₋₄ alkyl)₂
   NH(C₃₋₇ cycloalkyl),
   N(C ₁₋₄ alkyl)(C₃₋₇ cycloalkyl);
   NH(C₁₋₄alkylOC₁₋₄alkoxy),
   OC(O)NR₇R₈,
   NR₈C(O) R₇ or
   C(O)NR₇R₈;
R₂ represents hydrogen, or C₁₋₄ alkyl ;
R₃ and R₄ independently represent hydrogen, C₁₋₄ alkyl or R₃ together with R₄ represents C₃₋₇ cycloalkyl;
R₅ represents trifluoromethyl, S(O)_{q}C ₁₋₄ alkyl, C₁₋₄ alkyl, C₁₋₄ alkoxy, trifluoromethoxy, halogen or cyano;
R₇ and R₈ independently represent hydrogen, C₁₋₄ alkyl or C₃₋₇ cycloalkyl;
L is a single or a double bond;
n is an integer from 1 to 3;
m is zero or an integer from 1 to 3;
q is zero or an integer from 1 to 2;
provided that
   a) when L is a double bond, R₁ is not an optionally substituted 5 or 6 membered heteroaryl group, in which the 5-membered heteroaryl group contains at least one heteroatom selected from oxygen, sulphur or nitrogen and the 6-membered heteroaryl group contains from 1 to 3 nitrogen atoms;
   b) the group R₁ is linked to the carbon atom shown as * via a carbon atom;
      and
   c) when the heteroatom contained in the group R₁ is substituted, p is not zero;
and pharmaceutically acceptable salts and solvates thereof.

Suitable pharmaceutically acceptable salts of the compounds of general formula (I) include acid addition salts formed with pharmaceutically acceptable organic or inorganic acids, for example hydrochlorides, hydrobromides, sulphates, alkyl- or arylsulphonates (e.g. methanesulphonates or p-toluenesulphonates), phosphates, trifluoroacetates, acetates, citrates, succinates, tartrates,malates, lactates, fumarates and maleates.

The solvates may, for example, be hydrates.

References hereinafter to a compound according to the invention include both compounds of formula (I) and their pharmaceutically acceptable acid addition salts and their pharmaceutically acceptable solvates.

It is to be understood that when L is a single bond , the compounds of the invention have the general structure (IA) and when L is a double bond the compounds of the invention have the general structure (IB)

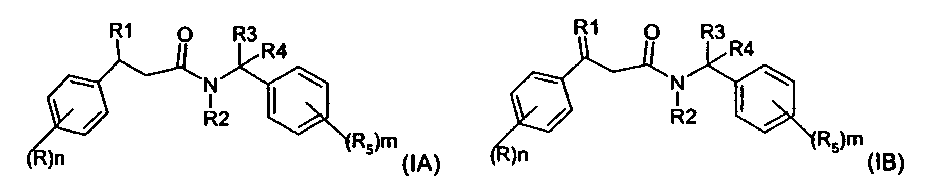

It will be appreciated by those skilled in the art that the compounds of formula (IA) contain at least one chiral centre (namely the carbon atom shown as * in formula (I)) and may be represented by formula (1 a) and (1b). 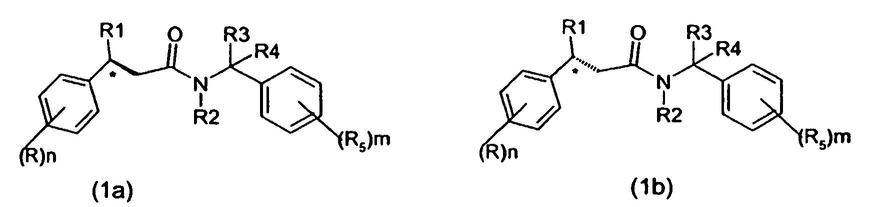 The wedge bond indicates that the bond is above the plane of the paper. The broken bond indicates that the bond is below the plane of the paper.
The configuration shown for the chiral carbon indicated as * in formula (1a) is β and in formula(1b) is α.

Further asymmetric carbon atoms are possible in the compounds of formula (I) when R₃ and R₄ are not the same group.

It is to be understood that all stereoisomeric forms including all enantiomers and mixtures thereof are encompassed within the scope of the present invention and the reference to compound of formula (I) include all stereisomeric forms unless otherwise stated.

It will be appreciated by those skilled in the art that the compounds of formula (IB) can exist in E and/or Z conformation and the invention includes all such isomers and mixtures thereof.

The term C₁₋₄ alkyl as used herein as a group or a part of the group refers to a straight or branched alkyl group containing from 1 to 4 carbon atoms; examples of such groups include methyl, ethyl, propyl, isopropyl, n-butyl, isobutyl, tert-butyl, 1 methylethyl or 2-methyl propyl.

The term halogen refers to fluorine, chlorine, bromine or iodine.

The term C₃₋₇ cycloalkyl group means a non aromatic monocyclic hydrocarbon ring of 3 to 7 carbon atom such as, for example, cyclopropyl, cyclobutyl, cyclopentyl, cyclohexyl or cycloheptyl.

When R₁ is a 5 or 6 membered heteroaryl group according to the invention this includes furanyl, thiophenyl, pyrrolyl, imidazolyl, thiazolyl, oxazolyl, pyrazolyl, isoxazolyl, isothiazolyl, 1,2,3-triazolyl, 1,2,3-oxadiazolyl, 1,2,3-thiadiazolyl, 1,2,4-triazolyl, 1,3,4-oxadiazolyl, 1,3,4-thiadiazolyl, 1,2,5-oxadiazolyl, 1,2,5-thiadiazolyl, 1,2,3,6-tetrahydro-4-pyridinyl, pyridyl, pyridazinyl, pyrimidinyl, pyrazinyl, 1,2,4 oxadiazolyl, 1,2,5-triazinyl or 1,3,5-triazinyl and the like.

The term 4, 5 or 6 membered heterocyclic group refers to 4, 5 or 6 ring member, containing at least one heteroatom selected from oxygen, sulphur or nitrogen, which may be saturated or partially unsaturated. Examples of such groups include azetidinyl, piperidyl, 2-oxodihydrofuranyl, piperazinyl-1,2,3,6-tetrahydro-4-pyridinyl, morpholinyl, pyrazolidinyl, 1,2 dihydro-3H-pyrazolyl, imidazolidinyl or pyrrolidinyl and the like.

The term C₁₋₄ alkoxy group may be a straight chain or a branched chain alkoxy group, for example methoxy, ethoxy, propoxy, prop-2-oxy, butoxy, but-2-oxy or methylprop-2-oxy.

R is preferably halogen (e.g. fluorine or chlorine) and/or a C₁₋₄ alkyl (e.g. methyl) group and n is preferably an integer from 1 to 2.

R₁ is preferably piperidyl, morpholinyl, 1,2,3,6-tetrahydro-4-pyridinyl, pyridyl or pyrrolidinyl.

When R₁ is substituted, preferably is substitued by one or two groups selected from halogen (e.g fluorine). C₁₋₄ alkyl (e.g. methyl) or ethyl C₁₋₄ alkoxy.

R₂ is preferably hydrogen or methyl.

R₃ is preferably hydrogen or methyl.

R₄ is preferably hydrogen, methyl or together with R₃ is cyclopropyl.

R₅ is preferably trifluoromethyl, methyl, methoxy, bromine, chlorine or fluorine atom and m is preferably an integer from 1 to 2.

Preferred compounds of the invention are those wherein R is halogen (e.g. fluorine or chlorine) and/or a C₁₋₄ alkyl (e.g. methyl) group and n is an integer from 1 to 2; R₁ is piperidyl, 2-morpholinyl, 1,2,3.6-tetrahydro-4-pyridinyl, pyridyl or pyrrolidinyl and wherein R₁ is optionally substituted by one or two groups selected from halogen (e.g fluorine), C₁₋₄ alkyl (e.g. methyl) or ethylC₁₋₄ alkoxy; R₂ and R₃ are independently hydrogen or methyl; R₄ is hydrogen, methyl or together with R₃ is cyclopropyl and R₅ is trifluoromethyl, methyl, methoxy, bromine, chlorine or fluorine atom and q is preferably an integer from 1 to 2.

Specific preferred compounds according to the invention are:
*N*-(3,5-Bis-trifluoromethyl-benzyl)-3-(4-fluoro-phenyl)-*N*-methyl-3-piperidin-4-yl-propionamide;
*N*-(3,5-Dichloro-benzyl)-3-(4-fluoro-phenyl)-*N*-methyl-3-piperidin-4-yl-propionamide;
*N*-[1-(3,5-Dichloro-phenyl)-ethyl]-3-(4-fluoro-phenyl)-*N*-methyl-3-piperidin-4-yl-propionamide;
*N*-[1-(3,5-Dichloro-phenyl)-ethyl]-3-(4-fluoro-phenyl)-*N*-methyl-3-[1-(2-methoxyethyl)-piperidin-4-yl]-propionamide;
*N*-(3,5-Dichloro-benzyl)-3-(4-fluoro-phenyl)-3-(4-fluoro-piperidin-4-yl)-*N*-methylpropionamide;
*N*-{[3,5-bis(trifluoromethyl)phenyl]methyl}-3-(4-fluorophenyl)-*N*-methyl-3-{1-[2-(methyloxy)ethyl]-4-piperidinyl}propionamide; *N*-{-1-[3,5-bis(trifluoromethyl)phenyl]ethyl}-3-(4-fluorophenyl)-*N*-methyl-3-(4-piperidinyl)propionamide;*N*-{1-[3,5-bis(trifluoromethyl)phenyl]-1-methylethyl}-3-(4-fluorophenyl)-3-(4-piperidinyl)propionamide;
*N*-{[3-bromo-4-(methyloxy)phenyl]methyl}-3-(4-fluorophenyl)-*N*-methyl-3-(4-piperidinyl)propionamide;
*N*-[(3,5-dimethylphenyl)methyl]-3-(4-fluorophenyl)-*N*-methyl-3-(4-piperidinyl)propionamide;
*N*-[(3,4-dibromophenyl)methyl]-3-(4-fluorophenyl)-*N*-methyl-3-(4-piperidinyl)propionamide;
*N*-[(3-fluoro-2-methylphenyl)methyl]-3-(4-fluorophenyl)-*N*-methyl-3-(4-piperidinyl)propionamide;
*N*-{[2-chloro-3-(trifluoromethyl)phenyl]methyl}-3-(4-fluorophenyl)-*N*-methyl-3-(4-piperidinyl)propionamide;
*N*-(-1-[3,5-bis(trifluoromethyl)phenyl]ethyl}-3-(4-fluorophenyl)-3-(4-fluoro-4-piperidinyl)-*N-*methylpropionamide;
*N*-[(3,5-dibromophenyl)methyl]-3-(4-fluorophenyl)-3-(4-fluoro-4-piperidinyl)-*N-*methylpropionamide;
*N*-{-1-[3,5-bis(trifluoromethyl)phenyl]ethyl}-3-(2,4-dichlorophenyl)-3-(4-fluoro-4-piperidinyl)-*N*-methylpropionamide;
*N*-{-1-[3,5-bis(trifluoromethyl)phenyl]ethyl}-3-(4-fluoro-2-methylphenyl)-3-(4-fluoro-4-piperidinyl)-*N*-methylpropionamide;
*N*-[(3,5-dibromophenyl)methyl]-3-(4-fluoro-2-methylphenyl)-3-(4-fluoro-4-piperidinyl)-*N-*methylpropionamide;
*N*-[(3,5-dibromophenyl)methyl]-3-(3,4-dichlorophenyl)-3-(4-fluoro-4-piperidinyl)-*N-*methylpropionamide;
*N*-{[3,5-bis(trifluoromethyl)phenyl]methyl}-3-(4-fluorophenyl)-3-(4-fluoro-4-piperidinyl)-*N-*methylpropionamide;
3-(4-chlorophenyl)-*N*-[(3,5-dibromophenyl)methyl]-3-(4-fluoro-4-piperidinyl)-*N-*methylpropionamide;
*N*-{[3,5-bis(trifluoromethyl)phenyl]methyl}-3-(4-fluorophenyl)-*N*-methyl-3-(3-piperidinylidene)propionamide;
*N*-[(3,5-dibromophenyl)methyl]-3-(4-fluorophenyl)-*N*-methyl-3-(4-piperidinylidene)propionamide;
*N*-{[3,5-bis(trifluoromethyl)phenyl]methyl}-3-(4-fluoro-2-methylphenyl)-*N*-methyl-3-(1,2,3,6-tetrahydro-4-pyridinyl)propionamide;
*N*-{(1*R*)-1-[3,5-bis(trifluoromethyl)phenyl]ethyl}-3-(4-fluoro-2-methylphenyl)-*N*-methyl-3-(1,2,3,6-tetrahydro-4-pyridinyl)propionamide;
*N*-{[3,5-bis(trifluoromethyl)phenyl]methyl}-3-(4-fluorophenyl)-*N*-methyl-3-(3-pyrrolidinyl)propionamide;
*N*-{[3,5-bis(trifluoromethyl)phenyl]methyl}-3-(4-fluorophenyl)-3-(3-fluoro-3-piperidinyl)-*N-*methylpropionamide;
*N*-{-1-[3,5-bis(trifluoromethyl)phenyl]ethyl}-3-(4-fluorophenyl)-*N*-methyl-3-(2-morpholinyl)propionamide;
*N*-{[3,5-bis(trifluoromethyl)phenyl]methyl}-3-(4-fluorophenyl)-*N*-methyl-3-(3-piperidinyl)propionamide;
*N*-{[3,5-bis(trinuoromethyl)phenyl]methyl}-3-(4-fluorophenyl)-*N*-methyl-3-(4-pyridinyl)propionamide;
and enantiomers, diastereiosomers, pharmaceutically acceptable salts(e.g hydrochloride) and solvates thereof.

Particular specific preferred compounds according to the invention are:
*N*-{(1*R*)1-[3,5-bis(trifluoromethyl)phenyl]ethyl}-3-(4-fluorophenyl)-*N*-methyl-3-(4-piperidinyl)propionamide(diastereoisamer 1);
*N*-{(1*S*}-1-[3,5-bis(trifluoromethyl)phenyl]ethyl}-3-(4-fluorophenyl)-*N*-methyl-3-(4-piperidinyl)propionamide (diastereoisomer 2);
*N*-{(1*R*}-1-[3,5-bis(trifluoromethyl)phenyl]ethyl}-3-(4-fluorophenyl)-3-(4-fluoro-4-piperidinyl)-*N*-methylpropionamide (diastereoisomer 1;
*N*-[(3,5-dibromophenyl)methyl]-3-(4-fluorophenyl)-3-(4-fluoro-4-piperidinyl)-*N-*methylpropionamide (enantiomer 2);
*N*-{[3,5-bis(trifluoromethyl)phenyl]methyl}-3-(4-fluorophenyl)-3-(3-fluoro-3-piperidinyl}-*N-*methylpropionamide (diastereoisomer A);
and pharmaceutically acceptable salts(e.g hydrochloride) and solvates thereof.

The compounds of the invention are antagonists of tachykinin receptors, including substance P and other neurokinins, both in vitro and in vivo and are thus of use in the treatment of conditions mediated by tachykinins, including substance P and other neurokinins.

Tachykinins are a family of peptides that share a common carboxyl-terminal sequence (Phe-X-Gly-Leu-Met-NH2). They are actively involved in the physiology of both lower and advanced lifeforms. In mammalian lifeforms the main tachykinins are subtance P (SP), Neurokinin A (NKA) and Neurokinin B (NKB) which act as neurotransmitters and neuromodulators. Mammalian tachykinins may contribute to the pathophysiology of a number of human diseases.
Three types of tachykinins receptors have been identified, namely NK1(SP-preferring), NK2 (NKA-preferring) and NK3 (NKB-preferring) which are widely distributed throughout the central nervous (CNS) and peripheral nervous system.

Particularly the compounds of the invention are antagonists of the NK1 receptor.

The compounds of the present invention also have activity as selective serotonin reuptake inhibitors (hereinafter referred to as SSRIs) and are thus of use in the treatment of conditions mediated by selective inhibition of the serotonin reuptake transporter protein.

Thus the compounds of the present invention combine dual activity as tachykinin antagonists, including substance P and other neurokinins, and as SSRIs. In particular, the compounds of the invention combine dual activity as NK1 receptor antagonists and as SSRIs.

NK₁-receptor binding affinity has been determined in vitro by measuring the compounds' ability to displace [3H] - Substance P (SP) from recombinant human NK₁ receptors expressed in Chinese Hamster Ovary (CHO) cell membranes.
CHO cell membranes were prepared by using a modification of the method described by Beattie D.T. et al. (Br. J. Pharmacol, 116:3149-3157, 1995). Briefly, ligand binding was performed in 0.2 ml of 50 mM HEPES, pH 7.4, containing 3 mM MnCl₂, 0.02% BSA, 0.5 nM [³H]-Substance P (30÷56 Ci/mmol, Amersham), a final membrane concentration of 20 ÷30 µg of protein/ml, and the test compounds. The incubation proceeded at room temperature for 40 min and stopped by filtration. Non-specific binding was determined using excess of substance P (1 µM) and represents about 6÷10% of the total binding.

NK₁-receptor binding affinity has been also determined in vitro in a binding Scintillation proximity assay (SPA) by measuring compounds' ability to displace [1251]TyrB-Substance P (SP) from recombinant human NK₁ receptors expressed in Chinese Hamster Ovary (CHO) cell membrane prepared as described above. Briefly, polystrene Leadseeker WGA-SPA beads (Amersham Biosciences) was mixed with cell membrane in a bead/membrane ratio of 100:1 (w/w) in assay buffer (75 mM Tris pH 7.8, 75 mM NaCl, 4 mM MnCl2, 1 mM EDTA, 0.05% Chaps, 1 mM PMSF). The mixture was placed on ice for 30 minutes to allow the formation of membrane/bead complex before BSA was added to a final concentration of 1%. After another 30 minutes incubation on ice, the bead/membrane complex was washed twice and suspended in assay buffer. [125I]Tyr8-Substance P (2200 Ci/mmol, PerkinElmer) was then added to the bead/membrane complex with a final concentration of 0.4 nM. 30 ul of the resulting mixture was then dispensed to each well of Nalgen NUNC 384-well plate with 1 ul compound pre-dispensed in DMSO. The plates were then sealed and pulse spin at 1100 rpm. After 3 hours incubation at room temperature with shaking, the plates were spin for 2 min at 1100 rpm and measured in Viewlux imager (PerkinElmer) for 5 minutes with a 618-nm filter. Inhibition of [1251]Tyr8-Substance P binding to NK₁-receptor was measured by the reduction of luminescent signal. IC50 of each compound was determined by an 11-point 3x-dilution inhibition curve. pKi was calculated using Kd of [1251]Tyr8-Substance P determined in a separate experiment.

Compounds of the invention were further characterised in a functional assay for the determination of their effect to inhibit the intracellular calcium increase induced by SP in Human-NK₁-CHO cells using FLIPR technology. Briefly, after 30min incubation with the cytoplasmic calcium indicator Fluo-4 AM (2µM), cells were washed and incubated in the absence or presence of three different concentrations of antagonist for 60min, at 37°C in Hank's balanced salts with 20mM Hepes and then non-cumulative concentration-response curves of SP (2pM-300nM) was performed. The potency of the antagonist (pK_{B} value) was calculated from Schild's analysis.

The ability to bind at the serotonin transporter may be determined using one of the following conventional binding or uptake assays.

The inhibitory activity of the compounds at the human Serotonin Transporter(hSERT) has been determined in vitro using hSERT-LLCPK cells (LLCPK cells tranfected with the h SERT). The cells have been plated onto 96-well plates (10000 cells/well). After 24 hr, cells have been washed in uptake buffer (Hank's balanced salt solution + 20 mM Hepes) and pre-incubated for 10 min at RT with 50 µl of buffer containing the test compounds. 50 µl of 50 nM [3H] Serotonin (5HT) solution (final concentration: 25 nM [3H] 5HT) have been added and plates have been incubated for 7 min at RT, during which cells take up radiolabelled 5HT. Aspirating the solution and rapidly washing the cells with cold buffer has terminated the uptake.
The amount of radioactive 5HT incorporated in the cells has been then measured by adding the scintillation cocktail directly onto the cells and reading the plate in the Top Count. The data have been digitally processed to obtain the plC50 values of the antagonists.

The inhibitory activity of the compounds at the rat Serotonin Transporter(rSERT) has been determined in vitro using rSERT-LLCPK cells (LLCPK cells tranfected with the rat SERT). The cells have been plated onto 96-well plates (60000 cells/well). After 24 hr, cells have been washed in uptake buffer (Hank's balanced salt solution + 20 mM Hepes) and pre-incubated for 10 min at RT with 50 µl of buffer containing the test compounds. 50 µl of 50 nM [3H] Serotonin (5HT) solution (final concentration: 25 nM [3H] 5HT) have been added and plates have been incubated for 7 min at RT, during which cells take up radiolabelled 5HT. Aspirating the solution and rapidly washing the cells with cold buffer has terminated the uptake.
The amount of radioactive 5HT incorporated in the cells has been then measured by adding the scintillation cocktail directly onto the cells and reading the plate in the Top Count. The data have been digitally processed to obtain the pIC50 values of the antagonists. The pKi values have been calculated using the Chen-Prusoff equation.

The action of the compounds of the invention at the NK₁ receptor and/or serotonin transporter may be determined by using conventional animal models. Thus, the ability to bind at the NK₁ receptor and/or serotonin transporter was determined using the guinea pig pup isolation calls model as described by Pettijohn, Psychol. Rep., 1979 and Rupniak et al., Neuropharmacology, 2000.

Compounds of the invention are useful in the treatment of CNS disorders and psychotic disorders, in particular in the treatment or prevention of depressive states and /or in the treatment of anxiety as defined in, but not restricted to, Diagnostic Statistical of Mental Disorder (DSM) IV edition edit by American Psychiatric Association and International Classification Diseases 10th revision (ICD10).
Thus, for example, depressive states include Major Depressive Disorders (MDD), including bipolar depression, unipolar depression, single or recurrent major depressive episodes, recurrent brief depression, with or without psychotic features, catatonic features, melancholic features including anorexia, weight loss, atypical features, anxious depression, cyclothymic or postpartum onset.
Other mood disorders encompassed within the term major depressive disorders include dysthymic disorders with early or late onset and with or without atypical features, neurotic depression, post-traumatic stress disorders and social phobia; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood. Major depressive disorders may also result from a general medical condition including, but not limited to, myocardial infarction, diabetes, miscarriage or abortion, etc.
The term anxiety includes anxiety disorders, such as panic disorders with or without agoraphobia, agoraphobia, phobias, for example, social phobias or agoraphobia, obsessive-compulsive disorder, stress disorders including post-traumatic stress disorders, generalised anxiety disorders, acute stress disorders and mixed anxiety-depression disorders.

Compounds of the invention are useful as analgesics. In particular, they are useful in the treatment of traumatic pain such as postoperative pain; traumatic avulsion pain such as brachial plexus; chronic pain such as arthritic pain such as occurring in osteo-, rheumatoid or psoriatic arthritis; neuropathic pain such as post-herpetic neuralgia, trigeminal neuralgia, segmental or intercostal neuralgia, fibromyalgia, causalgia, peripheral neuropathy, diabetic neuropathy, chemotherapy-induced neuropathy, AIDS related neuropathy, occipital neuralgia, geniculate neuralgia, glossopharyngeal neuralgia, reflex sympathetic dystrophy, phantom limb pain; various forms of headache such as migraine, acute or chronic tension headache, temporomandibular pain, maxillary sinus pain, cluster headache; odontalgia; cancer pain; pain of visceral origin; gastrointestinal pain; nerve entrapment pain; sport's injury pain; dysmennorrhoea; menstrual pain; meningitis; arachnoiditis; musculoskeletal pain; low back pain e.g. spinal stenosis; prolapsed disc; sciatica; angina; ankylosing spondyolitis; gout; burns; scar pain; itch and thalamic pain such as post stroke thalamic pain.

Compounds of the invention are also useful in the treatment of sleep disorders including dysomnia, insomnia, sleep apnea, narcolepsy, and circadian ritmic disorders.

Compounds of the invention are also useful in the treatment or prevention of the cognitive disorders. Cognitive disorders include dementia, amnestic disorders and cognitive disorders not otherwise specified.

Furthermore, compounds of the invention are also useful as memory and/or cognition enhancers in healthy humans with no cognitive and/or memory deficit.

Compounds of the invention are also useful in the treatment of tolerance to and dependence on a number of substances. For example, they are useful in the treatment of dependence on nicotine, alcohol, caffeine, phencyclidine (phencyclidine like compounds) or in the treatment of tolerance to and dependence on opiates (e.g. cannabis, heroin, morphine) or benzodiazepines; in the treatment of addiction to cocaine, sedative ipnotic, amphetamine or amphetamine-related drugs (e.g. dextroamphetamine, methylamphetamine) or a combination thereof.

Compounds of the invention are also useful as anti-inflammatory agents. In particular, they are useful in the treatment of inflammation in asthma, influenza, chronic bronchitis and rheumatoid arthritis; in the treatment of inflammatory diseases of the gastrointestinal tract such as Crohn's disease, ulcerative colitis, inflammatory bowel disease and non-steroidal anti-inflammatory drug induced damage; inflammatory diseases of the skin such as herpes and eczema; inflammatory diseases of the bladder such as cystitis and urge incontinence; and eye and dental inflammation.

Compounds of the invention are also useful in the treatment of allergic disorders, in particular allergic disorders of the skin such as urticaria, and allergic disorders of the airways such as rhinitis.

Compounds of the invention are also useful in the treatment or prevention of schizophrenic disorders including paranoid schizophrenia, disorganised schizophrenia, catatonic schizophrenia, undifferentiated schizophrenia, residual schizophrenia.

Compounds of the invention are also useful in the treatment of emesis, i.e. nausea, retching and vomiting. Emesis includes acute emesis, delayed emesis and anticipatory emesis. The compounds of the invention are useful in the treatment of emesis however induced. For example, emesis may be induced by drugs such as cancer chemotherapeutic agents such as alkylating agents, e.g. cyclophosphamide, carmustine, lomustine and chlorambucil; cytotoxic antibiotics, e.g. dactinomycin, doxorubicin, mitomycin-C and bleomycin; anti-metabolites, e.g. cytarabine, methotrexate and 5-fluorouracil; vinca alkaloids, e.g. etoposide, vinblastine and vincristine; and others such as cisplatin, dacarbazine, procarbazine and hydroxyurea; and combinations thereof; radiation sickness; radiation therapy, e.g. irradiation of the thorax or abdomen, such as in the treatment of cancer, poisons; toxins such as toxins caused by metabolic disorders or by infection, e.g. gastritis, or released during bacterial or viral gastrointestinal infection; pregnancy; vestibular disorders, such as motion sickness, vertigo, dizziness and Meniere's disease; post-operative sickness; gastrointestinal obstruction; reduced gastrointestinal motility; visceral pain, e.g. myocardial infarction or peritonitis; migraine; increased intercranial pressure; decreased intercranial pressure (e.g. altitude sickness); opioid analgesics, such as morphine; and gastro-oesophageal reflux disease (GERD) such as erosive GERD and symptomatic GERD or non erosive GERD, acid indigestion, over-indulgence of food or drink, acid stomach, sour stomach, waterbrash/regurgitation, heartburn, such as episodic heartburn, nocturnal heartburn, and meal-induced heartburn, dyspepsia and functional dyspepsia.

Compounds of the invention are also useful in the treatment of gastrointestinal disorders such as irritable bowel syndrome, gastro-oesophageal reflux disease (GERD) such as erosive GERD and symptomatic GERD or non erosive GERD, acid indigestion, over-indulgence of food or drink, acid stomach, sour stomach, waterbrash/regurgitation, heartburn, such as episodic heartburn, nocturnal heartburn, and meal-induced heartburn, dyspepsia and functional dyspepsia (such as ulcer-like dyspepsia, dysmotility-like dyspepsia and unspecified dyspepsia) chronic constipation; skin disorders such as psoriasis, pruritis and sunbum; vasospastic diseases such as angina, vascular headache and Reynaud's disease; cerebral ischeamia such as cerebral vasospasm following subarachnold haemorrhage; fibrosing and collagen diseases such as scleroderma and eosinophilic fascioliasis; disorders related to immune enhancement or suppression such as systemic lupus erythematosus and rheumatic diseases such as fibrositis: and cough.

The compounds of the invention are also useful in premenstrual dysphoric disorder (PMDD), in chronic fatigue syndrome and Multiple sclerosis. Compounds of the invention have been found to exhibit anxiolytic and antidepressant activity in conventional tests. For example in Guinea pig pups separation-induced vocalisations (Molewijk et al., 1996).

The invention therefore provides a compound of formula (I) or a pharmaceutically acceptable salt or solvate thereof for use in therapy, in particular in human medicine.

There is also provided as a further aspect of the invention the use of a compound of formula (I) or a pharmaceutically acceptable salt or solvate thereof in the preparation of a medicament for use in the treatment of conditions mediated by tachykinins (including substance P and other neurokinins) and/or by selective inhibition of serotonin reuptake.

In a further aspect there is provided the use of a compounds of formula(I) or a pharmaceutically acceptable salt or solvate thereof in the preparation of a medicament for use in the treatment of depression and /or anxiety.

It will be appreciated that reference to treatment is intended to include prophylaxis as well as the alleviation of established symptoms.
Compounds of formula (I) may be administered as the raw chemical but the active ingredient is preferably presented as a pharmaceutical formulation.

Accordingly, the invention also provides a pharmaceutical composition which comprises at least one compound of formula (I) or a pharmaceutically acceptable salt thereof and formulated for administration by any convenient route. Such compositions are preferably in a form adapted for use in medicine, in particular human medicine, and can conveniently be formulated in a conventional manner using one or more pharmaceutically acceptable carriers or excipients.

Thus compounds of formula (I) may be formulated for oral, buccal, parenteral, topical (including ophthalmic and nasal), depot or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. progelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the composition may take the form of tablets or formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device.

A proposed dose of the compounds of the invention is 1 to about 1000mg per day. It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected.
Thus for parenteral administration a daily dose will typically be in the range of 1 to about 100 mg, preferably 1 to 80 mg per day. For oral administration a daily dose will typically be within the range 1 to 300 mg e.g. 1 to 100 mg.

Compounds of formula (I), and salts and solvates thereof, may be prepared by the general methods outlined hereinafter. In the following description, the groups R, R₁ R₂, R₃, R₄, R₅, R₆ R₇ R₈, L, m, n, p and q have the meaning as previously defined for compounds of formula (I) unless otherwise stated.

Compounds of formula (I) may be prepared by reaction of of an activated derivative of the carboxylic acid (II), wherein R₁ has the meaning previously defined or is a protected group thereof, with amine (III) 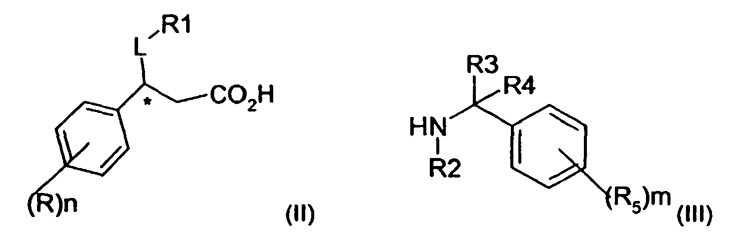 wherein R₂ is C₁₋₄ alkyl or a nitrogen protecting group, followed where necessary by removal of any protecting group.

Suitable activated derivatives of the carboxyl group include the acyl halide, mixed anhydride, activated ester such as thioester or the derivative formed between the carboxylic acid group and a coupling agent such as that used in peptide chemistry, for example carbonyl diimidazole or dicyclohexylcarbodiimide.
The reaction is preferably carried out in an aprotic solvent such as hydrocarbon, halohydrocarbon such as dichloromethane or an ether such as tetrahydrofuran.

The activated derivatives of the carboxylic acid (II) may be prepared by conventional means. A particular suitable activated derivative for use in this reaction is O-(Benzotriazol-1-yl) -N,N,N',N'-tetramethyluronium tetrafluoroborate.

Compounds of formula (II), wherein L is a single bond, may be prepared by reaction of a cyano derivative (IV), wherein Rg is a suitable carboxyl protecting group, with an acid such as for example concentrated sulfuric acid, followed , if it is still necessary, by removal of the carboxyl protecting group Rg 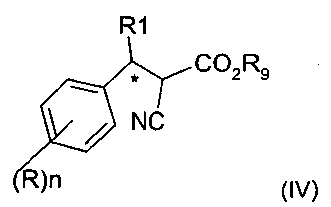 The reaction is conveniently carried out in a solvent such as acetic acid and heating the reaction mixture up to 150°

Suitable carboxyl protecting groups Rg for use in this reaction include alkyl, such as methyl or ethyl, trichloroalkyl, trialkylsilylalkyl, or arylmethyl groups such as benzyl, nitrobenzyl or trityl.

In one embodiment of the invention compounds of formula (II) wherein L is a single bond may be prepared by reduction of a compound of formula(V), 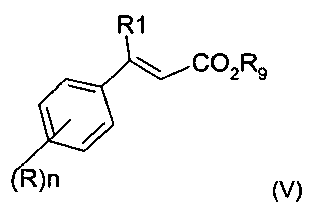 with a suitable reducing agent such as hydrogen in the presence of a catalyst such as platinum oxide, or palladium on carbon. Alternatively the reduction may be carried out with alkaline metal such as Mg. The reduction is carried out in a suitable solvent such as alcohol (e.g methanol or ethanol) at room temperature, followed by the removal of the carboxyl protecting group R₉.

Compound of formula (V) may be prepared by reaction of a keto compound of formula(VI) 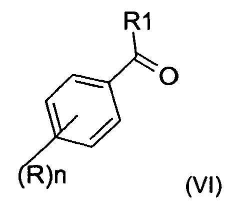 with an appropriate phosphorus reagent capable of converting the group C(O) into the group(VII). 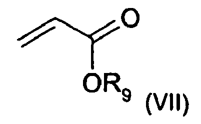 In one embodiment of this process the reaction may be carried out using a phosphorus ylide of formula (VIII) 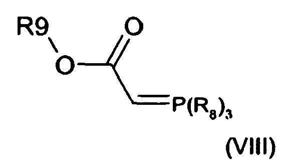 wherein R₈ is an alkyl or phenyl group.
The reaction is carried out in an aprotic solvent such as tetrahydrofuran, acetonitrile or dimethylformamide at a temperature ranging from -20°C to the reflux temperature of the solvent.

In a further embodiment of the invention compounds of formula(II), wherein L is a double bond, may be prepared by reduction followed by elimination of the leaving group of a compound of formula(V), wherein the carbon atom of the group R1 linked to the carbon atom*, is substituted by a leaving group such halogen (e.g fluorine, bromine or tosyl) 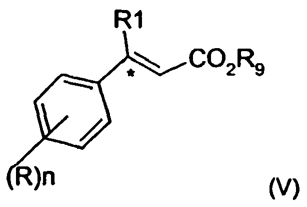 The reaction may be carried out with alkaline metal such as Mg in a suitable solvent such as alcohol (e.g methanol or ethanol) at room temperature.

Compounds of formula (IV) may be prepared by reaction of a compound of formula (IX) with a compound of formula (X), wherein X is a halogen group (i.e bromine). 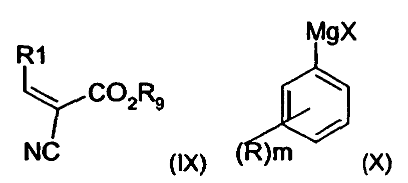 The reaction conveniently takes place in an aprotic solvent such as a hydrocarbon (i.e toluene) and at a temperature within the range 0-25°C.

Compounds of formula (IX) may be prepared by reaction of a compounds of formula (XI) with a cyano derivative (XII). 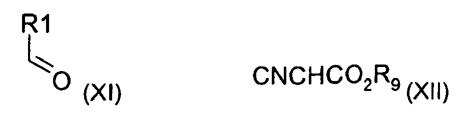 Compounds of formulae (VI) (X) (XI) and (XII) are known compounds or may be prepared according to the procedure used for known compounds.
Thus compounds of formula (VI) may be prepared according to the procedure described by Robert L. Duncan,et al. Journal. Med Chem (1970) Vol13, n°1, 1-6.
Thus compounds of formula (X) may be prepared according to the procedure described by Jones LA et al., J. Organomet. Chem. (1985), 284 (2), 159-169.
Compounds of formula(XI) may be prepared according to the procedure described by Itani, Hiromichi et al. Bioorganic & Medicinal Chemistry Letters (2002), 12(5), 799-802.
Compounds of formula(XII) may be prepared according to the procedure described by Lei, Yi Xiong; et al. Journal of Organic Chemistry (2003), 68(3), 947-959 .

Examples of suitable nitrogen protecting groups used for preparing compounds and or intermediates of the present invention include alkoxycarbonyl e.g. t-butoxycarbonyl, benzyloxycarbonyl, arylsulphonyl e.g. phenylsulphonyl or 2-trimethylsilylethoxymethyl.

Protection and deprotection may be effected using conventional techniques such as those described in "Protective Groups in Organic Synthesis 2^{nd} Ed." by T.W. Greene and P. G. M. Wuts (John Wiley and Sons, 1991) and as described in the examples hereinafter.

When a specific enantiomer of a compound of general formula (I) is required, this may be obtained for example by resolution of a corresponding enantiomeric mixture of a compound of formula (I) using conventional methods.
Thus, for example, specific enantiomers of the compounds of formula (I) may be obtained from the corresponding enantiomeric mixture of a compound of formula (I) using chiral HPLC procedure.
The chiral amine (III) may be prepared from the corresponding racemic amine (III) using any conventional procedures such as salt formation with a suitable optically active acid such as for example di-p-toluoyl-D-tartaric acid or di-p-toluoyl-L-tartaric acid, or using chiral HPLC procedure.
Alternatively, enantiomers of a compound of general formula (I) may be synthesised from the appropriate optically active intermediates using any of the general processes described herein.

Where it is desired to isolate a compound of formula (I) as a salt, for example a pharmaceutically acceptable salt, this may be achieved by reacting the compound of formula (I) in the form of the free base with an appropriate amount of suitable acid and in a suitable solvent such as an alcohol (e.g. ethanol or methanol), an ester (e.g. ethyl acetate) or an ether (e.g. diethyl ether, *tert*-butylmethyl ether or tetrahydrofuran).

Melting points (m.p.) were determined on a Buchi m.p. apparatus and are uncorrected. R.T. or r.t. refer to room temperature.
Infrared spectra (IR) were measures in chloroform or nujol solutions on a FT-IR instrument. Proton Magnetic Resonance (NMR) spectra were recorded on Varian instruments at 400 or 500 MHz, on Bruker instrument at 300 MHz, chemical shifts are reported in ppm (δ) using the residual solvent line as internal standard. Splitting patterns are designed as s, singlet; d, double; t, triple; q, quartet; m, multiplet; b, broad. The NMR spectra were recorded at temperature ranging from 25 to 60°C, where not specified the spectra are recorded at 25°C; when more than one conformer were detected the chemical shifts for the most abundant one is reported. Mass spectra (MS) were taken on a VG Quattro mass spectrometer. In the mass spectra the more abundant peak or the molecular ion peak is reported. Optical rotations were determined at 20°C with a Jasco DIP360 instrument (I=10 cm, cell volume = 1 mL, λ = 589 nm). Flash silica gel chromatography was carried out over silica gel 230-400 mesh supplied by Merck AG Darmstadt, Germany. T.I.c. refers to thin layer chromatography on 0.25 mm silica gel plates (60F-254 Merck) and visualized with UV light and/or ninhydrine.
For phase separations performed by using microfiltration device: phase separation cartridge with polypropylene frit by Whatman and Alltech. For purifications carried out by using SCX: SCX-cartridges (loading 0.75mmol\g) by Varian.
Solutions were dried over anhydrous sodium sulphate.
Methylene chloride was redistilled over calcium hydride and tetrahydrofuran was redistilled over sodium.
The following abbreviation are used in the text: AcOEt = ethyl acetate, ACN = acetonitrile, CH = cyclohexane, DCM = methylene chloride, DIPEA = N,N-diisopropylethylamine, DMF = N,N'-dimethylformamide, Et2O = diethyl ether, EtOH = ethanol, MeOH = methanol TEA = triethylamine, THF = tetrahydrofuran.
Enantiomer 1, enantiomer 2, diastereoisomer 1, diastereoisomer 2, diastereoisomer 3 or diastereoisomer 4 refer to a single enantiomer or a single diastereoisomer respectively, whose absolute stereochemistry was not characterised.
Isomer 1 and isomer 2 refer to isomers whose E or Z conformation at the double was not characterised.
Diastereoisomer A or diastereoisomer B refers to mixture of two diastereoisomers whose absolute stereochemistry was not characterised .

### Intermediate 1

### Piperidine-1,4-dicarboxylic acid 1-tert-butyl ester 4-ethyl ester

A solution of di-*tert-*butyl-dicarbonate (7.07 mg) in anhydrous DCM (20 mL) was added to a solution of ethyl isonipecotate (5 mL) in anhydrous DCM (40 mL) previously cooled to 0°C under a Nitrogen atmosphere. The solution was stirred at r.t. overnight, then it was washed with 1N hydrochloric acid solution and brine. The organic phase was dried and concentrated *in vacuo* to give the title compound (8 g) as a pale yellow oil.
T.I.c.: CH/AcOEt 1:1, Rf=0.67.
NMR (CDCl₃): δ (ppm) 4.11 (q, 2H); 3.96 (m, 2H); 2.8 (m, 2H); 2.38 (m, 1H); 1.85 (m, 2H); 1.6 (m, 2H); 1.42 (s, 9H); 1.25 (t, 3H).

### Intermediate 2

### Hydroxymethylpiperidine-1-carboxylic acid tert-butyl ester

Lithium aluminium hydride (1M solution in THF -17.8 mL) was added to a solution of intermediate 1 (8.0 g) in anhydrous THF (80 mL) previously cooled to 0°C under a Nitrogen atmosphere. The mixture was stirred at 0°C for 20 minutes, then it was treated with water (0.7 mL), 1 M sodium hydroxide solution (0.7 mL) and water (2 mL). The inorganic salts were filtered off and the organic layer was concentrated *in vacuo* to give the title compound (7.0 g) as a yellow oil.
T.I.c.: CH/AcOEt 1:1, Rf=0.25 (detection with ninhydrine).
NMR (CDCl₃): δ (ppm) 4.1 (m, 2H); 3.71 (m, 1 H); 3.45 (m, 2H); 2.67 (m, 2H); 1.83 (m, 1 H); 1.65 (m, 2H); 1.44 (s, 9H); 1.15 (m, 2H).

### Intermediate 3

### 4-Formyl-piperidine-1-carboxylic acid tert-butyl ester

### Method A

2,2,6,6-Tetramethylpiperidin-1-yl-oxy (free radical, TEMPO - 0.486 g) and iodobenzene diacetate (11.05 g) were added to a solution of intermediate 2 (7.0 g) in anhydrous DCM (40 mL). The mixture was stirred at r.t. overnight, then it was washed with a 20% sodium thiosulphate solution (50 mL) and brine.
The organic phase was dried, concentrated *in vacuo* and the residue was purified by flash chromatography (CH/AcOEt 7:3) to give the title compound (4.76 g) as a pale yellow foam.

### Method B

Diisobutylaluminium hydride (1M in toluene - 8.6 mL) was dropped into a solution of intermediate 1 (1 g) in anhydrous toluene (13 mL) previously cooled to -78°C under a Nitrogen atmosphere over 30 minutes. The solution was stirred at -78°C for 2 hours, then it was quenched with a 10% sodium hydroxide solution (16 mL) at -78°C. The mixture was allowed to warm to r.t. and the layers were separated. The organic layer was washed with a 10% sodium hydroxide solution and brine, dried and concentrated *in vacuo* to give the title compound (0.7 g) as yellow foam.
T.l.c.: CH/AcOEt 7:3, Rf=0.35 (detection with ninhydrine).
NMR (CDCl₃): δ (ppm) 9.63 (s, 1 H); 3.98 (m, 2H); 2.89 (m, 2H); 2.35 (m, 1 H); 1.85 (m, 2H); 1.57 (m, 2H).

### Intermediate 4

### 4-(2-Cyano-2-ethoxycarbonyl-vinyl)-piperidine-1-carboxylic acid tert-butyl ester

A mixture of intermediate 3 (4.5 g), ethyl cyanoacetate (2.47 mL), ammonium acetate (0.813 g) and acetic acid (1.27 mL) in anhydrous toluene (90 mL) was heated to 80°C for 3 hours, then it was allowed to cool to r..t, and washed with water, 1 N sodium hydroxide solution and brine. The organic phase was dried and concentrated *in vacuo* to a residue, which was purified by flash chromatography (from CH to CH/AcOEt 8:2) to give the title compound (5.5 g) as a white solid.
T.I.c.: CH/AcOEt 7:3, Rf=0.45 (detection with ninhydrine).
NMR (d₆-DMSO): δ (ppm) 7.6 (d, 1H); 4.24 (q, 2H); 3.96 (bd, 2H); 2.8-2.71 (m, 3H); 1.61 (bd, 2H); 1.47-1.38 (bd, 2H); 1.39 (s, 9H); 1.25 (t, 3H).
MS (ES/+): m/z=309 [M+H]⁺.

### Intermediate 5

### 4-[2-Cyano-2-ethoxycarbonyl-1-(4-fluoro-phenyl)-ethyl]-piperidine-1-carboxylic acid tert-butyl ester

A solution of 4-fluorophenyl magnesium bromide (2M in Et2O - 12.16 mL) in anhydrous Et2O (5 mL) was dropped into a solution of intermediate 4 (2.5 g) in anhydrous Et2O (20 mL) previously heated to reflux under a Nitrogen atmosphere. The mixture was diluted with further Et2O (10 mL) and heated to reflux for 30 minutes. The mixture was allowed to warm to r.t. and treated with 3N sulfuric acid solution (15 mL). The mixture was stirred at r.t. for 30 minutes, then AcOEt was added and the layers were separated. The aqueous layer was extracted with further AcOEt. The combined organic extracts were washed with a saturated sodium hydrogen carbonate and brine, then dried and concentrated *in vacuo.* The residue was purified by flash chromatography (from CH to CH/AcOEt 8:2) to give the title compound (2.47 g- diastereoisomeric mixture) as a white foam.
T.l.c.: CH/AcOEt 7:3, Rf=0.38 (detection with ninhydrine).
NMR (d₆-DMSO - 80°C): δ (ppm) 7.32 (dd, 2H); 7.15 (t, 2H); 4.65 (d, 1H); 4.0 (q; 3H); 3.83 (m, 1 H); 3.1 (dd, 1 H); 2.76 (m, 1H); 2.63 (m, 1 H); 1.95 (m, 1 H); 1.87 (m, 1H); 1.38 (s, 9H); 1.2 (m, 1H); 1.14 (m, 1H); 0.92 (m, 1H).
MS (ES/+): m/z=405 [M+H]⁺.

### Intermediate 6

### 4-[2-Carboxy-1-(4-fluoro-phenyl)-ethyl]-piperidine-1-carboxylic acid tert-butyl ester Method A

A mixture of intermediate **5** (2.4 g) in acetic acid (30 mL), conc sulfuric acid (8 mL) and water (9 mL) was heated to 140°C for 2.5 hours. The solution was allowed to cool to r.t. and dropped into a 2.5M sodium hydroxide solution (300 mL). Then, di-*tert*-butyl-dicarbonate (1.35 g) was added and the resulting mixture was stirred at r.t. overnight. It was cooled to 0°C and treated with 3M hydrochloric acid solution until pH=6-7 and then extracted many times with AcOEt. The combined organic extracts were washed with brine, dried and concentrated *in vacuo.* The residue was purified by flash chromatography (CH/AcOEt from 1:1 to 2:8) to give the title compound (0.12 g) as a pale yellow foam.

### Method B

A 3M potassium hydroxide solution (1.5 mL) was added to a solution of intermediate 9 (90 mg) in ethanol (2 mL). The mixture was heated in microwave (300W, P=300p.s.i..) at 150°C for 40 minutes. The solution was allowed to cool to r.t., acidified to pH=5 with a buffer solution pH3 (citrate-hydrochloric acid buffer solution purchase from Fluka) and extracted with AcOEt. The organic extract was dried and concentrated *in vacuo* to give the title compound (58 mg) as a white foam.

### Method C

A solution of lithium hydroxide monohydrate (1.78 g) in water (27 mL) was added to a solution of intermediate **12** (3.1 g) in MeOH (100 mL). The mixture was heated to 80°C for 1 hour, then it was allowed to cool to r.t., diluted with water and concentrated to half volume. The residue was acidified with 3M hydrochloric acid solution and extracted with AcOEt. The organic layer was dried and concentrated *in vacuo* to give the title compound (2.7 g) as a white foam.
T.I.c.: CH/AcOEt 1:1, Rf=0.2 (detection with ninhydrine).
IR (nujol, cm⁻¹): 3200 (COOH), 1732 and 1690 (C=O).
NMR (d₆-DMSO): δ (ppm) 11.94 (bs, 1H); 7.2 (dd, 2H); 7.08 (dd, 2H); 3.95-2.6 (2m, 4H); 2.83-2.72 (m, 2H); 2.5 (m, 1 H); 1.59 (m, 1 H); 1.34 (s, 9H); 1.72-0.94 (m, 2H); 1.22-0.82 (m, 2H).

### Intermediate 7

### 4-[2-[(3,5-Bis-trifluoromethyl-benzyl)-methyl-carbamoyl)1-1-(4-fluoro-phenyl)-ethyl]-piperidine-1-carboxylic acid tert-butyl ester

DIPEA (0.116 mL) and O-(benzotriazol-1-yl)-N,N,N'N'-tetramethyluronium tetrafluoroborate (0.071 g) were added to a solution of intermediate 6 (0.06 g) in anhydrous DMF (5 mL) under a Nitrogen atmosphere. After stirring for 20 minutes, (3,5-bis-trifluoromethyl-benzyl)-methylamine hydrochloride (0.1 g) was added. The mixture was stirred at r.t. for 4 hours, then it was diluted with AcOEt and washed with a 5% sodium hydrogen carbonate solution and brine. The organic layer was dried, concentrated *in vacuo* and the residue was purified by flash chromatography (CH/AcOEt 7:3) to give the title compound (0.062 g) as a colourless oil.
T.I.c.: CH/AcOEt 1:1, Rf=0.35.
IR (nujol, cm⁻¹): 1684 and 1645 (C=O).
NMR (d₆-DMSO): δ (ppm) 7.95 (s, 1 H); 7.7 (s, 2H); 7.2 (dd, 2H); 7.0 (dd, 2H); 4.54 (q, 2H); 3.94-3.93 (2d, 2H); 3.3 (m, 1H); 3.01-2.98 (m, 1H); 2.96 (s, 3H); 2.81-2.75 (m, 2H); 1.74-1.58 (m, 2H); 1.34 (s, 9H); 1.27-1.18 (m, 2H); 0.99-0.76 (2q, 2H).
MS (ES/+): m/z=591 [M+H]⁺.

### Intermediate 8

### 4-[2-[(3,5-Dichloro-benzyl)-methyl-carbamoyl)]-1-(4-fluoro-phenyl)-ethyl]-piperidine-1-carboxylic acid tert-butyl ester

DIPEA (0.116 mL) and O-(benzotriazol-1-yl)-N,N,N'N'-tetramethyluronium tetrafluoroborate (0.071 g) were added to a solution of intermediate 6 (0.06 g) in anhydrous DMF (5 mL) under a Nitrogen atmosphere. After stirring for 20 minutes, (3,5-dichloro-benzyl)-methylamine hydrochloride (0.077 g) was added. The mixture was stirred at r.t. for 4 hours, then it was diluted with AcOEt and washed with a 5% sodium hydrogen carbonate solution and brine. The organic layer was dried, concentrated *in vacuo* and the residue was purified by flash chromatography (CH/AcOEt 7:3) to give the title compound (0.048 g) as a colourless oil.
T.I.c.: CH/AcOEt 1:1, Rf=0.31.
IR (nujol, cm⁻¹): 1688 and 1646 (C=O).
NMR (d₆-DMSO): δ (ppm) 7.43 (s, 1H); 7.22 (dd, 2H); 7.05 (dd, 2H); 6.98 (s, 2H); 4.37 (q, 2H); 3.9 (2d, 2H); 3.0-2.71 (m, 4H); 2.9 (s, 3H); 1.77-1.59 (m, 2H); 1.34 (s, 9H); 1.345-1.18 (m, 2H); 1.02-0.79 (2q, 2H).
MS (ES/+): m/z=523 [M+H]⁺.

### Intermediate 9

### 4-[2-Cyano-1-(4-fluoro-phenyl)-ethyl]-piperidine-1-carboxylic acid tert-butyl ester

A mixture of intermediate 5 (0.35 g), sodium chloride (17.5 mg) and water (35 L) in dimethylsulfoxide (3 mL) was heated to 160°C for 2 hours. The mixture was allowed to cool to r.t., diluted with water and extracted with AcOEt. The organic layer was dried and concentrated *in vacuo.* The residue was purified by flash chromatography (from CH to CH/AcOEt 7:3) to give the title compound (0.27 g) as a yellow oil.
T.l.c.: CH/AcOEt 1:1, Rf=0.61 (detection with ninhydrine).
IR (nujol, cm⁻¹): 2245 (C≡N), 1681 (C=O).
NMR (d₆-DMSO): δ (ppm) 7.31 (dd, 2H); 7.16 (dd, 2H); 3.95-2.6 (2m, 4H); 2.9-2.8 (m, 2H); 2.7 (m, 1H); 1.7 (m, 1H); 1.35 (s, 9H); 1.74-0.99 (m, 2H).
MS (ES/+): m/z=333 [M+H]⁺.

### Intermediate 10

### 4-(4-Fluoro-benzoyl)-piperidine-1-carboxylic acid tert-butyl ester

A solution of intermediate 3 (126.6 mg) in anhydrous Et2O (2 mL) was dropped into a solution of 4-fluorophenyl magnesium bromide (2M in Et2O - 1 mL) in anhydrous Et2O (0.5 mL) previously cooled to 0°C under a Nitrogen atmosphere. At the end of the addition the mixture was allowed to warm to r.t. and stirred at 23°C for 30 minutes. The mixture was quenched with a saturated ammonium chloride solution (2 mL). The aqueous layer was further extracted with AcOEt. The combined organic extracts were washed with brine, dried and concentrated *in vacuo* to give 4-[(4-fluoro-phenyl)hydroxy-methyl]-piperidine-1-carboxylic acid *tert*-butyl ester (292.3 mg- T.l.c.: DCM/AcOEt 9:1, Rf=0.4).
This material was dissolved in anhydrous DCM (4 mL) and treated portion-wise with Dess Martin periodinane (254.5 mg) under a Nitrogen atmosphere. The brown solution was stirred at r.t. for 3 hours, then further Dess Martin periodinane (127.2 mg) was added. The solution was diluted with Et2O (16 mL) and poured into a saturated sodium hydrogen solution (16 mL) containing sodium thiosulphate (392 mg). The mixture was stirred for 10 minutes, then the phases were separated. The organic layer was washed with a saturated sodium hydrogen carbonate solution. The organic layer was dried and concentrated in *vacuo* to a residue which was purified by flash chromatography (CH/AcOEt 8:2) to give the title compound (58 mg) as a yellow solid.
T.I.c.: DCM/AcOEt 9:1, Rf=0.82 (detection with ninhydrine).
IR (nujol, cm⁻¹): 1685 and 1675 (C=O).
NMR (d₆-DMSO): δ (ppm) 8.07 (t, 2H); 7.35 (t, 2H); 3.96 (d, 2H); 3.62 (t, 1 H); 2.9 (bs, 2H); 1.74-1.39 (m, 4H); 1.39 (s, 9H).
MS (ES/+): m/z=308 [M+H]⁺, 330 [M+Na]⁺.

### Intermediate 11

### E-4-[2-Ethoxycarbonyl-1-(4-fluoro-phenyl)-vinyl]-piperidine-1-carboxylic acid tert-butyl ester (11 a) and Z-4-[2-Ethoxycarbonyl-1-(4-fluoro-phenyl)-vinyl]-piperidine-1-carboxylic acid tert-butyl ester (11 b)

Triethylphosphonoacetate (1.33 g) was added to a suspension of sodium hydride (60% suspension in mineral oil - 200 mg) in anhydrous THF (7.4 mL) previously cooled to 0°C under a Nitrogen atmosphere. The mixture was allowed to warm to r.t. and stirred at 23°C for 20 minutes. Then, a solution of intermediate 10 (58 mg) in anhydrous THF (7.4 mL) was added and the resulting solution was heated to reflux for 48 hours. The solution was allowed to cool to r.t., diluted with water and extracted with Et2O. The organic layer was washed with water and brine, dried and concentrated *in vacuo* to a residue, which was purified by flash chromatography (CH/AcOEt 9:1) to give:
intermediate **11a** (181 mg) as yellow oil;
intermediate **11b** (267 mg) as yellow oil.

### Intermediate 11a:

T.I.c.: CH/AcOEt 7:3, Rf=0.64 (detection with ninhydrine).
NMR (d₆-DMSO): δ (ppm): 7.2 (m, 4H); 5.67 (s, 1H); 4.13 (q, 2H); 3.76 (t, 1H); 3.97-2.7 (m, 4H); 1.34 (s, 9H); 1.22 (t, 3H).
MS (ES/+): m/z=378 [M+H]⁺, 400 [M+Na]⁺.

### Intermediate 11 b:

T.I.c.: CH/AcOEt 7:3, Rf= 0.53 (detection with ninhydrine).
NMR (d₆-DMSO): δ (ppm): 7.2 (m, 4H); 5.83 (d, 1 H); 3.87 (q, 2H); 3.96-2.63 (m, 4H); 3.6 (m, 1 H); 1.63-1.17 (m, 4H); 1.36 (s, 9H); 0.97 (t, 3H).
MS (ES/+): m/z=378 [M+H]⁺, 400 [M+Na]⁺.

### Intermediate 12

### 4-[2-Methoxycarbonyl-1-(4-fluoro-phenyl]-ethyl]-piperidine-1-carboxyl acid tert-butyl ester

Magnesium turnings (20.3 mg) were added to a solution of intermediate 11a and 11b (63 mg) in anhydrous MeOH (1.7 mL) under a Nitrogen atmosphere with a low heating until hydrogen evolution is observed. Then, the reaction mixture was stirred at r.t. overnight, then water (2 mL) and a 10% acetic acid solution were added until dissolution of the magnesium salts. The mixture was made basic until pH=8.5 with 32% ammonium hydroxide solution and extracted with Et2O. The organic phase was washed with water and a saturated sodium hydrogen carbonate solution, dried and concentrated *in vacuo.* The residue was purified by flash chromatography (CH/AcOEt 7:3) to give the title compound (37 mg) as a yellow oil.
IR (nujol, cm⁻¹): 1739 and 1694 (C=O).
NMR (d₆-DMSO): δ (ppm) 7.08 (t, 2H); 7.2 (dd, 2H); 3.93-3.84 (dd, 2H); 3.41 (s, 3H); 2.84-2.61 (q, 4H); 2.8 (m, 1 H); 1.73-0.93 (m, 2H); 1.6 (m, 1 H); 1.34 (s, 9H); 1.21-0.84 (m, 2H).
MS (ES/+): m/z=366 [M+H]⁺.

### Intermediate 13

### 4-[2-[1-(3,5-Dichloro-phenyl)-ethyl]-methyl-carbamoyl]-1-(4-fluoro-phenyl)-ethyl]-piperidine-1-carboxylic acid tert-butyl ester (13a -diastereoisomer A)and 4-[2-[1-(3,5-Dichlorophenyl)-ethyl]-methyl-carbamoyl]-1-(4-fluoro-phenyl)-ethyl]-piperidine-1-carboxylic acid tert-butyl ester (13b -diastereoisomer B)

DIPEA (595 µL) and O-(benzotriazol-1-yl)-N,N,N'N'-tetramethyluronium tetrafluoroborate (0.563 g) were added to a solution of intermediate 6 (0.4 g) in anhydrous DMF (7 mL) under a Nitrogen atmosphere. After stirring for 30 minutes, intermediate 15 (0.256 g) was added. The mixture was stirred at r.t. overnight, then it was diluted with AcOEt and washed with a saturated ammonium chloride solution. The organic layer was dried, concentrated *in vacuo* and the residue was purified by flash chromatography (CH/AcOEt 7:3) to give:
intermediate 13a (180 mg) as yellow oil
intermediate 13b (180 mg) as yellow oil.

### Intermediate 13a:

T.I.c.: CH/AcOEt 1:1, Rf=0.37.
NMR (d₆-DMSO): δ (ppm) 7.48 (s, 1H); 7.15 (s, 2H); 7.21 (m, 2H); 7.09 (t, 2H); 5.58 (q, 1H); 4.0-3.8 (dd, 2H); 3.0-2.8 (dd, 2H); 3.0 (m, 1H); 2.8 (s, 3H); 2.6 (m, 1H); 1.8-0.8 (m, 4H); 1.4-1.2 (d, 3H); 1.34 (s, 9H).

### Intermediate 13b:

T.l.c.: CH/AcOEt 1:1, Rf=0.30.
MS (ES/+): m/z=559 [M+Na]⁺.

### Intermediate 14

### 3',5'-Dichloroacetophenone

A solution of methyl iodide (4 mL) in anhydrous Et2O (40 mL) was dropped into a suspension of magnesium (1.6 g) in anhydrous Et2O (16 mL) under a Nitrogen atmosphere. At the end of the dropping, benzene (120 mL) was added and the Et2O eliminated with a Nitrogen flux. Then, a solution of 3,5-dichlorobenzonitrile (4 g) in benzene (48 mL) was added and the mixture was heated to reflux for 3 hours. The solution was cooled to 0°C and a 6N hydrochloric acid solution was added and the mixture was stirred overnight at r.t.. Water and Et2O were added and the layers were separated. The organic phase was washed with a saturated sodium hydrogen carbonate solution and brine, dried and concentrated *in vacuo.* The residue was purified by flash chromatography (CH/AcOEt 95:5) to give the title compound (2.55 g) as an orange oil.
T.I.c.: CH/AcOEt 8:2, Rf=0.64.
NMR (CDCl₃): δ (ppm) 7.75 (s, 2H); 7.6 (s, 1 H); 2.55 (s, 3H).

### Intermediate 15

### [1-(3,5-Dichloro-phenyl)-ethyl-methylamine

Methylamine (2M solution in MeOH - 13 mL) was added to a solution of intermediate 14 (500 mg) in MeOH (26 mL) under a Nitrogen atmosphere. The mixture was stirred at r.t. for 18 hours, then it was cooled to 0°C and sodium borohydride (98 mg) was added. The mixture was stirred at 0°C for 2 hours, then it was quenched with water and extracted with DCM. The organic layer was dried and concentrated *in vacuo* to give the title compound (340 mg) as yellow oil.
T.I.c.: CH/AcOEt 1:1, Rf=0.15.
NMR (CDCl₃): δ (ppm) 7.3 (m, 3H); 3.6 (q, 1H); 2.3 (s, 3H); 1.35 (d, 3H).
MS (ES/+): m/z=204 [M+H]⁺.

### Intermediate 16

### 1-Oxa-6-aza-spiro[2.5]octane-6-carboxylic acid tert-butyl ester

Sodium hydride (60% suspension in mineral oil - 3.6 g) was added to a solution of trimethylsulfoxonium iodide (19.8 g) in anhydrous DMSO (200 mL) under a Nitrogen atmosphere. The mixture was stirred at r.t. for 1 hour, then a solution of 1-(*tert-*butoxycarbonyl)-4-piperidone (15 g) in anhydrous DMSO (200 mL) was added. The mixture was heated to 60°C for 1.5 hours. The mixture was diluted with AcOEt (1 I) and washed with water and ice. The layers were separated. The aqueous layer was extracted with further AcOEt (500 mL). The combined organic extracts were washed with brine and dried. After concentration *in vacuo,* the crude was purified using a Biotage column (CH/AcOEt 8:2) to give the title compound (14.2 g).
T.I.c.: CH/AcOEt 7:3, Rf=0.61.
IR (nujol, cm⁻¹): 1699 (C=O).
NMR (CDCl₃): δ (ppm) 3.54 (m, 2H); 3.4 (m, 2H); 2.68 (s, 2H); 1.67 (m, 2H); 1.44 (s, 9H); 1.42 (m, 2H).

### Intermediate 17

### 4-Fluoro-4-hydroxymethyl-piperidine-1-carboxylic acid tert-butyl ester

A 70% solution of hydrofluoric acid in pyridine (12 mL) was added drop-wise to a solution of intermediate 16 (10 g) in anhydrous DCM (200 mL) previously cooled to -40°C under a Nitrogen atmosphere. After 1.5 hours, further hydrofluoric acid in pyridine (6 mL) was added.
After stirring for further 15 minutes, a saturated sodium hydrogen carbonate solution was added. The layers were separated and the aqueous phase was extracted three times with further DCM. The combined organic extracts were washed with brine, dried and concentrated *in vacuo* to a residue which was purified by flash chromatography (CH/AcOEt 6:4) to give the pure title compound (990 mg) and a fraction of title compound (6.01 g) impure of pyridine. Thus, this material was diluted with AcOEt and washed three times with a pH3 buffer solution and brine. The organic layer was dried and concentrated *in vacuo* to give a further amount of title compound (5.12 g).
T.l.c.: CH/AcOEt 1:1, Rf=0.35.
NMR (d₆-DMSO): δ (ppm) 4.99 (t, 1H); 3.79 (m, 2H); 3.43 (dd, 2H); 3.01 (bt, 2H); 1.8-1.4 (m, 4H); 1.43 (s, 9H).

### Intermediate 18

### 4-Fluoro-4-formyl-piperidine-1-carboxylic acid tert-butyl ester

Dimethylsulfoxide (3.8 mL) was added to a solution of oxalyl choride (1.7 mL) in anhydrous DCM (126 mL) previously cooled to -78°C under a Nitrogen atmosphere. The solution was stirred at -78°C for 1 hour, then a solution of intermediate 17 (2.5 g) in anhydrous DCM (36 mL) was dropped over 1 hour. The mixture was stirred at -78°C for 30 minutes, then TEA (7.4 mL) was added. The mixture was allowed to warm to r.t. over 1.5 hours, then it was washed with water, 5% sodium hydrogen carbonate solution and brine. The organic layer was dried and concentrated *in vacuo* to a residue which was purified by flash chromatography (CH/AcOEt 6:4) to give the title compound (1.7 g).
NMR (d₆-DMSO): δ (ppm) 9.64 (d, 1H); 3.89-3.78 (m, 4H); 1.82-1.59 (m, 4H); 1.39 (s, 9H). MS (ES/+): m/z=232[M+H]⁺.

### Intermediate 19

### 4-(2-Cyano-2-ethoxycarbonyl-vinyl)-4-fluoro-piperidine-1-carboxylic acid tert-butyl ester

A round bottom flask equipped with a Dean stark apparatus was charged with intermediate 18 (1.4 g), ethyl cyanoacetate (0.71 mL), ammonium acetate (233.5 mg) and acetic acid (0.35 mL) in anhydrous toluene (30 mL) under a Nitrogen atmosphere. The mixture was heated to 95°C for 4 hours, then to 120°C for 30 minutes. The mixture was allowed to cool to r.t. and further ethyl cyanoacetate (0.2 mL) was added. The mixture was heated to 120°C for 1 hour, then left at 90°C overnight. The reaction mixture was allowed to cool to r.t... diluted with AcOEt and washed with water (20 mL), 0.5M sodium hydroxide solution (3 x 20 mL), water (20 mL) and brine (30 mL). The organic phase was dried and concentrated *in vacuo* to a residue, which was purified by flash chromatography (CH/AcOEt 8:2) to give the title compound (1.33 g).
T.l.c.: CH/AcOEt 7:3, Rf=0.26 (detection with ninhydrine).
NMR (d₆-DMSO): δ (ppm) 7.74 (d, 1H); 4.26 (q, 2H); 3.9 (bs, 2H); 2.98 (m, 2H); 1.99 (m. 2H); 1.78 (m, 2H); 1.4 (s, 9H); 1.26 (t, 3H).
MS (ES/+): m/z=327 [M+H]⁺.

### Intermediate 20

### 4-(2-Cyano-2-ethoxycarbonyl)-1-(4-fluoro-phenyl)-4-fluoro-piperidine-1-carboxylic acid tert-butyl ester

4-Fluorophenyl magnesium bromide (1 M in THF - 4.6 mL) was dropped into a solution of intermediate 19 (500 mg) in anhydrous Et2O (20 mL) over 15 minutes under a Nitrogen atmosphere. The mixture was heated to reflux for 40 minutes. The mixture was allowed to warm to r.t. and treated with a saturated ammonium chloride solution (15 mL) and water (15 mL). The mixture was extracted with Et2O (3 x 30 mL). The combined organic extracts were washed with a saturated sodium hydrogen carbonate and brine, then dried and concentrated *in vacuo.* The residue was purified by flash chromatography (CH/AcOEt 8:2) to give the title compound (520 mg) as a colourless oil.
T.I.c.: CH/AcOEt 7:3, Rf=0.26(detection with ninhydrine).
IR (nujol, cm⁻¹): 2247 (CN); 1747 and 1690 (C=O).
NMR (d₆-DMSO): δ (ppm) 7.46 (dd, 2H); 7.2 (dd, 2H); 4.92 (d, 1H); 4.02 (q, 2H); 3.87 (bm, 1H); 3.75 (bm, 1H); 3.64 (dd, 1H); 2.94 (bm, 1H); 2.8 (bm, 1H); 1.64 (td, 1H); 1.54 (m, 1H); 1.44 (m, 1 H); 1.36 (s, 9H); 1.03 (t, 3H).
MS (ES/+): m/z=423 [M+H]⁺.

### Intermediate 21

### 4-(2-Cyano-1-(4-fluoro-phenyl)-ethyl]-4-fluoro-piperidine-1-carboxylic acid tert-butyl ester

A mixture of intermediate 20 (500 mg), sodium chloride (20 mg) and water (130 L) in anhydrous dimethylsulfoxide (4 mL) was heated to 150°C for 1.5 hours. The mixture was allowed to cool to r.t., diluted with AcOEt (30 mL) and washed with water and brine. The organic layer was dried and concentrated *in vacuo.* The residue was purified by flash chromatography (CH/AcOEt 8:2) to give the title compound (260 mg) as a colourless wax.
T.I.c.: CH/AcOEt 6:4, Rf=0.45(detection with ninhydrine).
MS (ES/+): m/z=295 [M-tBu]⁺.

### Intermediate 22

### 4-[2-Carboxy-1-(4-fluoro-phenyl)-ethyl]-4-fluoro-piperidine-1-carboxylic acid tert-butyl ester

### Method A

A 3M potassium hydroxide solution (3 mL) was added to a solution of intermediate 21 (80 mg) in abs. EtOH (3 mL). The mixture was heated in microwave (300W, P=90p.s.i.) at 150°C for 10+10+10 minutes. The solution was allowed to cool to r.t., acidified to pH=5 with a pH3 buffer solution (citrate-hydrochloric acid buffer solution purchase from Fluka) and 2N hydrochloric acid solution and extracted with AcOEt (3 x 25 mL). The organic extract was dried and concentrated *in vacuo* to give the title compound (56 mg) as a white solid.

### Method B

A solution of lithium hydroxide monohydrate (951 mg) in water (16 mL) was added to a solution of intermediate 37 (1.8 g) in MeOH (64 mL). The mixture was heated to 70°C for 1.5 hours, then it was allowed to cool to r.t., diluted with AcOEt/H₂O, acidified to pH 4 with a pH3 buffer solution (citrate-hydrochloric acid buffer solution purchase from Fluka) and 1N hydrochloric acid solution. The aqueous layer was extracted with further AcOEt. The organic extract was dried and concentrated *in vacuo* to give the title compound (1.6 g) as a white solid.
T.I.c.: AcOEt 100%, Rf=0.7 (detection with ninhydrine).
NMR (d₆-DMSO): δ (ppm) 12.05 (bs, 1 H); 7.28 (m. 2H); 7.12 (m, 2H); 3.79 (m, 2H); 3.26 (m, 1H); 2.88 (dd, 1 H); 2.8 (bm, 2H); 2.67 (dd, 1 H); 1.8-1.4 (m, 4H).
MS (ES/+): m/z=370 [M+H]⁺, 314 [M-tBu]⁺.

### Intermediate 23

### 4-[2-[(3,5-Dichloro-benzyl)-methyl-carbamoyl]-1-(4-fluoro-phenyl)-ethyl]-4-fluoropiperidine-1-carboxylic acid tert-butyl ester

DIPEA (80 µL) and O-(benzotriazol-1-yl)-N,N,N'N'-tetramethyluronium tetrafluoroborate (65 mg) were added to a solution of intermediate 21 (56 mg) in anhydrous DMF (4 mL) under a Nitrogen atmosphere. After stirring for 10 minutes, 3,5-dichlorobenzylmethylamine hydrochloride (40 mg) was added. The mixture was stirred at r.t. overnight, then it was diluted with water (5 mL) and extracted with AcOEt (20 mL). The aqueous layer was extracted with AcOEt (3 x 15 mL). The combined organic extracts were washed with brine, dried, concentrated *in vacuo* and the residue was purified by flash chromatography (CH/AcOEt 7:3) to give the title compound (65 mg) as a whitish foam.
T.I.c.: CH/AcOEt 4:6, Rf=0.5.
IR (nujol, cm⁻¹): 1688 and 1646 (C=O).
MS (ES/+): m/z=563 [M+Na]⁺, 465 [M-tBu-HF]⁺.

### Intermediate 24

### 1,1-dimethylethyl 4[3-[{(1R)-1-[3,5-bis(trifluoromethyl)phenyl]ethyl}(methyl)amino]-1-(4-fluorophenyl)-3-oxopropyl]-1-piperidinecarboxylate (24a) (diastereoisomer 1)and 1,1-dimethylethyl 4-[3-[{(1R)-1-[3,5-bis(trifluoromethyl)phenyl]ethyl}(methyl)amino]-1-(4-fluorophenyl)-3-oxopropyl]-1-piperidinecarboxylate (24b) (diastereoisomer 2)

DIPEA (110 µL) and O-(7-azabenzotriazol-1-yl)-N,N,N'N'-tetramethyluronium hexafluorophosphate (104 mg) were added to a solution of intermediate 6 (75 mg) in anhydrous DMF (3 mL) under a Nitrogen atmosphere. After stirring for 1 hour, (1*R*)-1-[3,5-bis(trifluoromethyl)phenyl]-*N*-methylethanamine (62 mg) was added. The mixture was stirred at r.t. overnight, then it was diluted with AcOEt and washed with water. The organic layer was dried, concentrated *in vacuo* and the residue was purified by flash chromatography (CH, then CH/AcOEt from 9/1 to 7/3) to give:
intermediate 24a (28 mg) as a colourless oil;
intermediate 24b (31 mg) as a colourless oil.

### Intermediate 24a:

T.I.c.: CH/AcOEt 1:1, Rf=0.55.
NMR (CDCl3): δ (ppm) 7.78 (s, 1H); 7.63 (s, 2H); 7.14 (dd, 2H); 7.00 (dd, 2H); 6.00 (q, 1H); 4.16 (b, 1 H); 4.04 (b, 1 H); 3.08 (dd, 1H); 2.79 (dd, 1H); 2.70 (m, 1H); 2.65 (dd, 1H); 2.56 (s, 3H); 2.55 (m, 1 H); 1.81 (m, 1 H); 1.66 (m, 1 H); 1.44 (s, 9H); 1.35 (bd, 1 H); 1.30 (d, 3H); 1.26 (m, 1 H); 1.02 (m, 1 H).
MS (ES/+): m/z=627 [M+Na]⁺.

### Intermediate 24b:

T.I.c.: CH/AcOEt 1:1, Rf=0.48.
NMR (CDCl3): δ (ppm) 7.80 (s, 1H); 7.45 (s, 2H); 7.12 (dd, 2H); 6.96 (dd, 2H); 6.01 (q, 1H); 4.16 (b, 1 H); 4.04 (b, 1H); 3.10 (dd, 1H); 2.74 (dd, 1 H); 2.72 (dd, 1 H); 2.70 (m, 1H); 2.58 (s, 3H); 2.56 (m, 1 H); 1.82 (m, 1H); 1.69 (m, 1H); 1.48 (d, 3H); 1.44 (s, 9H); 1.35 (bd, 1H); 1.15 (m, 1H); 1.02 (m, 1 H).
MS (ES/+): m/z=627 [M+Na]⁺.

### Intermediate 25

### 1,1-dimethylethyl 4-[3-[{(1S)-1-[3,5-bis(trifluoromethyl)phenyl]ethyl}(methyl)amino]-1 -[4-fluorophenyl)-3-oxopropyl-1 -piperidinecarboxylate(25a) (diastereoisomer 1) and 1,1-dimethylethyl 4-[3-[{(1S)-1-(3,5-bis(trifluoromethyl)phenyl]ethyl}(methyl)aminol-1-(4-fluorophenyl)-3-oxopropyl]-1-piperidinecarboxylate(25b) (diastereoisomer 2)

DIPEA (595 µL) and O-(7-azabenzotriazol-1-yl)-1,1,3,3-tetramethyluronium hexafluorophosphate (563 mg) were added to a solution of intermediate 6 (400 mg) in anhydrous DMF (7 mL) under a Nitrogen atmosphere. After stirring for 30 minutes, (1*S*)-1-[3,5-bis(trifluoromethyl)phenyl]-*N-*methylethanamine (339 mg) was added. The mixture was stirred at r.t. for 12 hours, then it was diluted with AcOEt and washed with a 5% sodium hydrogen carbonate solution and brine. The organic layer was dried, concentrated *in vacuo* and the residue was purified three times by flash chromatography (CH/AcOEt 7:3) to give:
intermediate 25a (198 mg) as a colourless oil
intermediate 25b (165 mg) as a colourless oil

### Intermediate 25a

T.I.c.: CH/AcOEt 7:3, Rf=0.40.
NMR (d₆-DMSO): δ (ppm) 8.0-7.6 (m, 3H); 5.71 (q, 1H); 3.9 (m, 2H); 3.0-2.4 (m, 2H); 3.0-2.4 (m, 2H); 1.7-0.8 (m, 5H); 2.71 (s, 3H); 1.34 (s, 9H); 1.34 (d, 3H).

### Intermediate 25b

T.I.c.: CH/AcOEt 7:3, Rf=0.35.
NMR (d₆-DMSO): δ (ppm) 8.1-7.56 (m, 3H); 7.17 (m, 2H); 6.97 (m, 2H); 5.73 (m, 1H); 3.90 (m, 2H); 3.00-2.6 (m, 3H); 3.0-2.6 (m, 2H); 1.8-0.8 (m, 5H); 2.69 (s, 3H); 1.43 (d, 3H); 1.34 (s, 9H).

### Intermediate 26

### 1,1-dimethylethyl 4-[3-({1-[3,5-bis(trifluoromethyl)phenyl]-1-methylethyl}amino)-1-(4-fluorophenyl)-3-oxopropyl]-1-piperidinecarboxylate

DIPEA (74 µL) and O-(benzotriazol-1-yl)-N,N,N'N'-tetramethyluronium tetrafluoroborate (59 mg) were added to a solution of intermediate 6 (50 mg) in anhydrous DMF (3 mL) under a Nitrogen atmosphere. After stirring for 30 minutes {1-[3,5-bis(trifluoromethyl)phenyl]-1-methylethyl}amine (52 mg) was added. The mixture was stirred at r.t. overnight, then it was diluted with AcOEt (10 mL) and washed with water (10 mL), then with a 0.1 M hydrochloric acid cold solution (5 mL) and finally with brine (5 mL). The organic layer was dried and concentrated *in vacuo* to give the title compound (91 mg) as a colourless oil.
T.I.c.: CH/AcOEt 1:1, Rf=0.52 (detection UV and with ninhydrine).
MS (ES/+): m/z=627 [M+Na]⁺.

### Intermediate 27

### 1,1-dimethylethyl 4-[3-[{1-[3,5-bis(trifluoromethyl)phenyl]-1-methylethyl}(methyl)amino]-1-(4-fluorophenyl)-3-oxopropyl]-1-piperidinecarboxylate

Sodium tert-butoxide (17 mg) was added to a solution of intermediate 26 (90 mg) in anhydrous DMF (1 mL) under a Nitrogen atmosphere. The yellow solution was stirred at r.t. for 30 minutes, then iodomethane (108 µL) was added. The mixture was stirred at r.t. overnight, then it was diluted with AcOEt (10 mL), washed with water (5 mL) and then with brine (5 mL). The organic layer was dried, concentrated *in vacuo* and the residue was purified by flash chromatography (CH/AcOEt 8:2) to give the title compound (50 mg) as a colourless oil.
T.l.c.: CH/AcOEt 6:4, Rf=0.53 (detection UV and with ninhydrine). MS (ES/+): m/z=519 [M-BOC+H]⁺.

### Intermediate 28

### 1 1-dimethylethyl 4-[3-[{[3-bromo-4-(methyloxy)phenyl]methyl)(methyl)aminol-1-(4-fluorophenyl)-3-oxopropyl]-1-piperidinecarboxylate

DIPEA (222 µl) and O-(7-azabenzotriazol-1-yl)-N,N,N',N'-tetramethyluronium hexafluoroposphate (212 mg) were added to a solution of intermediate 6 (150 mg) in anhydrous DMF (1 ml). After stirring for 20 minutes, {[3-bromo-4-(methyloxy)phenyl]methyl)methylamine (108 mg), was added. The mixture was then stirred at r.t. overnight, then it was diluted with DCM (2 ml), washed with a 5% sodium hydrogen carbonate solution (1 ml), filtered on Whatman polypropilene support filter tubes, washed with an ammonium chloride saturated solution (2 ml) and filtered once again on Whatman tubes. The filtrate was diluted with dry DCM (5 ml) and a portion of isocyanate polymer bound (230 mg) was added. The resulting suspension was stirring at r.t. overnight. Then the resin was filtered off and the mixture was concentrated *in vacuo* to obtain the title compound a as crude.
MS (ES/+): m/z=483 [M+H]⁺.

Following the same procedure described for intermediate 28 , intermediates 29-32 as a crude were obtained.

### Intermediate 29

### 1,1 -dimethylethyl 4-(3-[[(3,5-dimethylphenyl)methyl)methyl)aminol-1-(4-fluorophenyl)-3-oxopropyl]-1-piperidinecarboxylate

Starting from [(3,5-dimethylphenyl)methyl]methylamine hydrochloride (87 mg).
MS (ES/+): m/z=611 [M +H]⁺.

### Intermediate 30

### 1,1-dimethylethyl 4-[3-[[(3,4-dibromophenyl)methyl](methyl)amino]-1-(4-fluorophenyl)-3-oxopropyl]-1-piperidinecarboxylate

Starting from [(3,4-dibromophenyl)methyl]methylamine (131 mg).
MS (ES/+): m/z=563 [M +H]⁺.

### Intermediate 31

### 1,1-dimethylethyl 4-[3-[[(3-fluoro-2-methylphenyl)methyl](methyl)amino]-1-(4-fluorophenyl)-3-oxopropyl]-1-piperidinecarboxylate

Starting from [(3-fluoro-2-methylphenyl)methyl]methylamine (89 mg).
MS (ES/+): m/z=487 [M +H]⁺.

### Intermediate 32

### 1.1-dimethylethyl 4-(3-[{[2-chloro-3-(trifluoromethyl)phenyl]methyl}(methyl)amino]-1-(4-fluorophenyl)-3-oxopropyl]-1-piperidinecarboxylate

Starting from {[2-chloro-3-(trifluoromethyl)phenyl]methyl}methylamine hydrochloride (122 mg).
MS (ES/+): m/z=563 [M +H]⁺.

### Intermediate 33

**1,1-dimethylethyl 4-[3-[{(1*R*)-1-[3,5-bis(trifluoromethyl)phenyl]ethyl)(methyl)aminol-1-(4-fluorophenyl)-3-oxopropyl]-4-fluoro-1-piperidinecarboxylate (33a) (diastereoisomer 1)and 1,1-dimethylethyl 4-[3-[{(1*R*)-1-[3,5-bis(trifluoromethyl)phenyl]ethyl}(methyl)amino]-1-(4-fluorophenyl)-3-oxopropyl]-4-fluoro-1-piperidinecarboxylate (33b) (diastereoisomer 2)DIPEA** (0.08 mL) and O-(benzotriazol-1-yl)-N,N,N'N'-tetramethyluronium tetrafluoroborate (0.092 g) were added to a solution of intermediate 22 (0.08 g) in anhydrous DMF (4 mL) under a Nitrogen atmosphere. After stirring for 20 minutes, (1*R*)-1-[3,5-bis(trifluoromethyl)phenyl]-*N*-methylethanamine (0.065 g) was added. The mixture was stirred at r.t. overnight, then it was taken up with AcOEt (30 mL) and washed with water (4 x 5 mL) and brine (2 x 5 mL). The organic layer was dried, concentrated *in vacuo* and the residue was purified by flash chromatography (CH/AcOEt 7/3) to give:
intermediate 33a (0.053 g) as a white foam;
intermediate 33b (0.058 g) as a white foam.

### Intermediate 33a

T.I.c.: CH/AcOEt 6:4, Rf=0.4 (detection with ninhydrine).
MS (ES/+): m/z= 645 [M+Na]⁺.

### Intermediate 33b

T.I.c.: CH/AcOEt 6:4, Rf=0.35 (detection with ninhydrine).
MS (ES/+): m/z= 645 [M+Na]⁺.

### Intermediate 34

**1,1-dimethylethyl 4-[3-[{(1*S*)-1-[3,5-bis(trifluoromethyl)phenyl]ethyl}(methyl)amino]-1-(4-fluorophenyl)-3-oxopropyl]-4-fluoro-1-piperidinecarboxylate (34a) (diastereoisomer 1)and1,1-dimethylethyl 4-[3-[{(1*S*)-1-[3,5-bis(trifluoromethyl)phenyl]ethyl)(methyl)amino]-1-(4-fluorophenyl)-3-oxopropyl]-4-fluoro-1-piperidinecarboxylate (34b) (diastereoisomer 2)** DIPEA (0.08 mL) and O-(benzotriazol-1-yl)-N,N,N'N'-tetramethyluronium tetrafluoroborate (0.092 g) were added to a solution of intermediate 22 (0.08 g) in anhydrous DMF (4 mL) under a Nitrogen atmosphere. After stirring for 20 minutes, [1-(S)-(3,5-bis-trifluoromethyl-phenyl)-ethyl]-methyl-amine (0.065 g) was added. The mixture was stirred at r.t. overnight, then it was taken up with AcOEt (30 mL) and washed with water (4 x 5 mL) and brine (2 x 5 mL). The organic layer was dried, concentrated *in vacuo* and the residue was purified by flash chromatography (CH/AcOEt 7/3) to give:
intermediate 34a (0.054 g) as a white foam;
intermediate **34b** (0.056 g) as a white foam.

### Intermediate 34a

T.I.c.: CH/AcOEt 6:4, Rf=0.4 (detection with ninhydrine).
MS (ES/+): m/z= 645 [M+Na]⁺.

### Intermediate 34b

T.l.c.: CH/AcOEt 6:4, Rf=0.35 (detection with ninhydrine).
MS (ES/+): m/z= 645 [M+Na]⁺.

### Intermediate 35

### 1,1-dimethylethyl 4-fluoro-4-[(4-fluorophenyl)carbonyl]-piperidinecarboxylate

A solution of Lithyum bis(trimethylsilyl)-amide 1 M in THF (13.8 mL) was dropped into a solution of intermediate 10 (2.637 g) dissolved in anhydrous THF (170 mL) previously cooled to -78°C under a Nitrogen atmosphere. At the end of the addition the mixture was stirred for 15 min. and then allowed to warm to -10°C and stirred for 1.5 hours. Then the mixture was cooled to -40°C and N-Fluorobenzene-sulfonimide (3.53 g) dissolved in anhydrous THF (28 mL) was added. The mixture was allowed to warm to r.t. in 1.5 hours, then it was treated with ammonium chloride saturated solution. AcOEt was added, the organic layer was washed with brine, dried and concentrated *in vacuo* to a residue which was purified by flash chromatography (from CH to CH/AcOEt 9:1) to give the title compound (2.15 g) as colourless oil???
T.I.c.: CH/AcOEt 9:1, Rf=0.29 (detection with ninhydrine).
MS (ES/+): m/z=270 [M-tBut +H]⁺.

### Intermediate 36

### 1,1-dimethylethyl 4-[(1E)-3-(ethyloxy)-1-(4-fluorophenyl)-3-oxo-1-propen-1-yl]-4-fluoro-1-piperidinecarboxylate

Triethylphosphono Acetate (5.2 mL) was dropped into a sospension of NaH (60% in mineral oil 1.05 g) in anhydrous THF (66 mL) previously cooled to 0°C under a Nitrogen atmosphere. At the end of the addition the mixture was allowed to warm to r.t. and stirred for 20 minutes. Then intermediate 35 (2.15 g) dissolved in anhydrous THF (66 mL) was added and the mixture was stirred at 70°. After 1 hour the mixture was allowed to reach r.t. and was treated with water and extracted with AcOEt. The organic layer was dried and concentrated *in vacuo* to a residue which was purified by silica cartridge (from CH/AcOEt 9:1 to CH/AcOEt 8:2) to give the title compound (2.0 g) as a white solid.T.I.c.: CH/AcOEt 8:2, Rf=0.43 (detection with ninhydrine).
MS (ES/+): m/z=418 [M+Na]⁺.

### Intermediate 37

### 1,1-dimethylethyl 4-[3-(ethyloxy)-1-(4-fluorophenyl)-3-oxopropyl]-4-fluoro-1-piperidinecarboxylate

PtO2 (460 mg) was added to a solution of intermediate 36 (2.0 g) in anhydrous EtOH (180 mL) under a Nitrogen atmosphere. The reaction mixture was stirred under Hydrogen atmosphere overnight. Then further PtO2 (500 mg) was added and the reaction mixture was stirred under Hydrogen atmosphere for 4 hours. It was filtered over a celte pad washing with AcOEt and concentrated *in vacuo* to a residue which was purified by flash chromatography (CH/AcOEt 8:2) to give the title compound (1.8 g) as a yellow oil.
T.I.c.: CH/AcOEt 8:2, Rf=0.33 (detection with ninhydrine).
MS (ES/+): m/z=420 [M+Na]⁺.

### Intermediate 38

### 1,1-dimethylethyl 4-[3-[[(3,5-dibromophenyl)methyl](methyl)amino]-1-(4-fluorophenyl)-3-oxopropyl]-4-fluoro-1-piperidinecarboxylate

DIPEA (704 µL) and O-(benzotriazol-1-yl)-N,N,N'N'-tetramethyluronium tetrafluoroborate (563 mg) were added to a solution of intermediate 22 (500 mg) in anhydrous DMF (15 mL) under a Nitrogen atmosphere. After stirring for 10 minutes, 1-(3,5-dibromophenyl)-*N-*methylmethanamine (411 mg) was added. The mixture was stirred at r.t. overweekend. Then further DIPEA (188 µL), TBTU (151 mg) and after 10 min. 1-(3,5-dibromophenyl)-*N-*methylmethanamine (112 mg) were added and the mixture was stirred for additional 3 hours. It was diluted with water/ice and extracted with AcOEt. The organic layer was dried, concentrated *in vacuo* and the residue was purified by flash chromatography (CH/AcOEt from 8:2 to 7:3) to give the title compound (765 mg) as a white foam.T.l.c.: CH/AcOEt 6:4, Rf=0.42 (detection with ninhydrine).
MS (ES/+): m/z=651 [M+Na]⁺.

### Intermediate 39

### 1,1-dimethylethyl 4-[(2,4-dichlorophenyl)(hydroxy)methyl]-1-piperidinecarboxylate

A solution of 2,4-dichloroiodobenzene (2.23 mL) in anhydrous Et2O (8 mL) was dropped into a suspension of magnesium turning (0.395 g) and few crystals of iodine in anhydrous Et2O (8 mL) under a Nitrogen atmosphere. The mixture was refluxed for 30 minutes, then it was allowed to cool to r.t. then cooled to 0°C and a mixture of intermediate 3 (1.3 g) in anhydrous Et2O (8 mL) was added dropwise. At the end of the addition the mixture was allowed to warm to r.t. and stirred at 23°C for 30 minutes. The mixture was quenched with ammonium chloride saturated solution, stirred for 10 minutes then extracted with DCM (3 x 50 mL). The combined organic extracts were concentrated *in vacuo.* The residue was purified by flash chromatography (CH/AcOEt from 95:5 to 8:2) to give the title compound (0.9 g) as a white foam.
T.I.c.: CH/AcOEt 75:25, Rf=0.35
MS (ES/+): m/z=360 [M+H]⁺.

### Intermediate 40

### 1,1-dimethylethyl 4-[(2,4-dichlorophenyl)carbonyl]-1-piperidinecarboxylate

A solution of mixture of intermediate 39 (0.67 g) was dissolved in anhydrous DCM (11 mL) and treated portionwise with Dess Martin periodinane (0.79 g) under a Nitrogen atmosphere. The brown solution was stirred at r.t. for 1.5 hours, then the solution was diluted with DCM (30 mL) and poured into a saturated sodium hydrogen carbonate solution (50 mL) containing sodium thiosulphate (2.5 g). The mixture was stirred for 30 minutes, then the phases were separated. The organic layer was washed with a saturated sodium hydrogen carbonate solution. The organic layer was dried and concentrated *in vacuo* to a residue which was purified by flash chromatography (CH/AcOEt (95:5 to 75:15) to give the title compound (0.47 g) as a colourless oil.
T.I.c.: CH/AcOEt 7:3, Rf=0.53 (detection with ninhydrine).
MS (ES/+): m/z=358 [M+H]⁺.

### Intermediate 41

### 1,1-dimethylethyl 4-[(2,4-dichlorophenyl)carbonyl-4-fluoro-1-piperidinecarboxylate

A solution of Lithyum bis(trimethylsilyl)-amide 1 M in THF (5.1 mL) was dropped into a solution of intermediate 40 (1.14 g) dissolved in anhydrous THF (64 mL) previously cooled to -78°C under a Nitrogen atmosphere. After 15 min. the mixture was allowed to warm to - 10°C and stirred for 1.5 hours. The mixture was cooled to -40°C and N-Fluorobenzene-sulfonimide (1.31 g) dissolved in anhydrous THF (11 mL) was added. The mixture was allowed to stir to r.t. for 1.5 hours and then treated with ammonium chloride saturated solution. AcOEt was added, the organic layer was washed with brine, dried and concentrated *in vacuo* to a residue which was purified by flash chromatography (from CH to CH/AcOEt 9:1) to give the title compound (985 mg).
T.I.c.: CH/AcOEt 7:3, Rf=0.58 (detection with ninhydrine).
MS (ES/+): m/z=398 [M+Na]⁺.

### Intermediate 42

### 1,1-dimethylethyl 4-[1-(2,4-dichlorophenyl)-3-(ethyloxy)-3-oxo-1-propen-1-yl]-4-fluoro-1-piperidinecarboxylate

Triethylphosphono Acetate (2.1 mL) was dropped into a sospension of NaH (60% in mineral oil, 420 mg) in anhydrous THF (26 mL) previously cooled to 0°C under a Nitrogen atmosphere. At the end of the addition the mixture was allowed to warm to r.t. and stirred for 20 minutes. Then intermediate 41 (985 mg) dissolved in anhydrous THF (26 mL) was added and the mixture was stirred at 65°C for 1.5 hours and then at 70°C for an additional hour. The mixture was allowed to reach r.t., treated with water and extracted with AcOEt. The organic layer was dried and concentrated *in vacuo* to a residue which was purified by silica cartridge (CH/AcOEt 9:1) to give the title compound (1.1 g) as a mixture of isomers E/Z 70:30.
T.I.c.: CH/AcOEt 9:1, Rf=0.20 (detection with ninhydrine).
MS (ES/+): m/z=468 [M+Na]⁺.

### Intermediate 43

### 1,1-dimethylethyl 4-[1-(2,4-dichlorophenyl)-3-(ethyloxy)-3-oxopropyl]-4-fluoro-1-piperidinecarboxylate

PtO2 (130 mg) was added to a solution of intermediate 42 (850 mg) in anhydrous EtOH (70 mL) under a Nitrogen atmosphere. The reaction mixture was stirred under Hydrogen atmosphere for 2 hours. Then further PtO2 (130 mg x 3) was added and the reaction mixture was stirred under Hydrogen atmosphere for 13 hours overall. It was filtered over a celte pad and concentrated *in vacuo* to a residue which was purified by flash chromatography (CH/AcOEt 8:2) to give the title compound (610 mg) as a yellow oil.
T.I.c.: CH/AcOEt 7:3, Rf=0.75 (detection with ninhydrine).
MS (ES/+): m/z=470 [M+Na]⁺.

### Intermediate 44

### 3-(2,4-dichlorophenyl)-3-(1-{[(1,1-dimethylethyl)oxy]carbonyl}-4-fluoro-4-piperidinyl)propanoic acid

A solution of lithium hydroxide monohydrate (285 mg) in water (4.5 mL) was added to a solution of intermediate 43 (610 mg) in MeOH (18.5 mL). The mixture was heated to 70°C for 1 hour, then it was allowed to cool to r.t., diluted with AcOEt/H₂O, acidified to pH 4 with a pH3 buffer solution (citrate-hydrochloric acid buffer solution purchase from Fluka) and 1N hydrochloric acid solution. The aqueous layer was extracted with further AcOEt (3 x 100 mL). The organic extract was dried and concentrated *in vacuo* to give the title compound (574 mg) as a white solid.
MS (ES/-): m/z=418 [M-H]⁻.

### Intermediate 45

### 1,1-dimethylethyl 4-[3-[{(1R)-1-[3,5-bis(trifluoromethyl)phenyllethyl)(methyl)amino]-1-(2,4-dichlorophenyl)-3-oxopropyl]-4-fluoro-1-piperidinecarboxylate (45a) (Diastereoisomer 1) and 1,1-dimethylethyl 4-[3-[{(1R)-1-[3,5-bis(trifluoromethyl)phenyl]ethyl)(methyl)amino]-1-(2,4-dichlorophenyl)-3-oxopropyl]-4-fluoro-1-piperidinecarboxylate (45b) (diastereoisomer 2)

DIPEA (80 µL) and O-(benzotriazol-1-yl)-N,N,N'N'-tetramethyluronium tetrafluoroborate (100 mg) were added to a solution of intermediate 44 (95 mg) in anhydrous DMF (3 mL) under a Nitrogen atmosphere. After stirring for 10 minutes, {(1*R*)-1-[3,5-bis(trifluoromethyl)phenyl]ethyl}methylamine (68 mg) was added. The mixture was stirred at r.t. overweekend, then it was diluted with water (5 mL) and extracted with AcOEt (10 mL). The organic layer was dried, concentrated *in vacuo* and the residue was purified by flash chromatography (CH/AcOEt 8:2) to give:
intermediate 45a (51 mg) as a white foam;
intermediate 45b (57 mg) as a white foam

### Intermediate 45a

T.I.c.: CH/AcOEt 8:2, Rf=0.54.
NMR (d₁-CDCl₃): δ (ppm): 7.78 (s, 1 H); 7.62 (s, 2H); 7.39 (s, 1 H); 7.26 (d, 1H); 7.18 (dd, 1 H); 6.01 (q, 1 H); 4.15 (dm, 1H); 4.02 (bm, 1H); 3.87 (bm, 1H); 3.05 (dd, 1 H); 2.92 (bm, 1 H); 2.73 (dd, 1H); 2.65-2.75 (bm, 1H); 2.68 (s, 3H); 1.94 (bm, 1H); 1.68 (m, 1H); 1.62 (m, 1 H); 1.48 (m, 1H); 1.43 (s, 9H); 1.37 (d, 3H).

### Intermediate 45b

T.I.c.: CH/AcOEt 8:2, Rf=0.45.
MS (ES/+): m/z=695 [M+Na]⁺.

### Intermediate 46

### 1,1-dimethylethyl 4-[2-cyano-3-(ethyloxy)-1-(4-fluoro-2-methylphenyl)-3-oxopropyl]-4-fluoro-1-piperidinecarboxylate

A solution of 2-bromo, 5-fluorotoluene (2.26 mL) in anhydrous Et2O (6.5 mL) was dropped into a suspension of magnesium turning (0.390 g) and few crystals of iodine in anhydrous Et2O (1.6 mL) under a Nitrogen atmosphere. The mixture refluxed for 30 minutes, then it was allowed to cool to r.t.. 2.0 ml of this solution were added drop-wise to intermediate 19 (595 mg) in anhydrous Et2O (10 mL). At the end of the addition the mixture was stirred for 2 hours at r.t.. The mixture was quenched with ammonium chloride saturated solution and extracted with AcOEt. The aqueous layer was extracted with further AcOEt. The combined organic extracts were dried and concentrated *in vacuo* to a residue which was purified by flash chromatography (CH/AcOEt 8:2) to give the title compound (400 mg).
T.I.c.: CH/AcOEt 7:3, Rf=0.30 (detection with ninhydrine).
MS (ES/+): m/z=459 [M+Na]⁺.

### Intermediate 47

### 1,1-dimethylethyl 4-[2-cyano-1-(4-fluoro-2-methylphenyl)ethyl]-4-fluoro-1-piperidinecarboxylate

A mixture of intermediate 46 (165 mg), sodium chloride (9 mg) and water (15 µL) in anhydrous dimethylsulfoxide (2.5 mL) was heated to 160°C for 2 hours. The mixture was allowed to cool to r.t., diluted with AcOEt and washed with water and brine. The aqueous layer was extracted with further AcOEt (3 x 20 mL). The organic layer was dried and concentrated *in vacuo.* The residue was purified by flash chromatography (CH/AcOEt 80:20) to give the title compound (102 mg) as a colourless oil.
MS (ES/+): m/z= 387 [M+Na]⁺.

### Intermediate 48

### 3-(1-{[(1.1-dimethylethyl)oxy]carbonyl)-4-fluoro-4-piperidinyl)-3-(4-fluoro-2-methylphenyl)propanoic acid

A 3M potassium hydroxide solution (4.6 mL) was added to a solution of intermediate 47 (99 mg) in abs. EtOH (4.6 mL). The mixture divided into two portions which were heated in microwave (300W, P=90p.s.i.) at 150°C for (10+10+10+5) minutes. Each solution was allowed to cool to r.t., diluted with AcOEt/H₂O, acidified to pH 4/5 with a pH3 buffer solution (citrate-hydrochloric acid buffer solution purchase from Fluka) and 2N hydrochloric acid solution. The aqueous layer was extracted with further AcOEt. The organic extract was dried and concentrated *in vacuo* to give the title compound (58 mg) as a white solid.
MS (ES/-): m/z=382 [M-1]⁻.

### Intermediate 49

### 1,1-dimethylethyl 4-[3-[{(1R)-1-[3,5-bis(trifluoromethyl)phenyl]ethyl}{methyl)amino]-1-(4-fluoro-2-methylphenyl)-3-oxopropyl]-4-fluoro-1-piperidinecarboxylate (49a) (diastereoisomer 1)

### 1.1-dimethylethyl 4-(3-[{(1R)-1-(3,5-bis(trifluoromethyl)phenyllethyl}(methyl)amino]-1-(4-fluoro-2-methylphenyl)-3-oxopropyl]-4-fluoro-1-piperidinecarboxylate (49b) (diastereoisomer 2)

DIPEA (37 µL) and O-(benzotriazol-1-yl)-N,N,N'N'-tetramethyluronium tetrafluoroborate (57 mg) were added to a solution of intermediate 48 (53 mg) in anhydrous DMF (4 mL) under a Nitrogen atmosphere. After stirring for 15 minutes (1*R*)-1-[3,5-bis(trifluoromethyl)phenyl]-*N*-methylethanamine (45 mg) was added. The mixture was stirred at r.t. overnight, then it was diluted with AcOEt (15 mL) and washed with water (3 x 15 mL). The organic layer was dried, concentrated *in vacuo* and the residue was purified by flash chromatography (CH/AcOEt from 95:5 to 75:25) to give:
intermediate **49a** (23 mg) as colourless oil;
intermediate **49b** (36mg) as colourless oil.

### Intermediate 49a

T.I.c.: CH/AcOEt 1:1, Rf=0.80 (detection with ninhydrine).
MS (ES/+): m/z=659 [M+Na]⁺, 561 [M-tBu-F+H]⁺, 517 [M-BOC-F+H]⁺.

### Intermediate 49b

T.I.c.: CH/AcOEt 1:1, Rf=0.64 (detection with ninhydrine).
MS (ES/+): m/z=659 [M+Na]⁺, 561 [M-tBu-F+H]⁺, 517 [M-BOC-F+H]⁺.

### Intermediate 50

### 1,1-dimethylethyl 4-[3-[[(3,5-dibromophenyl)methyl](methyl)amino]-1-(4-fluoro-2-methylphenyl)-3-oxopropyl]-4-fluoro-1-piperidinecarboxylate

DIPEA (74 µL) and O-(benzotriazol-1-yl)-N,N,N'N'-tetramethyluronium tetrafluoroborate (58 mg) were added to a solution of intermediate 48 (53 mg) in anhydrous DMF (3 mL) under a Nitrogen atmosphere. After stirring for 15 minutes intermediate [(3,5-dibromophenyl)methyl]methylamine (52 mg) was added. The mixture was stirred at r.t. overnight, then it was diluted with AcOEt (15 mL) and washed with water (4 x 15 mL). The organic layer was dried, concentrated *in vacuo* and the residue was purified by flash chromatography (CH/AcOEt from 95:5 to 75:25) to give the title compound (38 mg) as a colourless oil.
T.I.c.: CH/AcOEt 1:1, Rf=0.37 (detection with ninhydrine).

### Intermediate 51

**1 1-dimethylethyl 4-[(3,4-dichlorophenyl)carbonyl)-1-piperidinecarboxylate A** solution of 4-bromo-l,2-dichlorobenzene (2.44 mL) in anhydrous THF (2.5 mL) was dropped into a suspension of magnesium turning (0.444 mg) and few crystals of iodine in anhydrous THF (2.5 mL) under a Nitrogen atmosphere. The mixture was refluxed for 30 minutes and then cooled to 0°C. A solution of intermediate 3 (1.5 g) in anhydrous THF (5.0 mL) was dropped into the reaction mixture at 0°C under a Nitrogen atmosphere while stirring. At the end of the addition the mixture was allowed to warm to r.t. and stirred at 23°C for 45 minutes. The mixture was diluted with DCM (20 mL), washed with a saturated ammonium chloride solution (3 x 5 mL). The organic layer was dried and concentrated *in vacuo* to give 1,1-dimethylethyl 4-[(3,4-dichlorophenyl)(hydroxy)methyl]-1-piperidinecarboxylate (1.43 g)
T.I.c.: CH/AcOEt 75:25, Rf=0.23).
This material was dissolved in anhydrous DCM (23 mL) and treated portion-wise with Dess Martin periodinane (1.67 g) under a Nitrogen atmosphere. The brown solution was stirred at r.t. for 1.5 hours, then the solution was diluted with DCM (40 mL) and poured into a 5% solution of sodium thiosulphate in saturated sodium hydrogen solution (100 mL). The mixture was stirred for 30 minutes, then the phases were separated and the aqueous phase washed with DCM (2 x 10 mL). The collected organic layers were dried and concentrated *in vacuo* to give the title compound (1.41 g) as a white solid.
T.l.c.: CH/AcOEt 75:25, Rf=0.32 (detection with ninhydrine).

### Intermediate 52

### 1,1-dimethylethyl 4-[(3,4-dichlorophenyl)carbonyl]-4-fluoro-1-piperidinecarboxylate

Lithium bis(trimethylsilyl)amide (1M in THF - 6.30 mL) was added drop-wise to a stirred solution of intermediate 51 (1.41 g) in anhydrous THF (72 mL) previously cooled to -78°C under a Nitrogen atmosphere. After stirring at -78°C for 15 minutes and then at -10°C for 1.5 hours, the mixture was cooled to -40°C and a solution of N-fluorobenzenesulfonimide (1.614 g) in anhydrous THF (15 mL) added drop-wise. The mixture was stirred at -40°C for 30 minutes, then allowed to reach r.t. and stirred at 23°C for 75 minutes. The mixture, cooled to 0°C, was diluted AcOEt, washed with a saturated ammonium chloride solution, the aqueous phase extracted with AcOEt and the collected organic phases dried and concentrated *in vacuo.* The residue was purified by flash chromatography (CH, CH/AcOEt from 95:5 to 80:20) to give the title compound (1.09 g) as a white solid.
T.Lc.: CH/AcOEt 75:25, Rf=0.52 (detection with ninhydrine).
MS (ES/+): m/z=396 [M+Na]⁺.

### Intermediate 53

### 1,1-dimethylethyl 4-([(1E)-1-(3,4-dichlorophenyl)-3-(ethyloxy)-3-oxo-1-propen-1-yl]-4-fluoro-1-piperidinecarboxylate

Triethylphosphonoacetate (2.37 mL) was added to a suspension of sodium hydride (60% suspension in mineral oil - 477.5 mg) in anhydrous THF (25 mL) previously cooled to 0°C under a Nitrogen atmosphere. The mixture was allowed to warm to r.t. and stirred at 23°C for 20 minutes. Then, a solution of intermediate 52 (1.123 g) in anhydrous THF (25 mL) was added drop-wise and the resulting solution was heated to reflux for 30 minutes. The solution was allowed to cool to r.t., diluted with AcOEt and water. The aqueous phase was extracted with AcOEt, the collected organic phases washed with brine, dried and concentrated *in vacuo* to a residue, which was purified by flash chromatography (CH. CH/AcOEt from 9:1 to 8:2) to give the title compound (1.28 g) as a white solid.
T.I.c.: CH/AcOEt 9:1, Rf=0.31 (detection with ninhydrine).
MS (ES/+): m/z=368 [M+Na]⁺.

### Intermediate 54

### 1,1-dimethylethyl 4-[1-(3.4-dichlorophenyl)-3-(ethyloxyl)-3-oxopropyl)-4-fluoro-1-Piperldinecarboxylate

PtO2 (194 mg) was added to a solution of intermediate 53 (1.27 g) in abs. EtOH (100 mL) under a Nitrogen atmosphere. The reaction mixture was stirred under Hydrogen atmosphere (1 atm) for 2 hours. The reaction mixture was filtered over a celite pad washing with MeOH and concentrated *in vacuo* to give the title compound (1.28 g) as a yellow oil.
T.I.c.: CH/AcOEt 7:3, Rf=0.38 (detection with ninhydrine).
MS (ES/+): m/z=470 [M+Na]⁺.

### Intermediate 55

### 3-(3,4-dichlorophenyl)-3-(1-{[(1,1-dimethylethyl)oxy]carbonyl}-4-fluoro-4-piperidinyl)propanoic acid

A solution of lithium hydroxide monohydrate (597 mg) in water (8.5 mL) was added to a solution of intermediate 54 (1.27 g) in MeOH (34 mL). The mixture was heated to 80°C for 1 hour, then it was allowed to cool to r.t., diluted with water and concentrated to half volume. The residue was acidified with 3M hydrochloric acid solution and extracted with AcOEt. The organic layer was dried and concentrated *in vacuo* to give the title compound (1.204 g) as a white foam.
MS (ES/+): m/z=442 [M+Na]⁺.

### Intermediate 56

### 1 1-dimethylethyl 4-[3-[[(3,5-dibromophenyl)methyl](methyl)amino]-1-(3,4-dichlorophenyl)-3-oxopropyl]-4-fluoro-1-piperidinecarboxylate

DIPEA (124 µL) and O-(benzotriazol-1-yl)-N,N,N'N'-tetramethyluronium tetrafluoroborate (99 mg) were added to a solution of intermediate 55 (100 mg) in anhydrous DMF (6 mL) under a Nitrogen atmosphere. After stirring for 15 minutes intermediate [(3,5-dibromophenyl)methyl]methylamine (90 mg) was added. The mixture was stirred at r.t. overnight, then it was diluted with AcOEt (20 mL) and washed with water (4 x 20 mL). The organic layer was dried, concentrated *in vacuo* and the residue was purified by flash chromatography (CH/AcOEt from 95:5 to 75:25) to give the title compound (135 mg) as a colourless oil.
T.I.c.: CH/AcOEt 1:1, Rf=0.50 (detection with ninhydrine).
MS (ES/+): m/z=704 [M+Na]⁺; 605, 607 [M-tBu-F+H]⁺.

### Intermediate 57

### 1,1-dimethylethyl 4-[3-({[3,5-bis(trifluoromethyl)phenyl]methyl}(methyl)amino]-1-(4-fluorophenyl)-3-oxopropyl]-4-fluoro-1-piperidinecarboxylate

DIPEA (0.430 mL) and O-(benzotriazol-1-yl)-N,N,N'N'-tetramethyluronium tetrafluoroborate (339 mg) were added to a solution of intermediate 22 (300 mg) in anhydrous DMF (10 mL) under a Nitrogen atmosphere. After stirring for 10 minutes, {[3,5-bis(trifluoromethyl)phenyl]methyl}methylamine hydrochloride (286.1 mg) was added. The mixture was stirred at r.t. for 2 days, then it was diluted with water (10 mL) and extracted with AcOEt (20 mL). The organic layer was washed with brine (10 mL), dried, concentrated *in vacuo* and the residue was purified by flash chromatography (CH/AcOEt 6:4) to give the title compound (440 mg) as a yellow oil.
T.I.c.: CH/AcOEt 1:1, Rf=0.52 (detection with ninhydrine).
MS (ES/+): m/z=609 [M+H]⁺.

### Intermediate 58

### 1,1-dimethylethyl 4-[(4-chlorophenyl)(hydroxy)methyl]-1-piperidinecarboxylate

A solution of 4-chlorobromobenzene (2.47 mL) in anhydrous THF (5 mL) was dropped into a suspension of magnesium turning (0.304 g) and few crystals of iodine in anhydrous THF (5 mL) under a Nitrogen atmosphere. The mixture refluxed for 30 minutes, then it was allowed to cool to r.t. and a mixture of intermediate 3 (1.0 g) in anhydrous THF (5 mL) was added drop-wise. At the end of the addition the mixture was allowed to warm to r.t. and stirred at 23°C for 45 minutes. The mixture was quenched with ammonium chloride saturated solution, stirred for 10 minutes then extracted with DCM (3 x 50 mL). The combined organic extracts were concentrated *in vacuo.* The residue was purified by flash chromatography (CH/AcOEt 95:5 to 8:2) to give the title compound (1.33 g) as a white foam.
T.I.c.: CH/AcOEt 75:25, Rf=0.31
MS (ES/+): m/z=326 [M+H]⁺.

### Intermediate 59

### 1,1-dimethylethyl 4-[(4-chlorophenyl)carbonyl]-1-piperldinecarboxylate

A solution of mixture of intermediate 58 (1.33 g) dissolved in anhydrous DCM (25 ml) was treated portion-wise with Dess Martin periodinane (1.74 g) under a Nitrogen atmosphere. The brown solution was stirred at r.t. for 1.5 hours, then the solution was diluted with DCM (50 mL) and poured into a saturated sodium hydrogen carbonate solution (50 mL) containing sodium thiosulphate (2.5 g). The mixture was stirred for 30 minutes, then the phases were separated. The organic layer was washed with a saturated sodium hydrogen carbonate solution. The organic layer was dried and concentrated *in vacuo* to give the title compound (1.18 g) as a white solid.
T.I.c.: CH/AcOEt 75:25, Rf=0.5 (detection with ninhydrine).
MS (ES/+): m/z=324 [M+H]⁺.

### Intermediate 60

### 1,1-dimethylethyl 4-[(4-chlorophenyl)carbonyl]-4-fluoro-1-piperidinecarboxytate

A solution of Lithyum bis(trimethylsilyl)-amide (1M in THF - 5.84 mL) was dropped into a solution of intermediate 59 (1.18 g) dissolved in anhydrous THF (67 mL) previously cooled to -78°C under a Nitrogen atmosphere. At the end of the addition the mixture was allowed to warm to 0°C and stirred for 1.5 hours. Then the mixture was cooled to -40°C and N-Fluorobenzene-sulfonimide (1.49 g) dissolved in anhydrous THF (14 mL) was added dropwise. Then the mixture was allowed to stirr to r.t. in about 2 hours and it was treated with ammonium chloride saturated solution and extracted with AcOEt. The organic layer was dried and concentrated *in vacuo* to a residue which was purified by flash chromatography (CH/AcOEt 95:5 to 75:15) to give the title compound (1.14 g).
T.I.c.: CH/AcOEt 7:3, Rf=0.66 (detection with ninhydrine).
MS (ES/+): m/z=286 [M-tBut+H]⁺.

### Intermediate 61

### 1,1-dimethytethyl 4-[1-14-chlorophenyl)-3-(ethyloxy)-3-oxo-1-propen-1-yl]-4-fluoro-1-piperidinecarboxylate

Triethylphosphono Acetate (0.46 mL) was dropped into a sospension of NaH (60% in mineral oil, 0.093 g) in anhydrous THF (5 mL) previously cooled to 0°C under a Nitrogen atmosphere. At the end of the addition the mixture was allowed to warm to r.t. and stirred for 20 minutes. Then intermediate **60** (0.20 g) dissolved in anhydrous THE (5 mL) was added and the mixture was stirred at reflux temperature. After 1 hour the mixture was allowed to reach r.t. and was treated with water and extracted with Et2O. The organic layer was dried and concentrated *in vacuo* to a residue which was purified by flash chromatography (CH/AcOEt from 95:5 to 9:1) to give the title compound (0.21 g) as a mixture E/Z and as a white solid.
T.I.c.: CH/AcOEt 7:3, Rf=0.61 (detection with ninhydrine).
MS (ES/+): m/z=412 [M+H]⁺; 434 [M+Na]⁺.

### Intermediate 62

### 1,1-dimethylethyl 4-[1-(4-chlorophenyl)-3-(ethyloxy)-3-oxopropyl]-4-fluoro-1-piperidinecarboxylate

PtO₂ (35 mg) was added to a solution of intermediate 61 (210 mg) in anhydrous EtOH (20 mL) under an hydrogen pressure of 1 atmosphere for 3 hours. Then, the reaction mixture was filtered over a celite pad washing with MeOH and concentrated *in vacuo* to give the title compound (208 mg) as a colourless oil.
T.I.c.: CH/AcOEt 8:2, Rf=0.41 (detection with ninhydrine).
MS (ES/+): m/z=414 [M+H]⁺.

### Intermediate 63

### 3-(4-chlorophenyl)-3-(1-{[(1,1-dimethylethyl)oxy]carbonyl)-4-fluoro-4-piperidinyl)propanoic acid

A solution of lithium hydroxide monohydrate (0.6 g) in water (8.5 mL) was added to a solution of intermediate 62 (1.19 g) in MeOH (34 mL). The mixture was heated to 65°C for 1 hour, then it was allowed to cool to r.t., acidified with 1 M hydrochloric acid solution to pH 3 and extracted with DCM (3 × 100 mL). The organic layer was washed with water, dried and concentrated *in vacuo* to give the title compound (1.06 g) as a white foam.
MS (ES/+): m/z=408 [M+Na]⁺.

### Intermediate 64

### 1,1-dimethylethyl 4-{1-(4-chlorophenyl)-3-[[(3,5-dibromophenyl)methyl](methyl)amino]-3-oxopropyl)-4-fluoro-1-piperidinecarboxylate

DIPEA (136 µL) and O-(benzotriazol-1-yl)-N,N,N'N'-tetramethyluronium tetrafluoroborate (108 mg) were added to a solution of intermediate 63 (100 mg) in anhydrous DMF (3 mL) under a Nitrogen atmosphere. After stirring for 15 minutes intermediate [(3,5-dibromophenyl)methyl]methylamine (98 mg) was added. The mixture was stirred at r.t. overnight, then it was diluted with AcOEt (15 mL) and washed with water (4 x 15 mL). The organic layer was dried, concentrated *in vacuo* and the residue was purified by flash chromatography (CH/AcOEt from 95:5 to 75:25) to give the title compound (120 mg) as a colourless oil.
T.l.c.: CH/AcOEt 1:1, Rf=0.64 (detection with ninhydrine).
MS (ES/+): m/z= 645 [M +H]⁺.

### Intermediate 65

### 1-(1,1-dimethylethyl) 3-ethyl 1,3-piperidinedicarboxylate

TEA (10.4 ml) and a solution of di-*tert*-butyl-dicarbonate (6 g) in anhydrous DCM (10 mL) was added to a solution of ethyl 3-piperidinecarboxylate (3.9 mL) in anhydrous DCM (50 mL) under a Nitrogen atmosphere. The solution was stirred at r.t. for 4 hours, then it was washed with brine, dried, concentrated *in vacuo* and the residue was purified by flash chromatography (CH/AcOEt 9:1) to give the title compound (5.92 g) as a pale yellow oil.
T.l.c.: CH/AcOEt 7:3, Rf=0.67 (detection with ninhydrine).

### Intermediate 66

### 1,1-dimethylethyl 3-formyl-1-piperidinecarboxylate

Diisobutylaluminium hydride (1M in toluene - 48.7 mL) was dropped into a solution of intermediate 65 (5.70 g) in anhydrous toluene (75 mL) previously cooled to -78°C under a Nitrogen atmosphere. The solution was stirred at -78°C for 2 hours, then it was quenched with a 10% sodium hydroxide solution (16 mL) at -78°C. The mixture was allowed to warm to r.t. and the layers were separated. The organic layer was washed with a 10% sodium hydroxide solution and brine, dried, concentrated *in vacuo* and the residue was purified by flash chromatography (CH/AcOEt 9:1) to give the title compound (5 g) as colourless oil.
T.Lc.: CH/AcOEt 7:3, Rf=0.35 (detection with ninhydrine).
MS (ES/+): m/z=236 [M+Na]⁺.

### Intermediate 67a and 67b

### 1,1-dimethytethyl 3-[(4-fluorophenyl)(hydroxy)methyl]-1-piperidinecarboxylate (67a) (diastereoisomer A) and 1,1-dimethylethyl 3-[(4-fluorophenyl)(hydroxy)methyl]-1-piperidinecarboxylate (67b) (diastereoisomer B)

A solution of intermediate 66 (2.5 g) in anhydrous Et2O (50 mL) was dropped into a solution of 4-fluorophenyl magnesium bromide (1M in THF - 35 mL) in anhydrous Et2O (35 mL) previously cooled to 0°C under a Nitrogen atmosphere. At the end of the addition the mixture was allowed to warm to r.t. and stirred at 23°C for 30 minutes. The mixture was quenched with a saturated ammonium chloride solution (50 mL). The aqueous layer was further extracted with AcOEt. The combined organic extracts were washed with brine, dried and concentrated *in vacuo* to a residue which was purified by flash chromatography (CH/AcOEt from 9:1 to 7:3) to give a mixture of the title compounds as a yellow oil (2.5 g). Samples of few milligrams of single isomers from purification were characterised:

### Intermediate 67a:

T.I.c.: CH/AcOEt 7:3, Rf=0.3
NMR (d₆-DMSO): δ (ppm): 7.31 (dd, 2H); 7.12 (t, 2H); 5.29 (d, 1 H); 4.25 (t, 1H); 4.05 (mb, 1 H); 3.82 (d, 1H); 2.5 (m, 1H); 1.51-1.18 (m, 6H); 1.34 (s, 9H).
MS (ES/+): m/z=310 [M+H]⁺.

### Intermediate 67b:

T.I.c.: CH/AcOEt 7:3, Rf= 0.28 (detection with ninhydrine).
NMR (d₆-DMSO): δ (ppm): 7.30 (dd, 2H); 7.13 (t, 2H); 5.27 (d, 1 H); 4.28 (sb, 1 H); 3.53 (mb, 1H); 3.77 (d, 1H); 2.60 (t, 1H); 1.85 (sb, 1); 1.61-1.22 (m, 5H); 1.29 (s, 9H).
MS (ES/+): m/z=310 [M+H]⁺.

### Intermediate 68

### 1,1-dimethylethyl 3-[(4-fluorophenyl)carbonyl]-1-piperidinecarboxylate

A solution of mixture of intermediate 67a and 67b (0.1 g) was dissolved in anhydrous DCM (2 mL) and treated portion-wise with Dess Martin periodinane (0.14 g) under a Nitrogen atmosphere. The brown solution was stirred at r.t. for 3 hours, then the solution was diluted with DCM (5 mL) and poured into a saturated sodium hydrogen carbonate solution (7 mL) containing sodium thiosulphate (0.35 g). The mixture was stirred for 30 minutes, then the phases were separated. The organic layer was washed with a saturated sodium hydrogen carbonate solution. The organic layer was dried and concentrated *in vacuo* to a residue which was purified by flash chromatography (CH/AcOEt from 9:1 to 8:2) to give the title compound (0.073 g) as a white solid.
T.I.c.: CH/AcOEt 7:3, Rf=0.5 (detection with ninhydrine).
NMR (d₆-DMSO): δ (ppm) 8.02 (dd, 2H); 7.16 (t, 2H); 4.27 (sb, 1H); 4.11 (db, 1H); 3.37 (t, 1H); 2.94 (sb, 1H); 2.77 (t, 1H); 2.03 (d, 1H); 1.78 (m, 1H); 1.71 (dd, 1H); 1.6 (m, 1H); 1.48 (s, 9H).
MS (ES/+): m/z=308 [M+H]⁺.

### Intermediate 69

### 1,1-dimethylethyl 3-fluoro-3-[(4-fluorophenyl)carbonyl]-1-piperidinecarboxylate

A solution of Lithyum bis(trimethylsilyl)-amide (1M in THF - 2.6 mL) was dropped into a solution of intermediate 68 (0.5 g) dissolved in anhydrous THF (30 mL) previously cooled to -78°C under a Nitrogen atmosphere. At the end of the addition the mixture was allowed to warm to -10°C and stirred for 1 hour. Then the mixture was cooled to -40°C and N-Fluorobenzene-sulfonimide (0.668 g) dissolved in anhydrous THF (6 mL) was added. The mixture was stirred for 1 hour then further N-Fluorobenzene-sulfonimide (0.050 g) was added and the mixture was allowed to stirr to r.t. then it was treated with ammonium chloride saturated solution (50 mL). The mixture was stirred at r.t. for 10 minutes, then a white precipitated was formed. The precipitate was filtered off and the mixture was extracted with AcOEt. The organic layer was dried and concentrated *in vacuo* to a residue which was purified by flash chromatography (CH/AcOEt 9:1) to give the title compound (0.38 g).
T.l.c.: CH/AcOEt 7:3, Rf=0.6 (detection with ninhydrine).
NMR (CDCl3): δ (ppm) 8.15 (t, 2H); 7.14 (t, 2H); 4.35 (m, 2H); 3.35 (m, 1H); 2.85 (s, 1H); 2.1 (m, 3H); 1.68 (d, 1H); 1.48 (s, 9H).
MS (ES/+): m/z=326 [M+H]⁺.

### Intermediate 70

### 1,1-dimethylethyl 3-[3-(ethyloxy)-1-(4-fluorophenyl)-3-oxo-1-propen-1-yl]-3-fluoro-1-piperidinecarboxylate

Triethylphosphono Acetate (0.93 mL) was dropped into a sospension of NaH (60% in mineral oil, 0.112 g) in anhydrous THF (10 mL) previously cooled to 0°C under a Nitrogen atmosphere. At the end of the addition the mixture was allowed to warm to r.t. and stirred for 20 minutes. Then intermediate 69 (0.38 g) dissolved in anhydrous THF (10 mL) was added and the mixture was stirred at reflux temperature. After 2 hours the mixture was allowed to reach r.t. and was treated with water and extracted with Et2O. The organic layer was dried and concentrated *in vacuo* to a residue which was purified by flash chromatography (CH/AcOEt (9:1) to give the title compound (0.45 g) as a yellow oil.
T.I.c.: CH/AcOEt 8:2, Rf=0.42 (detection with ninhydrine).
NMR (CD3OD): δ (ppm) 7.13 (m, 4H); 6.33 (s, 1H); 4.14 (t, 1 H); 3.98 (d, 1H); 3.93 (q, 2H); 3.07 (dd, 1H); 2.72 (t, 1 H); 2.05-1.75 (m, 3H); 1.56 (m, 1H); 1.43 (s, 9H); 1.01 (t, 3H).
MS (ES/+): m/z=396 [M+H]⁺.

### Intermediate 71

### 1,1-dimethylethyl-3-[3-(ethyloxy)-1-(4-fluorophenyl)-3-oxopropylidene]-1-piperidinecarboxylate (71 a) and 1,1-dimethylethyl-3-[1-(4-fluorophenyl)-3-(methyloxv)-3-oxopropylidene]-1-piperidinecarboxylate (71b)

Magnesium turnings (12 mg) were added to a solution of intermediate 70 (38 mg) in anhydrous MeOH (1 mL) under a Nitrogen atmosphere with a low heating until hydrogen evolution is observed. The reaction mixture was stirred at r.t. overnight, then water (2 m_{L}) and ammonium chloride saturated solution were added and the mixture was extracted with AcOEt. The organic phase was dried and concentrated *in vacuo* to give a mixture of title compounds (33 mg) as a colourless oil.

### Intermediate 71a:

MS (ES/+): m/z=400 [M+Na]⁺.

### Intermediate 71b:

MS (ES/+): m/z=386 [M+Na]⁺.

### Intermediate 72

### 3-(1{[(1,1-dimethylethyl)oxylcarbonyl)-3-piperidinylidene)-3-(4-fluorophenyl)propanoic acid

A solution of lithium hydroxide monohydrate (17 mg) in water (0.25 mL) was added to a solution of intermediate 71a and 71b (33 mg) in MeOH (1.0 mL). The mixture was heated to 65°C for 1 hour, then it was allowed to cool to r.t., acidified to ph=3 with 1M hydrochloric acid solution and extracted with AcOEt. The organic layer was washed with water, dried and concentrated *in vacuo* to give the title compound (30 mg) as a yellow oil.
T.I.c.: CH/AcOEt 1:1, Rf 0.18 (detection with ninhydrine).
MS (ES/-): m/z=348 [M-H]⁻.

### Intermediate 73

### 1,1-dimethylethyl 3-[3-[{[3,5-bis(trifluoromethyl)phenyl]methyl)(methyl)amino]-1-(4-fluorophenyl)-3-oxopropylidene]-1-piperidinecarboxylate(73a)(isomer 1)

### 1,1-dimethylethyl 3-(3-[{[3,5-bis(trifluoromethyl)phenyl]methyl}(methyl)amino]-1-(4-fluorophenyl)-3-oxopropylidene]-1-piperidinecarboxylate (73b) (isomer 2)

DIPEA (0.044 mL) and O-(benzotriazol-1-yl)-N,N,N'N'-tetramethyluronium tetrafluoroborate (0.034 g) were added to a solution of intermediate 72 (0.03 g) in anhydrous DMF (4 mL) under a Nitrogen atmosphere. After stirring for 20 minutes, (3,5-bis-trifluoromethyl-benzyl)-methylamine hydrochloride (0.028 g) was added. The mixture was stirred at r.t. overnight, then it was diluted with AcOEt and washed with cold water. The organic layer was dried, concentrated *in vacuo* to a residue that was purified by flash chromatography (CH/AcOEt 9:1) to give:
intermediate 73a (8 mg) as yellow oil;
intermediate 73b (14 mg) as yellow oil.

### intermediate 73a

T.I.c.: C H/AcOEt 1:1, Rf=0. 7.
NMR (CDCl3): δ (ppm) 7.78 (s, 1H); 7.61 (s, 2H); 7.11 (t, 2H); 6.98 (t, 2H); 4.60 (s, 2H); 4.13 (s, 2H); 3.61 (s, 2H); 3.49 (s, 2H); 2.90 (s, 3H); 2.20 (t, 2H); 1.5 (m, 2H); 1.48 (s, 9H).
MS (ES/+): m/z=589 [M+H]⁺.

### intermediate 73b

T.I.c.: CH/AcOEt 1:1, Rf=0.4.
NMR (CDCl3): δ (ppm) 7.79 (s, 1H); 7.60 (s, 2H); 7.13 (t, 2H); 6.99 (t, 2H); 4.60 (s, 2H); 3.87 (s, 2H); 3.51 (m, 4H); 2.85 (s, 3H); 2.46 (t, 2H); 1.73 (sb, 2H); 1.28 (db, 9H).
MS (ES/+): m/z=589 [M+H]⁺.

### Intermediate 74

### 1,1-dimethylethyl 3-formyl-1-pyrrolidinecarboxylate

Dimethylsulfoxide (0.106 mL) dissolved in anhydrous DCM (2 mL) was added to a solution of oxalyl chloride (0.065 mL) in anhydrous DCM (1 mL) previously cooled to - 78°C under a Nitrogen atmosphere. The solution was stirred at -78°C for 15 minutes, then a solution of 1,1-dimethylethyl 3-(hydroxymethyl)-1-pyrrolidinecarboxylate (0.075 g) in anhydrous DCM (2 mL) was slowly added. The mixture was allow to warm to -30°C in 2 hours, then TEA (0.312 mL) was added. The mixture was allowed to warm to r.t. over 1 hour, then it was diluted with DCM and washed with water. The organic layer was dried and concentrated *in vacuo* to a residue which was purified by flash chromatography (DCM/MeOH from 98:2 to 96:4) to give the title compound (0.065 g).
T.I.c.: DCM/MeOH 9:1, Rf=0.5.
MS (ES/+): m/z=200 [M+H]⁺.

### Intermediate 75

### 1,1-dimethylethyl 3-[(4-fluorophenyl)(hydroxy)methyl]-1-pyrrolidinecarboxylate

A solution of intermediate 74 (0.100 g) in anhydrous Et2O (2 mL) was dropped into a solution of 4-fluorophenyl magnesium bromide (1M in THF - 1.5 mL) in anhydrous Et2O (1 mL) previously cooled to 0°C under a Nitrogen atmosphere. At the end of the addition the mixture was allowed to warm to r.t. and stirred at 23°C for 30 minutes. The mixture was quenched with a saturated ammonium chloride solution (1 mL). The aqueous layer was further extracted with AcOEt. The combined organic extracts were washed with brine, dried and concentrated *in vacuo* to give a residue which was purified by flash chromatography (DCM/MeOH 98:2) to give the title compound (0.162 g) as diastereomeric mixture.
T.I.c.: DCM/MeOH 9:1, Rf=0.65
NMR (d₆-DMSO): δ (ppm): 7.36 (m, 2H); 7.13 (t, 2H); 5.41 (dd, 1 H); 4.41 (m, 1 H); 3.3 (m, 2H); 3.1 (m, 1 H); 2.9 (m, 1 H); 2.3 (m, 1 H); 1.8 (m, 2H); 1.36 (d, 9H).
MS (ES/+): m/z=296 [M+H]⁺.

### Intermediate 76

### 1,1-dimethylethyl 3-[(4-fluorophenyl)carbonyl]-1-pyrrolidinecarboxylate

A solution of mixture of intermediate 75 (0.160 g) was dissolved in anhydrous DCM (3 mL) and treated portion-wise with Dess Martin periodinane (0.232 g) under a Nitrogen atmosphere. The brown solution was stirred at r.t. for 3 hours, then the solution was diluted with DCM (5 mL) and poured into a saturated sodium hydrogen carbonate solution (7 mL) containing sodium thiosulphate (0.3 g). The mixture was stirred for 30 minutes, then the phases were separated. The organic layer was washed with a saturated sodium hydrogen carbonate solution. The organic layer was dried and concentrated *in vacuo* to a residue which was purified by flash chromatography (DCM/MeOH (98:2) to give the title compound (0.100 g) as a white solid.
NMR (d₆-DMSO): δ (ppm) 8.09 (dd, 2H); 7.37 (t, 2H); 4.17 (m, 1 H); 3.52 (dd, 1 H); 3.42 (dd, 1 H); 2.5 (mb, 2H); 2.16 (m, 1H); 1.95 (m, 1 H); 1.39 (s, 9H).
MS (ES/+): m/z=294 [M+H]⁺.

### Intermediate 77

### 1,1-dimethylethyl 3-fluoro-3-[(4-fluorophenyl)carbonyil-1-pyrrolidinecarboxylate

A solution of Lithyum bis(trimethylsilyl)-amide (1 M in THF - 0.287 mL) was dropped into a solution of intermediate 76 (0.056 g) dissolved in anhydrous THF (3 mL) previously cooled to -78°C under a Nitrogen atmosphere. At the end of the addition the mixture was allowed to warm to 0°C and stirred for 1.5 hours. Then the mixture was cooled to -40°C and N-Fluorobenzene-sulfonimide (0.072 g) dissolved in anhydrous THF (0.6 mL) was added. Then the mixture was allowed to stirr to r.t. in about 3.5 hours and it was treated with ammonium chloride saturated solution and extracted with AcOEt. The organic layer was dried and concentrated *in vacuo* to a residue which was purified by flash chromatography (CH/AcOEt from 9:1 to 7:3) to give the title compound (0.013 g) as a yellow oil.
T.I.c.: CH/AcOEt 7:3, Rf=0.57 (detection with ninhydrine).
NMR (d₆-DMSO, 60°C): δ (ppm) 8.1-8.02 (m, 2H); 7.4-7.3 (t, 2H); 3.89 (s, 1H); 3.80 (s, 1 H); 3.66 (m, 1 H); 3.40 (m, 1H); 2.6-2.4 (m, 2H); 1.4 (s, 9H).
MS (ES/+): m/z=312 [M+H]⁺; 334 [M+Na]⁺.

### Intermediate 78

### 1,1-dimethylethyl 3-[3-(ethyloxy)-1-(4-fluorophenyl)-3-oxo-1-propen-1-yl]-3-fluoro-1-pyrrolidinecarboxylate

Triethyl phosphonoacetate (0.135 mL) was dropped into a suspension of NaH (60% in mineral oil, 0.027 g) in anhydrous THF (2 mL) previously cooled to 0°C under a Nitrogen atmosphere. At the end of the addition the mixture was allowed to warm to r.t. and stirred for 20 minutes. Then intermediate 77 (0.05 g) dissolved in anhydrous THF (2 mL) was added and the mixture was stirred at reflux temperature. After 1 hours the mixture was allowed to reach r.t. and was treated with water and extracted with Et2O. The organic layer was dried and concentrated *in vacuo* to a residue which was purified by flash chromatography (CH/AcOEt 9:1) to give the title compound (0.057 g) as a thick colourless oil.
T.l.c.: CH/AcOEt 9:1, Rf=0.23 (detection with ninhydrine).
MS (ES/⁺): m/z=382 [M+H]⁺.

### Intermediate 79

### 1,1-dimethylethyl -3-[3-(ethyloxy)-1-(4-fluorophenyl)-3-oxopropylidene]-1-pyrrolidinecarboxylate

Magnesium turnings (18 mg) were added to a solution of intermediate 78 (55 mg) in anhydrous MeOH (1 mL) under a Nitrogen atmosphere with a low heating until hydrogen evolution is observed. Then, the reaction mixture was stirred at r.t. overnight, then water (2 mL) and ammonium chloride solution were added and extracted with AcOEt. The organic phase was dried and concentrated *in vacuo* to give the title compound (50 mg) as a colourless oil.
MS (ES/+): m/z=386 [M+Na]⁺.
T.I.c.: CH/AcOEt 7:3, Rf=0.6(detection with ninhydrine).

### Intermediate 80

### 3-(1-{[(1,1-dimethylethyl)oxy]carbonyl}-3-pyrrolidinylidene)-3-(4-fluorophenyl)propanoic acid

Intermediate 79 (50 mg) was dissolved in MeOH (1 mL), treated with a solution of lithium hydroxide monohydrate (29 mg) in water (0.25 mL). The mixture was heated to 65°C for 1 hour, then it was allowed to cool to r.t., acidified to pH 3 with 1 M hydrochloric acid solution and extracted with AcOEt. The organic layer was washed with water, dried and concentrated *in vacuo* to give the title compound (40 mg) as a white foam.
T.I.c.: CH/AcOEt 1:1, Rf 0.18 (detection with ninhydrine).
MS (ES/-): m/z=334 [M-H]⁻.

### Intermediate 81

**1,1-dimethylethyl (3E)-3-[3-[{[3,5-bis(trifluoromethyl)phenyl](methyl)(methyl)amino]-1-(4-fluorophenyl)-3-oxopropylldene]-1-pyrrolidinecarboxylate (81a) and1,1-dimethylethyl (3Z)-3-[3-[{(3,5-bis(trifluoromethyl)phenyl]methyl}(methyl)amino]-1-(4-fluorophenyl)-3-oxopropylidene]-1-pyrrolidinecarboxylate (81b)** DIPEA (0.08 mL) and O-(benzotriazol-1-yl)-N,N,N'N'-tetramethyluronium tetrafluoroborate (0.062 g) were added to a solution of intermediate 80 (0.04 g) in anhydrous DMF (4 mL) under a Nitrogen atmosphere. After stirring for 20 minutes, (3,5-bis-trifluoromethyl-benzyl)-methyl-amine hydrochloride(0.039 g) was added. The mixture was stirred at r.t. overnight, then it was taken up with AcOEt (30 mL) and washed with water (4 x 5 mL) and brine (2 x 5 mL). The organic layer was dried, concentrated *in vacuo* and the residue was purified by flash chromatography (CH/AcOEt 7/3) to give the title compounds:
Intermediate 81 a (0.023 g) as a white foam;
Intermediate 81b (0.025 g) as a white foam.

### Intermediate 81a

T.I.c.: CH/AcOEt 1:1, Rf=0.4 (detection with ninhydrine).
MS (ES/+): m/z=597 [M+Na]⁺

### Intermediate 81b

T.I.c.: CH/AcOEt 1:1, Rf=0.2 (detection with ninhydrine).
MS (ES/+): m/z=597 [M+Na]⁺

### Intermediate 82

### 1,1-dimethylethyl 4-[3-(ethyloxy)-1-(4-fluorophenyl)-3-oxopropylidene]-1-piperidinecarboxylate

Magnesium turnings (70 mg) were added to a solution of intermediate **36** (200 mg) in anhydrous MeOH (1 mL) under a Nitrogen atmosphere with a low heating until hydrogen evolution is observed. Then, the reaction mixture was stirred at r.t. for 6 h, then water (10 ml mL) and ammonium chloride solution were added and extracted with AcOEt (2x 10 mL). The organic phase was dried and concentrated *in vacuo;* the crude was purified by flash chromatography (CH/AcOEt 8:2) to give the title compound( 180 mg) of colourless oil.
T.I.c.: CH/AcOEt=7:3, Rf 0.64 (detection with ninhydrine).
MS (ES/+): m/z=378 [M+H]⁺.

### Intermediate 83

### 3-(1-{[(1,1-dimethylethyl)oxy]carbonyl}-4-piperidinylidene)-3-(4-fluorophenyl)propanoic acid

Intermediate 82 was dissolved in MeOH (5 mL), treated with a solution of Lithium hydroxide monohydrate (100 mg) in water (1.25 mL). The mixture was heated to 70°C for 2 hour, then it was allowed to cool to r.t., acidified to pH 3 with 1M Hydrochloric acid solution and extracted with AcOEt (2 x 15 mL). The organic layer was washed with water, dried and concentrated *in vacuo* to give the title compound (150 mg) as a white foam.
T.I.c.: CH/AcOEt 1:1, Rf 0.20 (detection with ninhydrine).
MS (ES/+): m/z=350 [M+H]⁺.

### Intermediate 84

### 1,1-dimethylethyl 4-[3-[{(1R)-1-[3,5-bis(trifluoromethyl)phenyl]ethyl}(methyl)amino]-1-(4-fluorophenyl)-3-oxopropylidene]-1-piperidinecarboxylate

DIPEA (0.023 mL) and O-(benzotriazol-1-yl)-N,N,N'N'-tetramethyluronium tetrafluoroborate (0.036 g) were added to a solution of intermediate 83 (0.03 g) in anhydrous DMF (2 mL) under a Nitrogen atmosphere. After stirring for 20 minutes, {(1*R*)-1-[3,5-bis(trifluoromethyl)phenyl]ethyl}methylamine (0.021 g) was added. The mixture was stirred at r.t. overnight, then it was diluted with AcOEt and washed with water. The organic layer was dried, concentrated *in vacuo* and the residue was purified by flash chromatography (CH/AcOEt from 8:2 to 7:3) to give the title compound (40.5 mg) as yellow oil.
MS (ES/+): m/z=589 [M+H]⁺.

### Intermediate 85

### 1,1-dimethylethyl 4-[3-[{(1S)-1-[3,5-bis(trifluoromethyl)phenyl]ethyl}(methyl)amino]-1-(4-fluorophenyl)-3-oxopropylidene]-1-piperidinecarboxylate

DIPEA (0.023 mL) and O-(benzotriazol-1-yl)-N,N,N'N'-tetramethyluronium tetrafluoroborate (0.036 g) were added to a solution of intermediate 83 (0.03 g) in anhydrous DMF (2 mL) under a Nitrogen atmosphere. After stirring for 20 minutes[1-(S)-(3,5-bis-trifluoromethyl-phenyl)-ehtyl]-methyl-amine (0.021 g) was added. The mixture was stirred at r.t. overnight, then it was diluted with AcOEt and washed with water. The organic layer was dried, concentrated *in vacuo* and the residue was purified by flash chromatography (CH/AcOEt from 8:2 to 7:3) to give the title compound (40 mg) as yellow oil.
MS (ES/+): m/z=589 [M+H]⁺

### Intermediate 86

### 1.1-dimethylethyl 4-[3-[[(3,5-dibromophenyl)methyl](methyl)amino]-1-{4-fluorophenyl)-3-oxopropylidene]-1-piperidinecarboxylate

DIPEA (0.046 mL) and O-(benzotriazol-1-yl)-N,N,N'N'-tetramethyluronium tetrafluoroborate (0.036 g) were added to a solution of intermediate 83 (0.03 g) in anhydrous DMF (2 mL) under a Nitrogen atmosphere. After stirring for 20 minutes(3,5-bis-bromo-benzyl)-methylamine hydrochloride (0.025 g) was added. The mixture was stirred at r.t. overnight, then it was diluted with AcOEt and washed with water. The organic layer was dried, concentrated *in vacuo* and the residue was purified by flash chromatography (CH/AcOEt from 95:5 to 7:3) to give the title compound (44 mg) as yellow oil;
MS (ES/+): m/z=609 [M+H]⁺.
T.I.c.: CH:AcOEt=1:1 Rf 0.65 (detection with ninhydrine).

### Intermediate 87

### 1,1-dimethylethyl 4-[2-cyano-3-(ethyloxy)-1-(4-fluoro-2-methylphenyl)-3-oxopropyl]-3,6-dihydro-1(2M-pyridinecarboxylate

A solution of 2-bromo,5-fluorotoluene (2.06 mL) in anhydrous Et2O (6.5 mL) was dropped into a suspension of magnesium turning (0.396 g) and few crystals of iodine in anhydrous Et2O (1.6 mL) under a Nitrogen atmosphere. The mixture refluxed for 30 minutes, then it was allowed to cool to r.t.. 3.1 ml of this solution were added dropwise to intermediate 19 (665 mg) in anhydrous Et2O (10 mL). At the end of the addition the mixture was heated to reflux and stirred for 1 hour. The mixture was quenched with ammonium chloride saturated solution and extracted with AcOEt. The aqueous layer was extracted with further AcOEt. The combined organic extracts were dried and concentrated *in vacuo* to a residue which was purified by flash chromatography (CH/AcOEt from 9:1 to 8:2) to give the title compound (300 mg).
Tlc: CH/AcOEt 7:3, Rf=0.34 (detection with ninhydrine).
MS (ES/+): m/z=439 [M+Na]⁺.

### Intermediate 88

### 1,1-dimethylethyl 4-[2-cyano-1-(4-fluoro-2-methylphenyl)ethyl]-3,6-dihydro-1 (2H)-pyridinecarboxylate

A mixture of intermediate 87 (300 mg), sodium chloride (14 mg) and water (28 µL) in anhydrous dimethylsulfoxide (3 mL) was heated to 160°C for 2 hours. The mixture was allowed to cool to r.t., diluted with AcOEt and washed with water and brine. The aqueous layer was extracted with further AcOEt (3 x 20 mL). The organic layer was dried and concentrated *in vacuo.* The residue was purified by silica cartridge (CH/AcOEt from 98/2 to 80:20) to give the title compound (210 mg) as a colourless oil.
MS (ES/+): m/z=367 [M+Na]⁺.

### Intermediate 89

### 3-(1-{[(1,1-dimethylethyl)oxylcarbonyl}-1,2,3,6-tetrahydro-4-pyridinyl)-3-(4-fluoro-2-methylphenyl)propanoic acid

### (GSK 232254A - VDLU/6207/62/2)

A 3M potassium hydroxide solution (9 mL) was added to a solution of intermediate 88 (210 mg) in abs. EtOH (9 mL). The mixture divided into three portions which were heated in microwave (300W, P=90p.s.i.) at 150°C for (10+10+10) minutes. Each solution was allowed to cool to r.t., diluted with AcOEt/H₂O, acidified to pH 4/5 with a pH3 buffer solution (citrate-hydrochloric acid buffer solution purchase from Fluka) and 2N hydrochloric acid solution. The aqueous layer was extracted with further AcOEt (3 x 10 mL). The organic extract was dried and concentrated *in vacuo* to give the title compound (154 mg) as a white solid.
TIc: AcOEt, Rf=0.44 (detection with ninhydrine).
NMR (d₁-CDCl₃): δ (ppm) 7.06 (t, 1H); 6.87 (d, 2H); 5.50 (bs, 1H); 3.98-3.29 (m, 5H); 2.78 (dd, 2H); 6.08 (q, 1 H); 2.36 (s, 3H); 1.45 (s, 9H).

### Intermediate 90

### 1,1-dimethylethyl 4-[3-[{[3,5-bis(trifluoromethyl)phenyl]methyl}(methyl)amino]-1-(4-fluoro-2-methylphenyl)-3-oxopropyl]-3,6-dihydro-1(2H)-pyridinecarboxylate

DIPEA (65 µL) and O-(benzotriazol-1-yl)-N,N,N'N'-tetramethyluronium tetrafluoroborate (52 mg) were added to a solution of intermediate 89 (46 mg) in anhydrous DMF (4 mL) under a Nitrogen atmosphere. After stirring for 10 minutes, {[3,5-bis(trifluoromethyl)phenyl]methyl)methylamine hydrochloride (41 mg) was added. The mixture was stirred at r.t. overnight, then it was diluted with water (5 mL) and extracted with AcOEt (20 mL). The organic layer was dried, concentrated *in vacuo* and the residue was purified by flash chromatography (CH/AcOEt 7:3) to give the title compound (44 mg) as a white foam.
T.l.c.: CH/AcOEt 6:4, Rf=0.41.
MS (ES/+): m/z=367 [M+Na]⁺.

### Intermediate 91

### 1,1-dimethylethyl 4-[3-[{(1R)-1-[3,5-bis(trifluoromethyl)phenyl]ethyl}(methyl)amino]-1-(4-fluoro-2-methylphenyl)-3-oxopropyl]-3,6-dihydro-1 (2H)-pyridinecarboxylate (91a) (diastereoisomer 1) and 1,1-dimethylethyl 4-[3-[{(1R)-1-[3,5-bis(trifluoromethyl)phenyl]ethyl}(methyl)amino]-1-(4 fluoro-2-methylphenyl)-3-oxopropyl]-3,6-dihydro-1(2H)-pyridinecarboxylate (91b) (diastereoisomer 2)

DIPEA (42 µL) and O-(benzotriazol-1-yl)-N,N,N'N'-tetramethyluronium tetrafluoroborate (67 mg) were added to a solution of intermediate 89 (60 mg) in anhydrous DMF (4 mL) under a Nitrogen atmosphere. After stirring for 10 minutes, {(1*R*)-1-[3,5-bis(trifluoromethyl)phenyl]ethyl}methylamine (46.7 mg) was added. The mixture was stirred at r.t. overnight, then it was diluted with water (5 mL) and extracted with AcOEt (20 mL). The organic layer was dried, concentrated *in vacuo* and the residue was purified by flash chromatography (CH/AcOEt 7:3) to give:
intermediate 91a (25.5 mg) as a white foam;
intermediate 91 b (20.2. mg) as a white foam.

### Intermediate 91 a

T.I.c.: CH/AcOEt 7:3, Rf=0.73.
NMR (d₁-CDCl3): δ (ppm) 7.80 (s, 1 H); 7.66 (s, 2H); 7.05 (dd, 1H); 6.87 (dd, 1H); 6.84 (d, 1H); 6.08 (q, 1H); 5.39 (b, 1H); 4.21 (t, 1 H); 3.92 (bs, 2H); 3.50 (dd, 1 H); 3.31 (dd, 1H); 2.84 (dd, 1 H); 2.78 (dd, 1H); 2.61 (s, 3H); 2.40 (s, 3H); 2.01 (s, 2H); 1.45 (b, 9H); 1.41 (d, 3H).

### Intermediate 91b

T.l.c.: CH/AcOEt 7:3, Rf=0.63.
NMR (d₁-CDCl3): δ (ppm) 7.73 (s, 1H); 7.50 (s, 2H); 6.98 (dd, 1H); 6.81 (dd, 1H); 6.80 (d, 1 H); 6.02 (q, 1H); 5.36 (b, 1 H); 4.16 (t, 1H); 3.88 (bs, 2H); 3.44 (dd, 1H); 3.29 (dd, 1H); 2.77 (dd, 1H); 2.61 (dd, 1H); 2.62 (s, 3H); 2.34 (s, 3H); 1.96 (b, 2H); 1.49 (d, 3H); 1.42 (s, 9H).

### Intermediate 92

### 1,1-dimethylethyl 3-[3-(ethyloxy)-1-(4 fluorophenyl)-3-oxopropyl]-1-pyrrolidinecarboxylate

Pd/C (10%, 10 mg) was added to a solution of intermediate 78 (65 mg) in anhydrous EtOH (8 mL) under a hydrogen pressure of 3.5 atmospheres and reacted overnight. Then, the reaction mixture was filtered over a Celite pad washing with MeOH/DCM and concentrated *in vacuo* to give the title compound (45 mg) as a yellow oil:
T.l.c.: CH/AcOEt 7:3, Rf=0.44.
MS (ES/+): m/z=388 [M+Na]⁺.

### Intermediate 93

### 3-(1-{[(1,1-dimethylethyl)oxy]carbonyl}-3-pyrrolidinyl)-3-(4-fluorophenyl)propanoic acid

Intermediate **92** (45 mg) was dissolved in MeOH (2 mL), treated with a solution of lithium hydroxide monohydrate (25 mg) in water (0.5 mL). The mixture was heated to 65°C for 1 hour, then it was allowed to cool to r.t., acidified to pH 3 with 1 M hydrochloric acid solution and extracted with AcOEt. The organic layer was washed with water, dried and concentrated *in vacuo* to give the title compound (44 mg) as a white foam.
T.I.c.: CH/AcOEt 1:1, Rf 0.2 (detection with ninhydrine).
MS (ES/-): m/z=336 [M-H]⁻.

### Intermediate 94

### 1,1-dimethylethyl 3-[3-[{[3,5-bis(trifluoromethyl)phenyl]methyl}(methyl)amino]-1-(4-fluorophenyl)-3-oxopropyl]-1-pyrrolidinecarboxylate (94a) (diastereoisomer A)

### 1,1-dimethylethyl 3-[3-[{[3,5-bis(trifluoromethyl)phenyl]methyl}(methyl)amino]-1-(4-fluorophenlyl)-3-oxopropyl]-1-pyrrolidinecarboxylate (94b) (diastereoisomer B)

DIPEA (0.065 mL) and O-(benzotriazol-1-yl)-N,N,N'N'-tetramethyluronium tetrafluoroborate (0.052 g) were added to a solution of intermediate 93 (0.044 g) in anhydrous DMF (4 mL) under a Nitrogen atmosphere. After stirring for 20 minutes, {[3,5-bis(trifluoromethyl)phenyl]methyl)methylamine hydrochloride (0.045 g) was added. The mixture was stirred at r.t. overnight, then it was taken up with AcOEt (30 mL) and washed with water (4 x 5 mL) and brine (2 x 5 mL). The organic layer was dried, concentrated *in vacuo* and the residue was purified by flash chromatography (CH/AcOEt 8/2) to give:
intermediate 94a (20 mg) as a white foam;
intermediate 94b (20 mg) as a white foam.

### Intermediate 94a

T.I.c.: CH/AcOEt 7:3, Rf=0.35 (detection with ninhydrine).
MS (ES/+): m/z= 599 [M+Na]⁺.

### Intermediate 94b

T.I.c.: CH/AcOEt 7:3, Rf=0.28 (detection with ninhydrine).
MS (ES/+): m/z= 599 [M+Na]⁺.

### Intermediate 95

### 1,1-dimethylethyl 3-[3-(ethyloxy)-1-(4-fluorophenyl)-3-oxopropyl]-3-fluoro-1-piperidinecarboxylate

Pd/C (10%, 5 mg) was added to a solution of intermediate **70** (50 mg) in anhydrous EtOH (4 mL) under an hydrogen pressure of 1.5 atmosphere overnight. Then, the reaction mixture was filtered over a celite pad washing with MeOH/DCM and concentrated *in vacuo* to give the title compound (50 mg) as a yellow oil.
T.I.c.: CH/AcOEt 1:1, Rf=0.8 (detection with ninhydrine).
MS (ES/+): m/z=420 [M+Na]⁺.

### Intermediate 96

### 3-(1-{[(1,1-dimethylethyl)oxylcarbonyl}-3-fluoro-3-piperidinyl)-3-(4-fluorophenyl)propanoic acid

A solution of lithium hydroxide monohydrate (26 mg) in water (0.4 mL) was added to a solution of intermediate 95 (50 mg) in MeOH (1.6 mL). The mixture was heated to 65°C for 1 hour, then it was allowed to cool to r.t., acidified with 1 M hydrochloric acid solution and extracted with DCM. The organic layer was washed with water, dried and concentrated *in vacuo* to give the title compound (47 mg) as a yellow cil.
T.I.c.: CH/AcOEt 1:1, Rf=0.4, Rf=0.2 (detection with ninhydrine).
MS (ES/-): m/z=368 [M-H]-.

### Intermediate 97

### 1,1-dimethylethyl 3-[3-[{[5-bis(trifluoromethyl)phenyl]methyl)(methyl)amino]-1-(4-fluorophenyl)-3-oxopropyl]-3-fluoro-1-piperidinecarboxylate (97a) (diastereoisomer A)

### 1,1-dimethylethyl 3-[3-[{[3,5-bis(trifluoromethyl)phenyl]methyl}(methyl}amino]-1-(4-fluorophenyl)-3-oxopropyl]-3-fluoro-1-piperidinecarboxylate (97b) (diastereoisomer B)

DIPEA (0.067 mL) and O-(benzotriazol-1-yl)-N,N,N'N'-tetramethyluronium tetrafluoroborate (0.053 g) were added to a solution of intermediate 96 (0.047 g) in anhydrous DMF (6 mL) under a Nitrogen atmosphere. After stirring for 20 minutes, (3,5-bis-trifluoromethyl-benzyl)-methylamine hydrochloride (0.045 g) was added. The mixture was stirred at r.t. overnight, then it was diluted with AcOEt and washed with cold water. The organic layer was dried, concentrated *in vacuo* and the residue was purified by flash chromatography (CH/AcOEt from 9:1 to 6:4) to give:
intermediate 97a (19 mg) as yellow oil;
intermediate 97b (38 mg) as yellow oil.

### Intermediate 97a

T.l.c.: CH/AcOEt 6:4, Rf=0.46
MS (ES/+): m/z=609 [M+H]⁺

### Intermediate 97b

T.I.c.: CH/AcOEt 6:4, Rf=0.25
MS (ES/+): m/z=609 [M+H]⁺.

### Intermediate 98

### 1,1-dimethylethyl-2-[(4-fluorophenyl)(hydroxy)methyl]-4-morpholinecarboxylate

A solution of 4-fluorophenyl magnesium bromide (1M in THF - 0.460 mL) in anhydrous Et2O (0.5 mL) was dropped into a solution of 1,1-dimethylethyl 2-formyl-4-morpholinecarboxylate (0.05 g) in anhydrous Et2O (1 mL) previously cooled to 0°C under a Nitrogen atmosphere. The mixture was allowed to warm to r.t. and stirred for 45 minutes then treated with saturated ammonium chloride solution (1 mL). The mixture was stirred at r.t. for 30 minutes, then Et2O was added and the layers were separated. The aqueous layer was extracted with further Et20. The combined organic extracts were washed with a saturated sodium hydrogen carbonate and brine, then dried and concentrated *in vacuo.* The residue was purified by flash chromatography (DCM, DCM from 95:5 to 8:2) to give the title compound (0.044 g) as a white foam.
T.I.c.: CH2Cl2/AcOEt 9:1, Rf=0.29.
NMR (CDCl3): δ (ppm) 7.30 (dd, 2H), 7.00 (t, 2H), 4.55 (d, 1 H), 4.00 (d, 1 H), 3.80 (bd, 1H), 3.55 (m, 2H), 3.40 (m, 1 H), 3.05-2.85 (m, 2H), 2.65 (bs, 1 H), 1.45 (s, 9H).

### Intermediate 99

### 1,1-dimethylethyl 2-[(4-fluorophenyl)carbonyl]-4-morpholinecarboxylate

To a solution of intermediate 98 (200 mg) in anhydrous DCM (5 mL) Dess-Martin periodinane (272 mg) was added portionwise within 2 hours at r.t. under a Nitrogen atmosphere.The mixture was stirred at r.t. for 30 minutes, then a solution of Na₂S₂O₃ (5% in saturated aqueous solution of NaHCO₃, 4ml) was added. After stirring for **1** hour the mixture was extracted with DCM, the combined organic extracts were washed with a saturated sodium hydrogen carbonate and brine then dried and concentrated *in vacuo.* The residue was purified by flash chromatography (from DCM, DCM from 95:5 to 9:1) to give the title compound (135 mg).
T.I.c.: CH2Cl2/AcOEt 9:1, Rf=0.65.
NMR (CDCl3): δ (ppm): 8.03 (dd, 2H), 7.13 (t, 2H), 4.65 (bd, 1 H), 4.20 (bs, 1H), 4.01 (m, 1 H), 3.89 (bd, 1 H), 3.68 (m, 1H), 3.07 (m, 2H), 1.46 (s, 9H).
MS (ES/+): m/z=332 [M+Na]⁺.

### Intermediate 100

### 1,1-dimethylethyl 2-[(1Z)-3-(ethyloxy)-1-(4-fluorophenyl)-3-oxo-1-propen-1-yl]-4-morpholinecarboxylate (100a) 1,1-dimethylethyl 2-[(1E)-3-(ethyloxy)-1-(4-fluorophenyl)-3-oxo-1-propen-1-yl]-4-morpholinecarboxylate (100b)

Triethylphosphonoacetate (350 µL) was added to a suspension of sodium hydride (65% suspension in mineral oil - 65 mg) in anhydrous THF (5 mL) previously cooled to 0°C under a Nitrogen atmosphere. The mixture was allowed to warm to r.t. and stirred at 23°C for 20 minutes. Then, a solution of intermediate 99 (135 mg) in anhydrous THF (5 mL) was added and the resulting solution was heated to reflux for 1.5 hours. The solution was allowed to cool to r.t., diluted with water and extracted with Et2O. The organic layer was washed with water and brine, dried and concentrated *in vacuo* to a residue, which was purified by flash chromatography (CH, CH/AcOEt from 95:5 to CH/AcOEt 9:1) to give:
intermediate 100a (35 mg) as oil;
intermediate 100b (51 mg) as oil.

### Intermediate 100a:

T.I.c.: CH/AcOEt 8:2, Rf=0.40.
NMR (CDCl3): δ (ppm): 7.42 (dd, 2H), 7.00 (t, 2H), 5.92 (s, 1 H), 5.36 (dd, 1H), 4.20 (q, 2H), 4.02 (d, 2H), 3.90 (dd, 1H), 4.05-3.75 (broad, 1H), 3.61 (m, 1H), 2.88 (m, 2H), 1.44 (s, 9H), 1.30 (t, 3H).
MS (ES/+): m/z=402 [M+Na]⁺.

### Intermediate 100b:

T.l.c.: CH/AcOEt 8:2, Rf= 0.31.
NMR (CDCl₃): δ (ppm): 7.13 (dd, 2H), 7.03 (t, 2H), 6.24 (d, 1H), 4.06 (d, 1H), 4.05-3.95 (m, 1 H), 3.98 (q, 2H), 3.90-3.75 (bs, 2H), 3.59 (m, 1H), 2.92 (m, 1H), 2.55 (m, 1H), 1.37 (s, 9H), 1.08 (t, 3H).
MS (ES/+): m/z=402 [M+Na]⁺.

### Intermediate 101

### 1,1-dimethylethyl 2-[3-(ethyloxyl-1-(4-fluorophenyl)-3-oxopropyl]-4-morpholinecarboxytate (101a) (diastereoisomer A)

### and 1,1-dimethylethyl 2-[3-(ethyloxy)-1-(4-fluorophenyl)-3-oxopropyl]-4-morpholinecarboxylate (101b) (diastereoisomer B)

To a solution of a mixture of intermediate 100a and 100b (115 mg) in anhydrous MeOH (10 mL) Palladium (10% wt on carbon powder, 34 mg) was added. The mixture was saturated with hydrogen (4 atm) and stirred at r.t. for 3.5 hours. After filtration of the catalyst and evaporation of the solvent, the residue was purified by flash chromatography (CH, CH/AcOEt from 95:5 to 9:1) to give:
intermediate 101a (38 mg) as an oil;
intermediate 101b (41 mg) as an oil.

### Intermediate 101a:

T.l.c.: CH/AcOEt 7:3, Rf=0.43.
MS (ES/+): m/z=404 [M+Na]⁺.

### Intermediate 101b:

T.l.c.: CH/AcOEt 7:3, Rf= 0.36.
MS (ES/+): m/z=404 [M+Na]⁺.

### Intermediate 102

### 3-(4-{[(1,1-dimethylethyl)oxylcarbonyl)-2-morpholinyl)-3-(4-fluorophenyl)propanoic acid (diastereolsomer A)

A solution of lithium hydroxide monohydrate (21 mg) in water (0.5 mL) was added to a solution of intermediate 101a (38 mg) in MeOH (2 mL). The mixture was heated to 80°C for 1 hour, then it was allowed to cool to r.t.. The residue was acidified with 1 M hydrochloric acid solution and extracted with DCM. The organic layer was dried and concentrated *in vacuo* to give the title compound (37 mg) as a foam.
MS (ES/-): m/z=352 [M-H]⁻.

### Intermediate 103

### 3-(4-{[(1,1-dimethylethyl)oxylcarbonyl)-2-morpholinyl)-3-(4-fluorophenyl)propanoic acid (diastereoisomer B)

A solution of lithium hydroxide monohydrate (23 mg) in water (0.5 mL) was added to a solution of intermediate **101b** (41 mg) in MeOH (2 mL). The mixture was heated to 80°C for 1 hour, then it was allowed to cool to r.t.. The residue was acidified with 1M hydrochloric acid solution and extracted with DCM. The organic layer was dried and concentrated *in vacuo* to give the title compound (40 mg) as a foam.
MS (ES/-): m/z=352 [M-H]⁻.

### Intermediate 104

### 1,1-dimethylethyl 2-[3-[{(1R)-1-[3,5-bis(trifluoromethyl)phenyl]ethyl}(methyl)amino]-1-(4-fluorophenyl)-3-oxopropyl]-4-morpholinecarboxylate (104a) (diastereoisomer 1) and 1,1-dimethylethyl 2-[3-[{(1R)-1-[3,5-bis(trifluoromethyl)phenyl]ethyl}(methyl)amino]-1-(4-fluorophenyl)-3-oxopropyl]-4-morpholinecarboxylate (104b) (diastereoisomer 2)

DIPEA (52 µL) and O-(7-azabenzotriazol-1-yl)-N,N,N'N'-tetramethyluronium hexafluorophosphate (49 mg) were added to a solution of intermediate 102 (37 mg) in anhydrous DMF (3 mL) under a Nitrogen atmosphere. After stirring for 1 hour,
(1*R*)-1-[3,5-bis(trifluoromethyl)phenyl]-*N*-methylethanamine (32 mg) was added. The mixture was stirred at r.t. overnight, then it was diluted with AcOEt and washed with water. The organic layer was dried, concentrated *in vacuo* and the residue was purified by flash chromatography (CH, CH/AcOEt from 9:1 to7:3) to give:
intermediate 104a (31 mg) as oil.
intermediate 104b (20 mg) as oil.

### Intermediate 104a:

T.I.c.: CH/AcOEt 1:1, Rf=0.73.
MS (ES/+): m/z=629 [M+Na]⁺, 607 [M+H]⁺.

### Intermediate 104b:

T.I.c.: CH/AcOEt 1:1, Rf=0.65.
MS (ES/+): m/z=629 [M+Na]⁺, 607 [M+H]⁺.

### Intermediate 105

### 1,1-dimethylethyl 2-[3-[{(1R)-1-[3,5-bis(trifluoromethyl)phenyl]ethyl}(methyl)aminol-1-(4-fluorophenyl)-3-oxopropyl]-4-morpholinecarboxylate (105a) (diastereoisomer 3) and 1.1-dimethylethyl 2-[3-[{(1R)-1-[3,5-bis)(trifluoromethyl)phenyl]ethyl}(methyl)amino]-1-(4-fluorophenyl)-3-oxopropyl]-4-morpholinecarboxylate (105b) (diastereoisomer 4)

DIPEA (62 µL) and O-(7-azabenzotriazol-1-yl)-N,N,N'N'-tetramethyluronium hexafluorophosphate (57 mg) were added to a solution of intermediate 103 (40 mg) in anhydrous DMF (3 mL) under a Nitrogen atmosphere. After stirring for 1 hour, (1*R*)-1-[3,5-bis(trifluoromethyl)phenyl]-*N-*methylethanamine (35 mg) was added. The mixture was stirred at r.t. overnight, then it was diluted with AcOEt and washed with water. The organic layer was dried, concentrated *in vacuo* and the residue was purified by flash chromatography (CH, CH/AcOEt from 9:1 to 7:3) to give:
intermediate 105a (16 mg) as oil.
intermediate 105b (19 mg) as oil.

### Intermediate 105a:

T.I.c.: CH/AcOEt 1:1, Rf=0.56.
MS (ES/+): m/z=629 [M+Na]⁺, 607 [M+H]⁺.

### Intermediate 105b:

T.I.c.: CH/AcOEt 1:1, Rf=0.46.
MS (ES/+): m/z=629 [M+Na]⁺, 607 [M+H]⁺.

### Intermediate 106

### 1,1-dimethylethyl 3-[(1E)-3-(ethyloxy)-1-(4-fluorophenyl)-3-oxo-1-propen-1-yl]-1-piperidinecarboxylate

Triethylphosphono Acetate (0.3 mL) was dropped into a sospension of NaH (60% in mineral oil, 62 mg) in anhydrous THF (3 mL) previously cooled to 0°C under a Nitrogen atmosphere. At the end of the addition the mixture was allowed to warm to r.t. and stirred for 20 minutes. Then intermediate 68 (120 mg) dissolved in anhydrous THF (3 mL) was added and the mixture was stirred at reflux temperature. After 3 hours the mixture was allowed to reach r.t. and was treated with water and extracted with Et2O. The organic layer was dried and concentrated *in vacuo* to a residue which was purified by flash chromatography (CH/AcOEt (9:1) to give the title compound (100 mg) as a yellow oil.
T.I.c.: CH/AcOEt 8:2, Rf=0.5 and Rf=0.4 (detection with ninhydrine).
NMR (CD3OD): δ (ppm) 7..2-7.0 (m, 4H); 5.88 (s, 1 H); 4.1 (b, 1H); 4.02 (b, 1H); 3.99 (q, 2H); 2.68 (m 1H); 2.6 (m, 1H); 2.40 (m, 1 H); 1.92 (m, 1 H); 1.4-1.6 (m, 2H); 1.58 (s, 9H), 1.09 (t, 3H).
MS (ES/+): m/z=378 [M+H]⁺.

### Intermediate 107

### 1,1-dimethylethyl 3-[3-(ethyloxy]-1-(4-fluorophenyl)-3-oxopropyl]-1-piperidinecarboxylate

Magnesium turnings (33 mg) were added to a solution of intermediate 106 (100 mg) in anhydrous MeOH (2.65 mL) under a Nitrogen atmosphere with a low heating until hydrogen evolution is observed. Then, the reaction mixture was stirred at r.t. for 3 hours, then water (2 mL) and acetic acid 10% v/v was added (2ml). Ammonium hydroxy saturated solution (3 ml) was added until ph=8. The acqueous solution was extracted with Et2O. The organic phase was dried and concentrated *in vacuo* to give the title compounds (87 mg) as a colourless oil.
T.I.c.: CH/AcOEt 7:3, Rf 0.45 (detection with ninhydrine).
MS (ES/+): m/z=402 [M+Na]⁺.

### Intermediate 108

### 3-(1-{[(1,1-dimethylethyl)oxyl] carbonyl)-3-(4-fluorophenyl)propanoic acid

A solution of lithium hydroxide monohydrate (48mg) in water (0.7 mL) was added to a solution of intermediate 107 (87 mg) in MeOH (2.7 mL). The mixture was heated to 65°C for 2 hour, then it was allowed to cool to r.t., acidified with 1 M hydrochloric acid solution and extracted with DCM. The organic layer was washed with water, dried and concentrated *in vacuo* to give the title compound (75 mg) as a colourless oil.
T.I.c.: CH/AcOEt 1:1, Rf 0.18 (detection with ninhydrine).
MS (ES/-): m/z=350 [M-H]⁻.

### Intermediate 109

### 1 1-dimethylethyl 3-[3-[{[3,5-bis(trifluoromethyl)phenyl]methyl}(methyl)amino]-1-(4-fluorophenyl)-3-oxopropyl]-1-piperidinecarboxylate (109a) (diasteroisomer A) and 1,1-dimethylethyl 3-[3-[{[3,5-bis(trifluoromethyl)phenyl]methyl}(methyl)amino]-1-(4-fluorophenyl)-3-oxopropyl]-1-piperidinecarboxylate (109b) (diasteroisomer B)

TEA (0.090 mL) and O-(7-azabenzotriazol-1-yl)-N,N,N'N'-tetramethyluronium , hexafluorophosphate (0.106 g) were added to a solution of intermediate 108 (0.075 g) in anhydrous DCM (1 mL) under a Nitrogen atmosphere. After stirring for 20 minutes, {[3,5-bis(trifluoromethyl)phenyl}methylamine hydrochloride (0.075 g) was added. The mixture was stirred at r.t. overnight, then it was diluted with AcOEt and washed with water. The organic layer was dried, concentrated *in vacuo* to a residue that was purified by flash chromatography (CH/AcOEt 9:1) to give:
intermediate 109a (17.6 mg) as colourless oil;
intermediate 109b (41.6 mg) as colourless oil.

### Intermediate 109a

T.I.c.: CH/AcOEt 7:3, Rf=0.5.
NMR (CDCl3): δ (ppm) 7.74 (s, 1H); 7.49 (s, 2H); 7.12 (m, 2H); 6.93 (m, 2H); 4.66-4.41 (dd, 2H); 3.80 (m, 1 H); 3.10-2.5 (m, 4H); 2.97 (dd, 2H); 2.87 (s, 3H); 2.60 (m, 1 H); 1.8-1.4 (m, 4H); 1.45 (s, 9H).
MS (ES/+): m/z=491 [M+H-BOC]⁺.

### Intermediate 109b

T.I.c.: CH/AcOEt 7:3, Rf=0.35.
NMR (CDCl3): δ (ppm) 7.76 (s, 1H); 7.51 (s, 2H); 7.4 (m, 2H); 6.94 (m, 2H); 4.66-4.47 (dd, 2H); 4.00 (m, 1H); 3.10-2.0 (m, 4H); 2.97 (dd, 2H); 2.84 (s, 3H); 2.6 (m, 1H); 1.8-1.4 (m, 4H); 1.42 (s, 9H).
MS (ES/+): m/z=491 [M+H-BOC]⁺.

### Intermediate 110

### Ethyl (2E)-2-cyano-3-(4-pyridinyl)-2-propenoate

A mixture of 4-pyridinecarbaldehyde (5.0 g), ethyl cyanoacetate (5.46 mL), ammonium acetate (1.98 g) and acetic acid (2.67 mL) in anhydrous toluene (100 mL) was heated to 85°C for 4 hours, then it was allowed to cool to r..t. and washed with water, 1N sodium hydroxide solution and brine. The organic phase was dried and concentrated *in vacuo* to a residue, which was purified by crystallisation from MeOH to give the title compound (3.29 g) as a white solid.
T.I.c.: AcOEt, Rf=0.39.
NMR (CDCl3): δ (ppm) 8.85 (d, 2H); 8.23 (s, 1H); 7.78 (d, 2H); 4.45 (q, 2H); 1.45 (t, 3H).
MS (ES/+): m/z=203 [M+H]⁺.

### Intermediate 111

### Ethyl 2-cyano-3-(4-fluorophenyl)-3-(4-pyridinyl)propanoate

A solution of 4-fluorophenyl magnesium bromide (1M in THF - 14.83 mL) in anhydrous Et2O (17 mL) was dropped into a solution of intermediate 110 (1.0 g) in anhydrous Et2O (17 mL) previously heated to reflux under a Nitrogen atmosphere. The mixture was heated at reflux for 30 minutes, then cooled to 0°C and treated with 3M sulfuric acid solution (4 mL) diluted with AcOEt and the layers were separated. The aqueous layer was extracted with further AcOEt. The combined organic extracts were washed with a saturated sodium hydrogen carbonate solution then dried and concentrated *in vacuo.* The residue was purified by flash chromatography (CH, then CH/AcOEt from 90:10 to 85:15) to give the title compound (1.1 g) as a white foam.
T.l.c.: AcOEt, Rf=0.39.
MS (ES/+): m/z=299 [M+H]⁺.

### Intermediate 112

### 3-(4-Fluorophenyl)-3-(4-pyridinyl)propanoic acid

A mixture of intermediate 111 (265 mg) in acetic acid (4.5 mL), conc sulfuric acid (1.5 mL) and water (1.5 mL) was heated to 140°C for 3 hours. The solution was allowed to cool to 0°C and saturated sodium hydrogen carbonate solution followed by a 2.5M sodium hydroxide solution were added. AcOEt was added, and the aqueous phase extracted three times with AcOEt. The combined organic extracts were dried and concentrated *in vacuo.* The residue was triturated with AcOEt to give the title compound (105 mg) as a white solid.
IR (nujol, cm⁻¹): 1701 (str. C=O).
MS (ES/+): m/z=246 [M+H]⁺.

### Example 1

### N-(3,5-Bis-trifluoromethyl-benzyl)-3-(4-fluoro-phenyl)-N-methyl-3-piperidin-4-yl-propionamide

TFA (0.8 mL) was added to a solution of intermediate 7 (0.06 g) in DCM (1 mL) under a Nitrogen atmosphere. The resulting solution was stirred at r.t. for 3 hours, then the solution was diluted with further DCM and washed with a saturated potassium carbonate solution and brine. The combined organic extracts were dried, concentrated *in vacuo* and the residue was purified by flash chromatography (from AcOEt to AcOEUMeOH 1:1 and finally to AcOEt/MeOH 4:6 containing 2% of conc. ammonium hydroxide solution) to give the title compound (0.043 g) as a pale yellow oil.
T.l.c.: AcOEbMeOH 1:1 containing 2% of conc. NH4OH, Rf=0.1.
IR (film, cm⁻¹): 3320 (NH), 1646 (C=O).
NMR (d₆-DMSO): δ (ppm) 7.95 (s, 1 H); 7.7 (s. 2H); 7.17 (s, 2H); 6.98 (dd, 2H); 4.54 (q, 2H); 2.94 (s, 3H); 2.92-2.71 (m, 5H); 2.4-2.24 (2td, 2H); 1.69-1.43 (m, 2H); 1.2 (db, 1H); 1.04-0.75 (2qd, 2H).
MS (ES/+): m/z=491 [M+H]⁺.

### Example 2

### N-(3,5-Dichloro-benzyl)-3-(4-fluoro-phenyl)-N-methyl-3-piperidin-4-yl-propionamide

TFA (0.73 mL) was added to a solution of intermediate 8 (0.048 g) in DCM (0.96 mL) under a Nitrogen atmosphere. The resulting solution was stirred at r.t. for 3 hours, then the solution was diluted with further DCM and washed with a saturated potassium carbonate solution and brine. The combined organic extracts were dried, concentrated *in vacuo* and the residue was purified by flash chromatography (from AcOEt to AcOEt/MeOH 1:1 and finally to AcOEt/MeOH 4:6 containing 2% of conc. ammonium hydroxide solution) to give the title compound (0.031 g) as a pale yellow oil.
T.I.c.: AcOEt/MeOH 1:1 containing 2% of conc. NH4OH, Rf=0.08.
IR (film, cm⁻¹): 3320 (NH), 1646 (C=O).
NMR (d₆-DMSO): δ (ppm) 7.43 (t, 1H); 7.2 (dd, 2H); 7.04 (dd, 2H); 6.98 (s, 2H); 4.37 (q, 2H); 2.89 (s, 3H); 2.94-2.7 (m, 5H); 2.4-2.19 (2td, 2H); 1.74-1.15 (m, 3H); 1.04-0.77 (m, 2H).
MS (ES/+): m/z=423 [M+H]⁺.

### Example 3

### N-(3,5-Dichloro-benzyl)-3-(4-fluoro-phenyl)-N-methyl-3-piperidin-4-yl-propionamide 3a) (enantiomer 1) and N-(3,5-Dichloro-benzyl)-3-(4-fluoro-phenyl)-N-methyl-3-piperidin-4-yl-propionamide (3b) (enantiomer 2)

The compound of example 2 (10 mg) was separated into the enantiomers via HPLC (Column: Chiralcel OD 25cm x 4.6mm; mobile phase: n-hexane/EtOH 70:30; flux=1 mL/min; λ=225 nm). Thus, example 3a (3 mg) and example 3b (1.8 mg) were obtained.

### Example 3a:

Chiral HPLC: Column Chiralcel OD 25cm x 4.6mm, mobile phase n-hexane/EtOH 70:30, flux=1 mL/min. λ=225 nm, retention time 5.8 minutes. Ratio **3a**/**3b**=100:0.

### Example 3b:

Chiral HPLC: Column Chiralcel OD 25cm x 4.6mm, mobile phase n-hexane/EtOH 70:30, flux=1 mL/min, λ=225 nm, retention time 10.2 minutes. Ratio 3a/3b=0:100.

### Example 4

### N-[1-(3,5-Dichloro-phenyl)-ethyl]-3-(4-fluoro-phenyl)-N-methyl-3-piperidin-4-yl-propionamide

TFA (1.5 mL) was added to a solution of intermediate 13a (0.17 g) in DCM (6 mL) under a Nitrogen atmosphere. The resulting solution was stirred at r.t. for 30 minutes, then the solution was diluted with further DCM and washed with a saturated potassium carbonate solution. The organic extracts were dried and concentrated *in vacuo* to give the crude title compound (136 mg). A part of the residue (64 mg) was purified on SCX cartridge (ammonium hydroxide 0.25 M in MeOH) to give the title compound (60 mg) as a colourless oil.
NMR (d₆-DMSO): δ (ppm) 7.06 (t, 2H); 7.18 (m, 2H);7.15 (s, 2H); 7.48 (s, 1 H); 5.58 (q, 1H); 2.91-2.8 (2m, 2H); 2.91-2.4 (2 m, 2H); 2.8-2.25 (t + m , 2H); 2.64 (s, 3H); 2.6 (m, 1H); 1.67-0.99 (q + d, 2H); 1.2 (s, 3H); 1.2-0.83 (2m, 2H).
MS (ES/+): m/z=437 [M+H]⁺.

### Example 5

### N-[1-(3,5-Dichloro-phenyl)-ethyl]-3-(4-fluoro-phenyl)-N-methyl-3-piperidin-4-yl-propionamide

TFA (1.5 mL) was added to a solution of intermediate 13b (0.17 g) in DCM (6 mL) under a Nitrogen atmosphere. The resulting solution was stirred at r.t. for 30 minutes, then the solution was diluted with further DCM and washed with a saturated potassium carbonate solution. The organic extracts were dried and concentrated *in vacuo* to give the crude title compound (136 mg). A part of the residue (64 mg) was purified on SCX cartridge (ammonium hydroxide 0.25 M in MeOH) to give the title compound (60 mg) as a colourless oil.
NMR (d₆-OMSO): δ (ppm) 7.42 (s, 1H); 7.03 (t, 2H); 7.2 (dd, 2H);6.85 (s, 2H); 5.6 (q, 1H); 3.0-2.8 (m, 4H); 2.61 (s. 3H); 2.6 (m, 1H); 1.71 (bd, 1H); 1.51-1.0 (2m, 2H); 1.32 (d, 3H); 1.16-0.84- (2m, 2H).
MS (ES/+): rn/z=437 [M+H]⁺.

### Example 6

### N-[1-(3,5-Dichloro-phenyl)-ethyl]-3-(4-fluoro-phenyl)-N-methyl-3-piperidin-4-yl-proplonamide (6a) (diastereoisomer 1) and N-[1-(3,5-Dichloro-phenyl)-ethyl]-3-(4-fluoro-phenyl)-N-methyl-3-piperidin-4-yl-proplonamide (6b) (diastereoisomer 2)

The compound of example 4 (35 mg) was separated into the enantiomers via HPLC (Column: Chiralpack AD 25cm x 4.6mm; mobile phase: n-hexane/EtOH 80:20; flux=7 mL/min: 225 nm). Thus, example 6a (16 mg) and example 6b (14 mg) were obtained.

### Example 6a:

Chiral HPLC: Column Chiralpack AD 25cm x 4.6mm, mobile phase n-hexane/EtOH 80:20, flux=1 mL/min. λ=225 nm, retention time 8.9 minutes. Ratio **6a/6b**=100:0.

### Example 6b:

Chiral HPLC: Column Chiralpack AD 25cm x 4.6mm, mobile phase n-hexane/EtOH 80:20, flux=1 mL/min. λ=225 nm, retention time 10.7 minutes. Ratio 6a/6b=0:100.

### Example 7

### N-[1-(3,5-Dichloro-phenyl)-ethyl]-3-(4-fluoro-phenyl)-N-methyl-3-piperidin-4-yl-propionamide (7a) (diastereoisomer 3) and N-[1-(3,5-Dichloro-phenyl)-ethyl]-3-(4-fluoro-phenyl)-N-methyl-3-piperidin-4-yl-propionamide (7b)-(diastereoisomer 4)

The compound of example 5 (25mg) was separated into the enantiomers via HPLC (Column: Chiralcel OD 25cm x 4.6mm; mobile phase: n-hexane/EtOH 8:2; flux=1 mL/min; λ=225 nm). Thus, example 7a (9 mg) and example 7b (9 mg) were obtained.

### Example 7a:

Chiral HPLC: Column Chiralcel OD 25cm x 4.6mm, mobile phase n-hexane/EtOH 8:2, flux=1 mL/min, λ=225 nm, retention time 6.7 minutes. Ratio 7a/7b=100:0.

### Example 7b:

Chiral HPLC: Column Chiralcel OD 25cm x 4.6mm, mobile phase n-hexane/EtOH 8:2, flux=1 mL/min, λ=225 nm, retention time 10.1 minutes. Ratio 7a/7b=0:100.

### Example 8

### N-[1-(3,5-Dichloro-phenyl)-ethyl]-3-(4-fluoro-phenyl)-N-methyl-3-[1-(2-methoxyethyl)-piperidin-4-yl]-propionamide (diastereoisomer A)

A mixture of example 4 (72 mg), DIPEA (115 µL) and 2-bromoethyl methyl ether (19 µL) in acetonitrile (5 mL) was heated to reflux overnight. Water was added and the mixture was concentrated *in vacuo.* The residue was dissolved in AcOEt and washed with a saturated ammonium chloride solution. The layers were separated. The organic layer was extracted with further AcOEt. The combined organic extracts were dried and concentrated *in vacuo* to a residue which was purified by flash chromatography (DCM/MeOH 9:1) to give the title compound (57 mg) as a colourless oil.
NMR (d₈-DMSO): δ (ppm) 7.48 (s, 1H); 7.2 (m, 2H); 7.15 (s, 2H); 7.06 (t, 2H); 5.58 (q, 1H); 3.35 (t, 2H); 3.18 (s, 3H); 2.94-2.86 (2m, 2H); 2.9-2.4 (2 m, 2H); 2.8-2.25 (t + m , 2H); 2.63 (s, 3H); 2.6 (m, 1H); 2.37 (dd, 2H); 1.85-1.12 (q + t, 2 H); 1.73-0.97 (2q, 2H); 1.2 (s, 3H);.
MS (ES/+): m/z=495 [M+H]⁺.

### Example 9

### N-[1-(3,5-Dichloro-phenyl)-ethyl]-3-(4-fluoro-phenyl)-N-methyl-3-[1-(2-methoxyethyl)-piperidin-4-yl]-propionamide (diastereoisomer B)

A mixture of example 5 (79 mg), DIPEA (126 µL) and 2-bromoethyl methyl ether (20 µL) in acetonitrile (5 mL) was heated to reflux overnight. Water was added and the mixture was concentrated *in vacuo.* The residue was dissolved in AcOEt and washed with a saturated ammonium chloride solution. The layers were separated. The organic layer was extracted with further AcOEt. The combined organic extracts were dried and concentrated *in vacuo* to a residue, which was purified by flash chromatography (DCM/MeOH 95:5) to give the title compound (35 mg) as a colourless oil.
NMR (d₆-DMSO): δ (ppm) 7.42 (s, 1H); 7.15 (s, 2H); 7.04 (t, 2H); 6.85 (s, 2H); 5.6 (q, 1 H); 3.37 (t, 2H); 3.19 (s, 3H); 3.0-2.8 (m , 6H); 2.61 (s, 3H); 2.41 (bs, 2H); 1.76 (d, 1H); 1.45-1.21 (2m, 2H); 1.32 (d, 3H); 1.27-0.99 (q, 2H); 1.21 (m, 1 H).
MS (ES/+): m/z=495 [M+H]⁺.

### Example 10

### N-(3,5-Dichloro-benzyl)-3-(4-fluoro-phenyl)-3-(4-fluoro-piperidin-4-yl)-N-methylproprionamide

A solution of TFA (1 mL) in anhydrous DCM (3 mL) was added to a solution of intermediate 23 (64 mg) in anhydrous DCM (1 mL) previously cooled to -10°C under a Nitrogen atmosphere. The resulting solution was stirred at -10°C for 2 hours, then the solution was concentrated *in vacuo.* The residue was diluted with AcOEt/water and washed with a saturated potassium carbonate solution. The aqueous layer was extracted with further AcOEt (3 x 20 mL). The combined organic extracts were dried and concentrated *in vacuo* to a residue which was purified by flash chromatography (DCM/MeOH 8:2) to give the title compound (40 mg) as a white foam.
T.l.c.: DCM/MeOH 8:2, Rf=0.1.
NMR (d₆-DMSO): δ (ppm) 7.42 (t, 1H); 7.28 (dd, 2H); 7.07 (dd, 2H); 6.95 (d, 2H); 4.51 (d, 1H); 4.23 (d, 1 H); 3.38 (m, 1H); 2.97 (dd, 1 H); 2.94 (s, 3H); 2.86 (dd, 1H); 2.74 (m, 1H); 2.66 (m, 1H); 2.63 (m, 1 H); 2.54 (m, 1H); 1.78 (m, 1 H); 1.47 (m, 1H); 1.4 (m, 1 H); 1.34 (m, 1 H).

### Example 11

### N-(3,5-Dichloro-benzyl)-3-(4-fluoro-phenyl)-3-(4-fluoro-piperidin-4-yl)-N-methylproprionamide hydrochloride

Hydrochloric acid (1 m in Et2O - 20 µL) was added to a solution of example 10 (7.8 mg) in anhydrous Et2O (1 mL) previously cooled to 0°C under a Nitrogen atmosphere. The mixture was stirred at 0°C for 15 minutes, then the liquid phase was removed. The residue was triturated with pentane (2 x 1 mL) and with pentane/Et2O 1:1 (1.5 mL) to give the title compound (5 mg) as a white solid.
MS (ES/+): m/z=441 [M+H-HCl]⁺.

### Example 12

### N-(3,5-Dichloro-benzyl)-3-(4-fluoro-phenyl)-3-(4-fluoro-piperidin-4-yl)-N-methylproprionamide (12a - enantiomer 1) and N-(3,5-Dichloro-benzyl)-3-(4-fluoro-phenyl)-3-(4-fluoro-piperidin-4-yl)-N-methylproprionamide (12b - enantiomer 2)

The compound of example 10 (32 mg) was separated into the enantiomers via HPLC (Column: Chiralcel OD 25cm x 20mm; mobile phase: n-hexane/EtOH 70:30; flux=6.4 mL/min; 225 nm). Thus, example 12a (15 mg) and example 12b (14 mg) were obtained.

### Example 12a:

Chiral HPLC: Column Chiralcel OD 25cm x 4.6mm x 5µ, mobile phase n-hexane/EtOH 80:20, flux=1 mL/min. λ=225 nm, retention time 8.1 minutes. Ratio 12a/12b=100:0.

### Example 12b:

Chiral HPLC: Column Chiralcel OD 25cm x 4.6mm x 5µ, mobile phase n-hexane/EtOH 80:20, flux=1 mL/min, λ=225 nm, retention time 22.4 minutes. Ratio **12a/12b**=0:100.

### Example 13

### N-(3,5-Dichloro-benzyl)-3-(4-fluoro-phenyl)-3-(4-fluoro-piperidin-4-yl)-N-methylproprionamide hydrochloride

Hydrochloric acid (1M in Et2O - 38 µL) was added to a solution of example 12a (15 mg) in anhydrous Et2O (1.5 mL) previously cooled to -10°C under a Nitrogen atmosphere. The mixture was stirred at ―10°C for 30 minutes, then it was concentrated *in vacuo.* The residue was triturated with pentane (2 x 1 mL) and pentane/Et2O 1:1 (1.5 mL) to give the title compound (11 mg) as white solid.
Chiral HPLC: Column Chiralcel OD 25cm x 4.6mm x 5µ, mobile phase n-hexane/EtOH 80:20, flux=1 mL/min, λ=225 nm, retention time 7.96 minutes.
MS (ES/+): m/z=441 [M+H-HCl]⁺.

### Example 14

### N-(3,5-Dichloro-benzyl)-3-(4-fluoro-phenyl)-3-(4-fluoro-piperidin-4-yl)-N-methylproprionamide hydrochloride

Hydrochloric acid (1M in Et2O - 35 µL) was added to a solution of example 12b (14 mg) in anhydrous Et2O (1.5 mL) previously cooled to -10°C under a Nitrogen atmosphere. The mixture was stirred at -10°C for 30 minutes, then it was concentrated *in vacuo.* The residue was triturated with pentane (2 x 1 mL) and pentane/Et2O 1:1 (1.5 mL) to give the title compound (10 mg) as a white solid.
Chiral HPLC: Column Chiralcel OD 25cm x 4.6mm x 5µ, mobile phase n-hexane/EtOH 80:20, flux=1 mL/min, λ=225 nm, retention time 22.2 minutes.
NMR (d₆-DMSO): δ (ppm) 8.39 (bm, 1H); 7.39 (s, 1H); 7.31 (dd, 2H); 7.08 (dd, 2H); 6.92 (s, 2H); 4.5 (d, 1H); 4.18 (d, 1H); 3.47 (m, 1H); 3.17 (m, 1 H); 3.12 (m, 1H); 3.02 (dd, 1H); 2.92 (s, 3H); 2.89 (m, 1H); 2.85 (dd, 1H); 2.82 (m, 1H); 2.06 (m, 1H); 1.75 (m, 1H); 1.65 (m, 2H);
MS (ES/+): m/z=441 [M+H-HCl]⁺.

### Example 15

### N-(3,5-Bis-trifluoromethyl-benzyl)-3-(4-fluoro-phenyl)-N-methyl-3-piperidin-4-yl-propionamide hydrochloride

Hydrochloric acid (1M in Et2O - 12 µL) was added to a solution of example 1 (5 mg) in anhydrous Et2O (0.5 mL) previously cooled to 0°C under a Nitrogen atmosphere. The mixture was stirred at 0°C for 15 minutes, then it was concentrated *in vacuo.* The residue was triturated with pentane (2 x 1 mL) and with pentane/Et2O 1:1 (1.5 mL) to give the title compound (5 mg) as a white solid.
MS (ES/+): m/z=491 [M+H]⁺.

### Example 16

### N-{[3,5-bis(trifluoromethyl)phenylmethyl}-3-(4-fluorophenyl)-N-methyl-3-{1-[2-(methyloxy)ethyl]-4-piperidinyl}propionamide

A mixture of example 1 (10 mg), DIPEA (14 µL) and 2-bromoethyl methyl ether (2.1 µL) in acetonitrile (0.5 mL) was heated to reflux overnight. Water was added and the mixture was concentrated *in vacuo.* The residue was dissolved in DCM and washed with water. The layers were separated. The aqueous layer was extracted with further DCM (2 x 5 ml). The combined organic extracts were dried and concentrated *in vacuo* to a residue, which was purified by flash chromatography (from AcOEt to AcOEt/MeOH 4:6) to give the title compound (6 mg) as a colourless oil.
T.l.c.: AcOEt/MeOH 1:1, Rf=0.18.
MS (ES/+): m/z=549 [M+H]⁺.

### Example 17

### N-{[3,5-bis(trifluoromethyl)phenyl]methyl}-3-(4-fluorophenyl)-N-methyl-3-{1-[2-(methyloxy)ethyl]-4-piperidinyl} propionamide hydrochloride

Hydrochloric acid (1M in Et2O - 13.2 µL) was added to a solution of example 16 (6 mg) in anhydrous Et2O (0.5 mL) previously cooled to 0°C under a Nitrogen atmosphere. The mixture was stirred at 0°C for 15 minutes, then the liquid phase was removed. The residue was triturated with pentane (2 x 1 mL) and with pentane/Et2O 1:1 (1.5 mL) to give the title compound (5 mg) as a white solid.
MS (ES/+): m/z=549 [M+H]⁺.

### Example 18

### N-{(1R)-1-[3,5-bis(trifluoromethyl)phenyl]ethyl}-3-(4-fluorophenyl)-N-methyl-3-(4-piperidinyl)propionamide hydrochloride (diastereoisomer 1)

TFA (1 mL) was added to a solution of intermediate 24a (28 mg) in DCM (5 mL) under a Nitrogen atmosphere. The resulting solution was kept at -18°C overnight, then the solution was diluted with further DCM and washed with a saturated potassium carbonate solution. The combined organic extracts were dried, concentrated *in vacuo.* The residue was purified by flash chromatography (AcOEt/MeOH 1:1 and finally to AcOEt/MeOH 1:1 containing 1% of TEA) to give a compound (15 mg) that was dissolved in Et2O (0.7 mL). To this solution cooled to 0°C under a Nitrogen atmosphere hydrochloric acid (1 M in Et2O - 32 µL) was added. The mixture was stirred at 0°C for 30 minutes, then it was concentrated *in vacuo.* The residue was triturated with pentane (2 x 1 mL) and with pentane/Et2O 1:1 (1.5 mL) to give the title compound (13 mg) as a white solid.
NMR (d₆-DMSO): δ (ppm) 7.97 (s, 1H); 7.74 (s, 2H); 7.19 (dd, 2H); 7.06 (dd, 2H); 5.66 (q, 1 H); 3.20 (bd, 1 H); 3.12 (bd, 1 H); 2.97 (m, 1 H); 2.79 (dd, 1 H); 2.76 (dd, 1 H); 2.69 (m, 1 H); 2.67 (s, 3H); 2.64 (m, 1 H); 1.87 (bd, 1 H); 1.74 (m, 1H); 1.40 (bd, 1H); 1.29 (d, 3H); 1.22 (m, 1 H); 1.09 (bq, 1H).

### Example 19

### N-{(1R)-1-[3,5-bis(trifluoromethyl)phenyl]ethyl}-3-(4-fluorophenyl)-N-methyl-3-(4-piperidinyl)propionamide hydrochloride(diastereoisomer 2)

TFA (1 mL) was added to a solution of intermediate 24b (31 mg) in DCM (5 mL) under a Nitrogen atmosphere. The resulting solution was kept at -18°C overnight, then the solution was diluted with further DCM and washed with a saturated potassium carbonate solution. The combined organic extracts were dried, concentrated *in vacuo.* The residue was purified by flash chromatography (AcOEt/MeOH 1:1 and finally to AcOEt/MeOH 1:1 containing 1% of TEA) to give a compound (free base, 11 mg) that was dissolved in Et2O (0.7 mL). To this solution cooled to 0°C under a Nitrogen atmosphere hydrochloric acid (1M in Et2O - 21 µL). The mixture was stirred at 0°C for 30 minutes, then it was concentrated *in vacuo.* The residue was triturated with pentane (2 x 1 mL) to give the title compound (11 mg) as a white solid.
NMR (d₆-OMSO): δ (ppm) 7.92 (s, 1 H); 7.54 (s. 2H); 7.18 (dd, 2H); 6.98 (dd, 2H); 5.71 (q, 1 H); 3.23 (bd, 1H); 3.13 (bd, 1H): 2.98 (m, 1 H); 2.85 (dd, 1 H); 2.76 (dd, 1 H); 2.66 (s, 3H); 2.66 (m, 1H): 2.65 (m, 1 H); 1.88 (bd, 1H); 1.74 (m, 1 H); 1.41 (d, 3H); 1.39 (bd, 1H): 1.25 (m, 1 H); 1.09 (bq, 1H).

### Example 20

### N-{(1S)1-[3,5-bis(trifluoromethyl)phenyl]ethyl}-3-(4-fluorophenyl)-N-methyl-3-(4-piperidinyl)propionamide (diastereoisomer 1)

TFA (1.5 ml) was added to a solution of intermediate 25a (198 mg) in DCM (6 ml) under a Nitrogene atmosphere. The resulting solution was stirred at r.t. for 30 min, then it was diluted with DCM and washed with a saturated potassium carbonate solution and brine. The organic layer was dried, concentrated *in vacuo* and the residue was purified by SCX cartridge (load with DCM and elution with MeOH, NH₃ 0.25M in MeOH) to give the title compound as a colourless foam.
NMR (d₆-DMSO): δ (ppm) 7.95 (s, 1H); 7.73-7.56 (s, 2H); 7.16-6.86 (m, 2H); 5.68-5.44 (m, 1 H); 2.65 (s, 3H); 3.0-2.2 (m, 4H); 3.0-2.2 (m, 2H); 3.0-2.5 (m, 1H); 1.7-0.7 (m, 5H); 1.26 (d, 3H).
MS (ES/+): m/z=505 [M+H]⁺.

### Example 21

### N-{(1S)-1-(3,5-bis(trifluoromethyl)phenyl]ethyl}-3-(4-fluorophenyl)-N-methyl-3-(4-piperidinyl)propionamide (diastereoisomer 2)

TFA (1.5 ml) was added to a solution of intermediate 25b (165 mg) in DCM (6 ml) under a Nitrogene atmosphere. The resulting solution was stirred at r.t. for 30 min, then it was diluted with DCM and washed with a saturated potassium carbonate solution and brine. The organic layer was dried, concentrated *in vacuo* and the residue was purified by by SCX cartridge (load with DCM and elution with MeOH, NH₃ 0.25M in MeOH) to give the title compound as a colourless foam.
NMR (d₆-DMSO): δ (ppm) 7.94 (s, 1H); 7.56 (s, 2H); 7.17 (m, 2H); 6.96 (t, 2H); 5.74 (q, 1 H); 3.00-2.7 (m, 4H); 2.8-2.7 (m, 1 H); 2.4-2.2 (m, 2H); 1.7-1.0 (m, 2H); 1.5 (m, 1H); 1.2-0.8 (m, 2H); 2.67 (s, 3H); 1.43 (d, 3H).
MS (ES/+): m/z=505 [M+H]⁺.

### Example 22

### N-{1-[3,5-bis(trifluoromethyl)phenyl]-1-methylethyl}-3-(4-fluorophenyl)-3-(4-piperidinyl)propionamide

TFA (0.5 mL) was added to a solution of intermediate 26 (29 mg) in anhydrous DCM (1 mL) previously cooled at 0°C, under a Nitrogen atmosphere. The solution was stirred at 0°C for 1 hour, then it was evaporated at 0°C and treated with a 10% sodium hydrogen carbonate solution until pH=10. The aqueous layer was extracted with DCM (3 x 8 mL). The combined organic extracts were dried, concentrated *in vacuo* and the residue was purified by flash chromatography (DCM/MeOH from 98:2 to 75:25) to give the title compound as a white solid (17 mg).
T.I.c.: DCM/MeOH 8:2, Rf=0.03 (detection with ninhydrine).
NMR (CDCl3): δ (ppm) 7.69 (s, 1H); 7.60 (s, 2H); 7.12 (dd, 2H); 7.01 (t, 2H); 5.45 (s, 1H); 3.11 (d, 1H); 2.99 (d, 1H); 2.84 (m, 1H); 2.69 (dd,1H); 2.57 (dt, 1H); 2.46 (dt, 1H); 2.33 (dd, 1H); 1.79 (d, 1H); 1.60 (m, 1H); 1.46 (s, 3H); 1.43 (s, 3H); 1:33 (d, 1H); 1.16 (dt, 1H); 1,00 (dt, 1H).
MS (ES/+): m/z=505 [M+H]⁺.

### Example 23

### N-{1-[3,5-bis(trifluoromethyl)phenyl]-1-methylethyl}-3-(4-fluorophenyl)-N-methyl-3-(4-piperidinyl)propionamide

TFA (0.5 mL) was added to a solution of intermediate 27 (50 mg) in anhydrous DCM (1 mL) previously cooled at 0°C, under a Nitrogen atmosphere. The solution was stirred at 0°C for 1 hour, then it was evaporated at 0°C and treated with a 10% sodium hydrogen carbonate solution until pH=10. The aqueous layer was extracted with DCM (3 x 8 mL). The combined organic extracts were dried, concentrated *in vacuo* and the residue was purified by flash chromatography (DCM/MeOH from 100:0 to 75:25) to give the title compound as a white solid (23 mg).
T.l.c.: DCM/MeOH 8:2, Rf=0.05 (detection with ninhydrine).
NMR (CDCl3): δ (ppm) 7.64 (s, 1H); 7.54 (s, 2H); 7.06 (dd, 2H); 6.96 (t, 2H); 3.19 (d, 1H); 3.09 (d, 1H); 3.03 (s, 3H); 2.95 (m, 1 H); 2.71 (dd, 1H); 2.64 (m, 1H); 2.61 (dt, 1H); 2.52 (dt, 1H); 1.90 (m, 1H); 1.62 (m, 1H); 1.53 (s, 3H); 1.50 (s, 3H); 1.39 (d, 1 H); 1.27 (m, 1H); 1.12 (m, 1H).
MS (ES/+): m/z=519 [M+H]⁺.

### Example 24

***N*-{[3-bromo-4-(methyloxy)phenyl]methyl]-3-(4-fluorophenyl)-*N*-methyl-3-(4-piperidinyl)propionamide** Intermediate 28 were dissolved in dry DCM (3.6 ml) and trifluoroacetic acid (400 µl) was added. The mixtures were stirred at r.t. for 3 hours, then they were concentrated *in vacuo.* The residue was purified by semipreparative HPLC (column: X Terra MS C18 100x30mm, 5µm; mobile phase: A: H₂O+0.1% TFA; B: CH₃CN+0.1% TFA; gradient: 30% (B) for 1 min, from 30% (B) to 90% (B) in 12 min., 90% (B) for 2 min; flow rate=43 mL/min; detection: λ=200-400 nm); the solution recovered was concentrated *in vacuo,* and the residue was dissolved in 5% sodium hydrogen carbonate solution (5 mL) and extracted with DCM (2 x 8 mL), which was passed through a phase separation cartridge with polypropylene frit and concentrated *in vacuo.* to obtain the title compound (14 mg) as a yellow foam.
NMR (CDCl3): δ (ppm) 7.27-6.72 (m, 7H); 4.55 (dd, 1 H); 4.19 (dd, 1H); 3.87 (s, 3H); 3.19-3.04 (m, 1H); 3.19-3.04 (m, 2H); 2.79 (s, 3H); 2.82-2.7 (m, 2H); 2.82-2.49 (m, 2H); 1.7 (m, 1H); 1.7/1.4-1.0 (m, 2H); 1.4-1.0 (m, 2H).
MS (ES/+): m/z=463 [M+H]⁺.
Following the general procedure described for the preparation of example 24 the Examples 25-28 were obtained :

### Example 25

***N*-[(3,5-dimethylphenyl)methyl]-3-(4-fluorophenyl)-*N*-methyl-3-(4-piperidinyl)propionamide** Starting from intermediate 29 the title compound (20 mg) was obtained as a yellow foam.
NMR (CDCl3): δ (ppm) 7.15 (dd, 2H); 6.97 (t, 2H); 6.86 (d, 1 H); 6.62 (s, 2H); 4.52 (d, 1H); 4.28 (d, 1 H); 3.18 (m, 1 H); 3.13 (m, 1 H); 3.05 (m, 1H); 2.80 (s, 3H); 2.76 (dd, 1H); 2.66 (dd, 1H); 2.63 (m, 1H); 2.52 (m, 1H); 2.28 (s, 3H); 2.24 (s, 3H); 1.87 (bd, 1H); 1.67 (m, 1H); 1.40 (m, 1H);1.28 (m, 1 H); 1.08 (m, 1H)
MS (ES/+): m/z=383 [M+H]⁺.

### Example 26

***N*-[(3,4-dibromophenyl)methyl]-3-(4-fluorophenyl)-*N*-methyl-3-(4-piperidinyl)propionamide** Starting from intermediate 30 the title compound (15 mg) was obtained as a yellow foam.
NMR (CDCl3): δ (ppm) 7.44 (d, 1H); 7.29 (d, 1H); 7.14 (dd, 2H); 6.93 (dd, 2H); 6.63 (dd, 1H); 4.55 (d, 1H); 4.21 (d, 1H); 3.13 (bd, 1H); 3.08 (m, 1H); 3.00 (bd, 1H); 2.82 (s, 3H); 2.76 (dd, 1 H); 2.70 (dd, 1 H); 2.59 (m, 1H); 2.48 (m, 1H); 1.83 (bd, 1H); 1.62 (m, 1H); 1.36 (bd, 1H); 1.21 (m, 1H); 1.02 (m, 1H);

### Example 27

### N-[(3-fluoro-2-methylphenyl)methyl]-3-(4-fluorophenyl)-N-methyl-3-(4-piperidinyl)propionamide,

Starting from intermediate **31** the title compound (26 mg) was obtained as a yellow foam.
NMR (CDCl3): δ (ppm) 7.17 (m. 2H); 7.10-6.85 (m, 4H); 6.35 (d, 1 H); 4.71 (d, 1 H); 4.28 (d, 1H); 4.32 (dd, 2H); 3.2-2.95 (m, 4H); 2.75-2.4 (m, 3H); 2.86 (s, 3H); 2.02 (s, 3H); 1.9-1.6 (m, 3H); 1.4-0.95 (m, 2H).

### Example 28

***N*-{[2-chloro-3-(trifluoromethyl)phenyl]methyl}-3-(4-fluorophenyl)-*N*-methyl-3-(4-piperidinyl)propionamide** Starting from intermediate 32 the title compound (8 mg) was obtained as a yellow foam.
NMR (CDCl3): δ (ppm) 7.56 (d, 2H); 7.20-7.10 (m, 2H); 7.05-7.00 (m, 2H); 6.93 (t, 1H); 6.54 (d, 1H); 4.88 (d, 1H); 4.50 (dd, 2H); 4.39 (d, 1H); 3.2-2.95 (m, 4H); 2.80-2.40 (m, 3H); 2.92 (s, 3H); 1.90-1.60 (m, 3H); 1.4-0.95 (m. 2H).

### Example 29

### N-{(1R)-1-[3,5-bis(trifluoromethyl)phenyl]ethyl}-3-(4-fluorophenyl)-3-(4-fluoro-4-piperidinyl)-N-methylpropionamide (diastereoisomer 1)

TFA (1.0 mL) was added to a solution of intermediate 33a (50 mg) in anhydrous DCM (4 mL) previously cooled at 0°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried, concentrated *in vacuo* and the residue was purified by flash chromatography (DCM/MeOH from 9/1 to 7/3) to give the title compound (0.033 g) as a white foam.
T.I.c.: DCM/MeOH 8:2, Rf=0.15.
MS (ES/+): m/z=523 [M+H]⁺.

### Example 30

### N-{(1R)-1-(3,5-bis(trifluoromethyl)phenyl]ethyl}-3-(4-fluorophenyl)-3-(4-fluoro-4-piperidinyl)-N-methylpropionamide hydrochloride (diastereoisomer 1)

Hydrochloric acid (1 M in Et2O - 70 µL) was added to a solution of example 29 (33 mg) in anhydrous Et2O (1 mL) previously cooled to 0°C under a Nitrogen atmosphere. The mixture was stirred at 0°C for 15 minutes, then the liquid phase was removed. The residue was triturated with pentane (2 x 1 mL) and with pentane/Et2O 1:1 (1.5 mL) to give the title compound (33mg) as a white solid.
T.I.c.: DCM/MeOH 8:2, Rf=0.15.
NMR (d₆-DMSO): δ (ppm) 8.75 (bm, 1H); 8.4 (bm, 1H); 8.0 (s, 1H); 7.8 (s, 2H); 7.25 (m, 2H); 7.1 (m, 2H); 5.65 (q, 1H); 3.55 (m, 1 H); 3.2 (bm, 2H); 3.05-2.75 (m, 4H); 2.74 (s, 3H); 2.05 (bm, 1H); 1.75 (bm, 3H); 1.35 (d, 3H).
MS (ES/+): m/z=523 [M+H-HCl]⁺.

### Example 31

### N-{(1R)-1-[3,5-bis(trifluoromethyl)phenyl]ethyl}-3-(4-fluorophenyl)-3-(4-fluoro-4-piperidinyl)-N-methylpropionamide (diastereoisomer 2)

TFA (1.0 mL) was added to a solution of intermediate **33b** (55 mg) in anhydrous DCM (4 mL) previously cooled at 0°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried, concentrated *in vacuo* and the residue was purified by flash chromatography (DCM/MeOH from 9/1 to 7/3) to give the title compound (0.036 g) as a white foam.
T.l.c.: DCM/MeOH 8:2, Rf=0.15.
MS (ES/+): m/z=523 [M+H]⁺.

### Example 32

### N-{(1R)-1-[3,5-bis(trifluoromethyl)phenyl]ethyl}-3-(4-fluorophenyl)-3-(4-fluoro-4-piperidinyl)-N-methylpropionamide hydrochloride (diastereoisomer 2)

Hydrochloric acid (1 M in Et2O - 76 µL) was added to a solution of example 31 (36 mg) in anhydrous Et2O (1 mL) previously cooled to 0°C under a Nitrogen atmosphere. The mixture was stirred at 0°C for 15 minutes, then the liquid phase was removed. The residue was triturated with pentane (2 x 1 mL) and with pentane/Et2O 1:1 (1.5 mL) to give the title compound (34mg) as a white solid.
T.I.c.: DCM/MeOH 8:2, Rf=0.15.
NMR (d₆-DMSO): δ (ppm) 8.6 (bs, 2H); 7.95 (s, 1H); 7.55 (s, 2H); 7.35 (t, 2H); 7.05 (t, 2H); 5.7 (q, 1 H); 3.5 (m, 1H); 3.25-2.8 (m, 6H); 2.75 (s, 3H); 2.05 (bm, 1 H); 1.8 (bm, 3H); 1.45 (d, 3H).
MS (ES/+): mlz=523 [M+H-HCl]⁺.

### Example 33

### N-{(1S)-1-[3,5-bis(trifluoromethyl)phenyl]ethyl}-3-(4-fluorophenyl)-3-(4-fluoro-4-piperidinyl)-N methylpropionamide (diastereoisomer 1)

TFA (1.0 mL) was added to a solution of intermediate intermediate 34a (52 mg) in anhydrous DCM (4 mL) previously cooled at 0°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried, concentrated *in vacuo* and the residue was purified by flash chromatography (DCM/MeOH from 9/1 to 7/3) to give the title compound (0.042 g) as a white foam.
T.I.c.: DCM/MeOH 8:2, Rf=0.15.
MS (ES/+): m/z=523 [M+H]⁺.

### Example 34

### N-{(1S)-1-[3,5-bis(trifluoromethyl)phenyllethyl}-3-(4-fluorophenyl)-3-(4-fluoro-4-piperidinyl)-N-methylpropionamide hydrochloride (diastereoisomer 1)

Hydrochloric acid (1 M in Et2O - 90 µL) was added to a solution of example 33 (42 mg) in anhydrous Et20 (1 mL) previously cooled to 0°C under a Nitrogen atmosphere. The mixture was stirred at 0°C for 15 minutes, then the liquid phase was removed. The residue was triturated with pentane (2 x 1 mL) and with pentane/Et2O 1:1 (1.5 mL) to give the title compound (42mg) as a white solid.
T.I.c.: DCM/MeOH 8:2, Rf=0.15.
NMR (d₆-DMSO): δ (ppm) 8.75 (bm, 1H); 8.4 (bm, 1H); 8.0 (s, 1H); 7.8 (s, 2H); 7.25 (m, 2H); 7.1 (m, 2H); 5.65 (q, 1 H); 3.55 (m, 1H); 3.2 (bm, 2H); 3.05-2.75 (m, 4H); 2.74 (s, 3H); 2.05 (bm, 1H): 1.75 (bm, 3H); 1.35 (d, 3H).
MS (ES/+): m/z=523 [M+H-HCl]⁺.

### Example 35

### N-{(1S)-1-[3,5-bis(trifluoromethyl)phenyl]ethyl}-3-(4-fluorophenyl)-3-(4-fluoro-4-piperidinyl)-N-methylpropionamide (diastereoisomer 2)

TFA (1.0 mL) was added to a solution of intermediate 34b (50 mg) in anhydrous DCM (4 mL) previously cooled at 0°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried, concentrated *in vacuo* and the residue was purified by flash chromatography (DCM/MeOH from 9/1 to 7/3) to give the title compound (0.036 g) as a white foam.
T.l.c.: DCM/MeOH 8:2, Rf=0.15.
MS (ES/+): m/z=523 [M+H]⁺.

### Example 36

### N-{(1S)-1-[3,5-bis(trifluoromethyl)phenyl]ethyl}-3-(4-fluorophenyl)-3-(4-fluoro-4-piperidinyl)-N-methylpropionamide hydrochloride (diastereoisomer 2)

Hydrochloric acid (1 M in Et2O - 80 µL) was added to a solution of example 35 (36 mg) in anhydrous Et2O (1 mL) previously cooled to 0°C under a Nitrogen atmosphere. The mixture was stirred at 0°C for 15 minutes, then the liquid phase was removed. The residue was triturated with pentane (2 x 1 mL) and with pentane/Et2O 1:1 (1.5 mL) to give the title compound (33mg) as a white solid.
T.I.c.: DCM/MeOH 8:2, Rf=0.15.
NMR (d₆-DMSO): δ (ppm) 8.6 (bs, 2H); 7.95 (s, 1 H); 7.55 (s, 2H); 7.35 (t, 2H); 7.05 (t, 2H); 5.7 (q, 1 H); 3.5 (m, 1H); 3.25-2.8 (m, 6H); 2.75 (s, 3H); 2.05 (bm, 1H); 1.8 (bm, 3H); 1.45 (d. 3H).
MS (ES/+): m/z=523 [M+H-HCl]⁺.

### Example 37

### N-[(3,5-dibromophenyl)methyl]-3-(4-fluorophenyl)-3-(4-fluoro-4-piperidinyl)-N-methylpropionamide (37a) (enantiomer 1) and N-[(3,5-dibromophenyl)methyl]-3-(4-fluorophenyl)-3-(4-fluoro-4-piperidinyl)-N-methylproplonamide (37b) (enantiomer 2)

TFA (15 mL) was added to a solution of intermediate 38 (765 mg) in anhydrous DCM (40 mL) previously cooled to -10°C under a Nitrogen atmosphere. The solution was left in freezer overnight, then it was concentrated *in vacuo.* The residue was dissolved in DCM and washed with saturated potassium carbonate solution. The aqueous layer was extracted with further DCM. The organic extract was washed with brine, dried and concentrated *in vacuo* to give the title compound (565 mg) as racemate. The mixture was separated into the enantiomers via HPLC (Column: Chiralcel OD 25cm x 2cm; mobile phase: n-hexane/2-Propanol 70:30; flux=7 mL/min; λ=225 nm).
Thus, example 37a (263 mg) and example 37b (264 mg) were obtained.

### Example 37a:

Chiral HPLC: Column Chiralcel OD 25cm x 4.6mm; mobile phase: n-hexane/2-Propanol 80:20; flux=1 mL/min; λ=225 nm, retention time 18.4 minutes. ratio **37a/37b**=100/0
MS (ES/+): m/z=439 [M+H]⁺.

### Example 37b:

Chiral HPLC: Column Chiralcel OD 25cm x 4.6mm; mobile phase: n-hexane/2-Propanol 80:20; flux=1 mL/min; λ=225 nm, retention time 35 minutes. ratio 37a/37b=0/100
MS (ES/+): m/z=439 [M+H]⁺.**Example 38**
***N*-[(3,5-dibromophenyl)methyl]-3-(4-fluorophenyl)-3-(4-fluoro-4-piperidinyl)-*N-*methylpropionamide hydrochloride** Hydrochloric acid (1M in Et2O- 0.6 mL) was added to a solution of example 37a (261 mg) in anhydrous Et2O (5 mL) previously cooled to -10°C under a Nitrogen atmosphere. The mixture was stirred at -10°C for 10 minutes, then it was concentrated *in vacuo.* The residue was triturated with pentane (3 x 5 mL) to give the title compound (264 mg) as white solid.
NMR (d₆-DMSO): δ (ppm) 8.54 (sb, 2H); 7.66 (s, 1H); 7.35 (dd, 2H); 7.18 (s, 2H); 7.13 (t, 2H); 4.49-4.25 (dd, 2H); 3.55-3.46 (2dd, 1H); 3.24-2.84 (m, 6H); 2.68 (s, 3H); 2.09 (m, 1H); 1.84-1.65 (m, 3H).

### Example 39

### N-[(3,5-dibromophenyl)methyl]-3-(4-fluorophenyl)-3-(4-fluoro-4-piperidinyl)-N-methylproplonamide hydrochloride

Hydrochloric acid (1M in Et2O - 0.6 mL) was added to a solution of example 37b (261 mg) in anhydrous Et2O (5 mL) previously cooled to -10°C under a Nitrogen atmosphere. The mixture was stirred at -10°C for 10 minutes, then it was concentrated *in vacuo.* The residue was triturated with pentane (3 x 5 mL) to give the title compound (265 mg) as white solid.
NMR (d₆-DMSO): δ (ppm) 8.54 (sb, 2H); 7.66 (s, 1H): 7.35 (dd, 2H); 7.18 (s, 2H); 7.13 (t, 2H); 4.49-4.25 (dd, 2H); 3.55-3.46 (2dd, 1H);); 3.24-2.84 (m, 6H); 2.68 (s. 3H); 2.09 (m, 1 H); 1.84-1.65 (m, 3H).

### Example 40

### N-{(1R)-1-[3,5-bis(trifluoromethyl)phenyl]ethyl}-3-(2,4-dichlorophenyl)-3-(4-fluoro-4-piperidinyl)-N-methylpropionamide-(diastereoisomer 1)

TFA (1 mL) was added to a solution of intermediate 45a (51 mg) in anhydrous DCM (3 mL) previously cooled to -10°C under a Nitrogen atmosphere. The solution was stirred for 1.5 hours, then it was concentrated *in vacuo.* The residue was dissolved in AcOEt/H₂O (10 mU2 ml) and washed with saturated potassium carbonate solution. The organic layer was dried, concentrated *in vacuo* and the residue was purified by silica cartridge (from DCM to DCM/MeOH 80:20) to give the title compound (32 mg) as a white solid.
T.I.c.: DCM/MeOH 8:2, Rf=0.40 (detection with ninhydrine).
MS (ES/+): m/z=573 [M+H]⁺.

### Example 41

### N-{(1R)-1-[3,5-bis(trifluoromethyl)phenyl]ethyl}-3-(2,4-dichlorophenyl)-3-(4-fluoro-4-piperidinyl)-N-methylpropionamide hydrochloride-(diastereoisomer 1)

Hydrochloric acid (1M in Et2O - 80 µL) was added to a solution of example 40 (32 mg) in anhydrous Et2O (2 mL) previously cooled to -10°C under a Nitrogen atmosphere. The mixture was stirred at -10°C for 10 minutes, then it was concentrated *in vacuo.* The residue was triturated with pentane (3 x 2 mL) to give the title compound (27 mg) as white solid.
NMR (d₆-DMSO): δ (ppm) 8.50 (sb, 2H); 8.00 (s, 1H); 7.80 (s, 2H); 7.58 (s, 1H); 7.45 (dd, 2H); 5.65 (q, 1H); 4.03 (m, 1H); 3.23-2.87 (m, 6H); 2.82 (s, 3H); 2.17 (m, 1H);1.60 (m, 1 H); 1.94-1.78 (m, 2H); 1.37 (d, 3H).
MS (ES/+): m/z=573 [MH-HCl]⁺.

### Example 42

### N-{(1R)-1-[3,5-bis(trifluoromethyl)phenynethyl)-3-(2,4-dichlorophenyl)-3-(4-nuoro-4-piperidinyl)-N-methylpropionamide-(diastereoisomer 2)

(TFA (1 mL) was added to a solution of intermediate 45b (57 mg) in anhydrous DCM (3 mL) previously cooled to -10°C under a Nitrogen atmosphere. The solution was stirred for 1.5 hours, then it was concentrated *in vacuo.* The residue was dissolved in AcOEt/H₂O (10 mL/2 ml) and washed with saturated potassium carbonate solution. The organic layer was dried, concentrated *in vacuo* and the residue was purified by silica cartridge (from DCM to DCM/MeOH 80:20) to give the title compound (28 mg) as a white solid.
T.l.c.: DCM/MeOH 8:2, Rf=0.39 (detection with ninhydrine).
MS (ES/+): m/z=573 [M+H]⁺.

### Example 43

### N-{(1R)-1-(3,5-bis(trifluoromethyl)phenyl]ethyl}-3-(2,4-dichlorophenyl)-3-(4-fluoro-4-piperidinyl)-N-methylpropionamide hydrochloride-(diastereoisomer 2)

Hydrochloric acid (1M in Et2O - 80 µL) was added to a solution of example 42 (32 mg) in anhydrous Et2O (2 mL) previously cooled to -10°C under a Nitrogen atmosphere. The mixture was stirred at -10°C for 10 minutes, then it was concentrated *in vacuo.* The residue was triturated with pentane (3 x 2 mL) to give the title compound (27 mg) as white solid.
NMR (d₆-DMSO): δ (ppm) 8.44 (sb, 2H); 7.95 (s, 1H); 7.57 (s, 2H); 7.57 (s, 1H); 7.51 (d, 1H); 7.31 (d, 1 H); 5.73 (q, 1H): 4.02 (m, 1 H); 3.23-2.82 (m, 6H); 2.78 (s, 3H); 2.18/1.60 (m, 2H); 1.92-1.78 (m, 2H); 1.47 (d, 3H).
MS (ES/+): m/z=573 [MH-HCl]⁺.

### Example 44

### N-{(1R)-1-[3,5-bis(trifluoromethyl)phenyl]ethyl}-3-(4-fluoro-2-methylphenyl)-3-(4-fluoro-4-piperidinyl)-N-methylpropionamide-(diastereoisomer 1)

TFA (0.44 mL) was added to a solution of intermediate 49a (23 mg) in anhydrous DCM (2 mL) previously cooled at 0°C, under a Nitrogen atmosphere. The solution was stirred at 0°C for 1 hour, then it was treated at 0°C with a 10% sodium hydroxide solution until pH=10. The aqueous layer was extracted with DCM (3 x 8 mL). The combined organic extracts were dried, concentrated *in vacuo* and the residue was purified by flash chromatography (DCM/MeOH from 100:0 to 75:25) to give the title compound as a white foam (16 mg).
T.I.c.: DCM/MeOH 8:2, Rf=0.41 (detection with ninhydrine).
MS (ES/+): m/z=537 [M+H]⁺.

### Example 45

### N-{(1R)-1-[3,5-bis(trifluoromethyl)phenyl]ethyl}-3-(4-fluoro-2-methylphenyl)-3-(4-fluoro-4-piperidinyl)-N-methylpropionamide hydrochloride-(diastereoisomer 1)

The compound of Example **44** (16 mg) was dissolved in dry Et₂O (3 mL), cooled to 0°C and treated with hydrochloric acid (1 M in Et₂O - 32 µL). The mixture was stirred at 0°C for 1 hour, then Et2O was evaporated under Nitrogen flux and the residue was triturated in pentane (3 x 4 mL) and filtered to give the title compound (17 mg) as a white solid.
NMR (d₆-DMSO): δ (ppm) 8.72 (b, 1H); 8.41 (b, 1H); 8.00 (s, 1H); 7.79 (s, 2H); 7.34 (t, 1 H); 7.01 (d, 1H); 7.00 (t, 1H); 5,65 (q, 1 H); 3.72 (m, 1 H); 3.23 (bd, 1 H); 3.15 (bd, 1H); 2.94 (m, 2H); 2.84 (m, 2H); 2.76 (s, 3H); 2.35 (s, 3H); 2.16 (b, 1 H); 1.81 (m, 2H); 1.62 (bd, 1H); 1.30 (d, 3H).
MS (ES/+): m/z=537 [MH-HCl]⁺.

### Example 46

### N-{(1R)-1-[3,5-bis(trifluoromethyl)phenyl]ethyl}-3-(4-fluoro-2-methylphenyl)-3-(4-fluoro-4-piperidinyl)-N-methylpropionamide-(diastereoisomer 2)

TFA (0.66 mL) was added to a solution of intermediate 49b (34 mg) in anhydrous DCM (2.5 mL) previously cooled at 0°C, under a Nitrogen atmosphere. The solution was stirred at 0°C for 1 hour, then it was treated at 0°C with a 10% sodium hydroxide solution until pH=10. The aqueous layer was extracted with DCM (3 x 8 mL). The combined organic extracts were dried, concentrated *in vacuo* and the residue was purified by flash chromatography (DCM/MeOH from 100:0 to 75:25) to give the title compound as a colourless oii (22 mg).
T.I.c.: DCM/MeOH 9:1, containing 1.5% of conc. NH₄OH, Rf=0.43 (detection with ninhydrine).
MS (ES/+): m/z=537 [M+H]⁺.

### Example 47

### N-{(1R)-1-[3,5-bis(trifluoromethyl)phenyl]ethyl}-3-(4-fluoro-2-methylphenyl)-3-(4-fluoro-4-piperidinyl)-N-methylpropionamide hydrochloride-(diastereoisomer 2)

The compound of Example **46** (22 mg) was dissolved in dry Et₂O (3 mL), cooled to 0°C and treated with hydrochloric acid (1 M in Et₂O - 32 µL). The mixture was stirred at 0°C for 1 hour, then Et₂O was evaporated under Nitrogen flux and the residue was triturated in pentane (3 x 4 mL) and filtered to give the title compound (28 mg) as a white solid.
NMR (d₆-DMSO): δ (ppm) 8.48 (b, 2H); 7.93 (s, 1H); 7.49 (s, 2H); 7.33 (t, 1 H); 6.93 (d, 1H); 6.89 (t, 1 H); 5,73 (q, 1H); 3.71 (m, 1H); 3.24 (bd, 1H); 3.15 (bd, 1H); 2.93-2.91 (m, 2H); 2.84-2.81 (m, 2H); 2.72 (s, 3H); 2.35 (s, 3H); 2.16 (b, 1H); 1.82 (m, 2H); 1.64 (bd, 1 H); 1.45 (d, 3H).
MS (ES/+): m/z=537 [MH-HCl]⁺.

### Example 48

### N-[(3,5-dibromophenyl)methyl]-3-(4-fluoro-2-methylphenyl)-3-(4-fluoro-4-piperidinyl)-N-methylpropionamide

TFA (0.72 mL) was added to a solution of intermediate 50 (38 mg) in anhydrous DCM (3 mL) previously cooled at 0°C, under a Nitrogen atmosphere. The solution was stirred at 0°C for 1 hour, then it was treated at 0°C with a 10% sodium hydroxide solution until pH=10. The aqueous layer was extracted with DCM (2 x 10 mL). The combined organic extracts were dried, concentrated *in vacuo* and the residue was purified by flash chromatography (DCM/MeOH from 100:0 to 75:25) to give the title compound as a colourless oil (25 mg).
T.I.c.: DCM/MeOH 8:2, Rf=0.32 (detection with ninhydrine).
MS (ES/+): m/z=545 [M+H]⁺.

### Example 49

### N-[(3,5-dibromophenyl)methyl]-3-(4-fluoro-2-methylpheny)-3-(4-fluoro-4-piperidinyl)-N-methylpropionamide hydrochloride

The compound of Example **48** (25 mg) was dissolved in dry Et2O (3 mL) and treated at 0°C with hydrochloric acid (1M in Et₂O - 50 µL).
The mixture was stirred at 0°C for 1 hour, then Et2O was evaporated under Nitrogen flux and the residue was triturated in pentane (3 x 4 mL) and filtered to give the title compound (33 mg) as a white solid.
NMR (d₆-OMSO): δ (ppm) 8.57 (b, 2H); 7.66 (s, 1H); 7.35 (t, 1H); 7.14 (s, 2H); 7.01 (d, 1H); 6.98 (t, 1 H); 4.51 (d, 1H); 4.21 (d, 1H); 3.70 (dm, 1 H); 3.25 (bd, 1 H); 3.16 (bd, 1 H); 2.93-2.84 (m, 4H); 2.67 (s, 3H); 2.36 (s, 3H); 2.18 (b, 1H); 1.82 (m, 2H); 1.61 (bd, 1 H).
MS (ES/+): m/z=545 [MH-HCl]⁺.

### Example 50

### N-[(3,5-dibromophenyl)methyl)-3-(4-fluoro-2-methylphenyl)-3-(4-fluoro-4-piperidinyl)-N-methylpropionamide hydrochloride (50a) (enantiomer 1) and N-[(3,5-d)bromophenyl)methyl]-3-(4-fluoro-2-methylphenyl)-3-(4-fluoro-4-piperidinyl)-N-methylpropionamide hydrochloride (50b) (enantiomer 2)

The compound of example 49 (22 mg) was suspended in AcOEt (10ml) and washed with a 0.5M sodium hydroxide solution (10 mL); the organic phase was dried, concentrated *in vacuo* to give a colourless oil which was separated into the enantiomers via HPLC (column: Chiracel OD 25cm x 20mm; mobile phase: n-hexane/2-Propanol 75:25; flow rate=6 mL/min; λ=225 nm).
Thus, example 50a (6 mg) and example 50b (8.5 mg) were obtained.

### Example 50a

Chiral HPLC : Column Chiracel OD 25cm x 4.6mm; mobile phase: n-hexane/2-Propanol 80:20; flow rate=1 mL/min; λ=225 nm, retention time 12,4 minutes, ratio 50a/50b=100/0
MS (ES/+): m/z=545 [M+H]⁺.

### Example 50b

Chiral HPLC : Column Chiracel OD 25cm x 4.6mm; mobile phase: n-hexane/2-Propanol 80:20; flow rate=1 mL/min; λ=225 nm, retention time 15,4 minutes, ratio 50a/50b=0/100
MS (ES/+): m/z=545 [M+H]⁺.

### Example 51

### N-[(3,5-dibromophenyl)methyl]-3-(4-fluoro-2-methylphenyl)-3-(4-fluoro-4-piperidinyl)-N-methylpropionamide hydrochloride (enantiomer 1)

The compound of Example 50a (6 mg) was dissolved in dry Et2O (2 mL) and treated at 0°C with hydrochloric acid (1M in Et₂O-12 µL).
The mixture was stirred at 0°C for 15 minutes, then Et2O was evaporated under Nitrogen flux and the residue was triturated in pentane (3 x 2 mL) to give the title compound (6 mg) as a white solid.
NMR (d₆-DMSO): δ (ppm) 8.57 (b, 2H); 7.66 (s, 1 H); 7.35 (t, 1H); 7.14 (s, 2H); 7.01 (d, 1H); 6.98 (t, 1 H); 4.51 (d, 1H); 4.21 (d, 1H): 3.70 (m, 1H); 3.25 (bd, 1 H); 3.16 (bd, 1H); 2.93-2.84 (m, 4H); 2.67 (s, 3H); 2.36 (s, 3H); 2.18 (b, 1H); 1.82 (m, 2H); 1.61 (bd, 1H).
MS (ES/+): m/z=545 [MH-HCl]⁺.

### Example 52

### N-[(3,5-dibromophenyl)methyl]-3-(4-fluoro-2-methylphenyl)-3-(4-fluoro-4-piperidinyl)-N-methylpropionamide hydrochloride (enantiomer 2)

(The compound of Example 50b (8.5 mg) was dissolved in dry Et2O (2 mL) and treated at 0°C with hydrochloric acid (1M in Et₂O - 17 µL).
The mixture was stirred at 0°C for 15 minutes, then Et2O was evaporated under Nitrogen flux and the residue was triturated in pentane (3 x 2 mL) to give the title compound (6.5 mg) as a white solid.
NMR (d₆-DMSO): δ (ppm) 8.57 (b, 2H); 7.66 (s, 1H); 7.35 (t, 1H); 7.14 (s, 2H); 7.01 (d, 1 H); 6.98 (t, 1H); 4.51 (d, 1H); 4.21 (d, 1 H); 3.70 (m, 1 H); 3.25 (bd, 1 H); 3.16 (bd, 1H); 2.93-2.84 (m, 4H); 2.67 (s, 3H); 2.36 (s, 3H); 2.18 (b, 1H); 1.82 (m, 2H); 1.61 (bd, 1H).
MS (ES/+): m/z=545 [MH-HCl]⁺.

### Example 53

### N-[(3,5-dibromophenyl)methyl]-3-(3,4-dichlorophenyl)-3-(4-fluoro-4-piperidinyl)-N-methylpropionamide (53a) (enantiomer 1)

### N-[(3,5-dibromophenyl)methyl]-3-(3,4-dichlorophenyl)-3-(4-fluoro-4-piperldinyl)-N-methylproplonamide (53b) (enantiomer 2)

TFA (2.5 mL) was added to a solution of intermediate 56 (135 mg) in anhydrous DCM (10 mL) previously cooled at 0°C, under a Nitrogen atmosphere. The solution was stirred at 0°C for 1 hour, then it was treated at 0°C with a 10% sodium hydroxide solution until pH=10. The aqueous layer was extracted with DCM (2 x 10 mL). The combined organic extracts were dried, concentrated *in vacuo* to give a racemate.
The mixture was separated into the enantiomers via HPLC (column: Chiracel OD 25cmx 20mm; mobile phase: n-Hexane/2-Propanol 65:35; flow rate=6 mL/min; λ=225 nm). Thus, example **53a** (37 mg) and example **53b** (38 mg) were obtained as white foams.

### Example 53a

Chiral HPLC: Column Chiralcel 25cmx 4.6 mm; mobile phase: n-Hexane/2-Propanol 70:30; flow rate=1 mL/min; λ=225 nm, retention time 10.7 minutes; ratio 53a/53b=100/0

### Example 53b

Chiral HPLC: Column Chiralcel OD 25cmx 4.6 mm; mobile phase: n-Hexane/2-Propanol 70:30; flow rate=1 mL/min; λ=225 nm, retention time 13.7 minutes; ratio 53a/53b=0/100
NMR (d₆-DMSO): δ (ppm) 7.68 (m, 1H); 7.55 (m, 2H); 7.5-7.1 (m, 3H); 4.51-4.27 (dd, 2H); 3.5-2.5 (m, 7H); 3.00 (s, 3H); 2.3-1.9 (bs, 1 H); 1.9-1.3 (m, 4H).
MS (ES/+): m/z=581 [M+H]⁺.

### Example 54

### N-{[3,5-bis(trifluoromethyl)phenyl]methyl}-3-(4-fluorophenyl)-3-(4-fluoro-4 piperidinyl)-N-methylpropionamide(54a) (enantiomer 1) and N-{[3,5-bis(trifluoromethyl)phenyl]methyl}-3-(4-fluorophenyl)-3-(4-fluoro-4-piperidinyl)-N-methylpropionamide(54b) (enantiomer 2)

TFA (4 mL) was added to a solution of intermediate 57 (440 mg) in anhydrous DCM (16 mL) previously cooled to -10°C under a Nitrogen atmosphere. The resulting solution was stirred at 0°C for 18 hours, then the solution was concentrated *in vacuo.* The residue was diluted with AcOEt (20 mL) and washed with a saturated potassium carbonate solution (3 x 20 mL) and brine (20 mL). The organic phase was dried and concentrated *in vacuo* to give a yellow oil which was submitted to HPLC separation (Column: Chiralcel OD 25cm x 20mm; mobile phase: n-hexane/i-PrOH 75:25; flow rate=7 mL/min; λ=225 nm). Thus, the title compounds example 54a (134 mg) and example 54b (131 mg) were obtained as a colourless oils.

### Example 54a:

Chiral HPLC: Column Chiralcel OD 25cm x 4.6mm x 5µm, mobile phase n-hexane/i-PrOH 80:20, flow rate=1 mUmin, λ=225 nm, retention time: 12.6 minutes, ratio **54a/54b**=100/0.
MS (ES/+): m/z=509 [MH]⁺.

### Example 54b:

Chiral HPLC: Column Chiralcel OD 25cm x 4.6mm x 5µm, mobile phase n-hexane/i-PrOH 80:20, flow rate=1 mUmin, λ=225 nm, retention time: 15.9 minutes, ratio **54a/54b**=0/100
MS (ES/+): m/z=509 [MH]⁺.

### Example 55

### N-{[3,5-bis(trifluoromethyl)phenyl]methyl}-3-(4-fluorophenyl)-3-(4-fluoro-4-piperidinyl)-N-methylpropionamide hydrochloride (enantiomer 1)

The compound of example 54a (134 mg) was dissolved in Et2O (1 mL), previously cooled to -10°C under a Nitrogen atmosphere, and treated with hydrochloric acid (1M in Et2O - 309 µL). The mixture was stirred at 0°C for 10 minutes, then the solvent was decanted and the residue was triturated with pentane (2 x 1 mL) to give the title compound (114.7 mg) as a white solid.
NMR (d₆-DMSO): δ (ppm) 8.65 (b, 2H) 7.90 (s, 1H); 7.64 (s, 2H); 7.29 (dd, 2H); 7.03 (t, 2H); 4.65 (d, 1H); 4.39 (d, 1H), 3.47 (m, 1H), 3.14 (bt, 2H), 3.01 (dd, 1H), 2.97 (s, 3H), 2.95-2.75 (m, 2H), 2.83 (dd, 1H), 2.05 (m, 1H), 1.8-1.55 (m, 3H).
MS (ES/+): m/z=509 [MH-HCl]⁺.

### Example 56

### N-{[3,5-bis(trifluoromethyl)phenyl]methyl]-3-(4-fluorophenyl)-3-(4-fluoro-4-piperidinyl)-N-methylpropionamide hydrochloride (enantiomer 2)

The compound of example 54b (131 mg) was dissolved in Et20 (1 mL), previously cooled to -10°C under a Nitrogen atmosphere, and treated with hydrochloric acid (1M in Et2O-308 µL). The mixture was stirred at 0°C for 10 minutes, then the solvent was decanted and the residue was triturated with pentane (2 x 1mL) to give the title compound (118.5 mg) as a white solid.
NMR (d₆-DMSO): δ (ppm) 8.45 (b, 2H) 7.90 (s, 1H); 7.64 (s, 2H); 7.29 (dd, 2H); 7.03 (t, 2H); 4.65 (d, 1H); 4.39 (d, 1H), 3.47 (m, 1H), 3.14 (bt, 2H), 3.01 (dd, 1H), 2.97 (s, 3H), 2.95-2.75 (m, 2H), 2.83 (dd, 1H), 2.05 (m, 1H), 1.8-1.55 (m, 3H).
MS (ES/+): m/z=509 [MH-HCl]⁺.

### Example 57

**3-(4-chlorophenyl)-*N*-[(3,5-dibromophenyl)methyl]-3-(4-fluoro-4-piperidinyl)-*N-*methylpropionamide (57a) (enantiomer 1) and 3-(4-chlorophenyl)-*N*-[(3,5-dibromophenyl)methyl]-3-(4-fluoro-4-piperidinyl]-*N-*methylpropionamide (57b) (enantiomer 2)** TFA (1 mL) was added to a solution of intermediate 64 (115 mg) in anhydrous DCM (4 mL) previously cooled to -10°C under a Nitrogen atmosphere. The resulting solution was stirred at 0°C for 18 hours, then the solution was concentrated *in vacuo.* The residue was diluted with AcOEt (20 mL) and washed with a saturated potassium carbonate solution (3 x 20 mL) and brine (20 mL). The organic phase was dried and concentrated *in vacuo* to give a colourless oil which was submitted to HPLC separation (Column: Chiralcel OD 25cm x 20mm; mobile phase: n-hexane/ethanol 70:30; flow rate=6.5 mL/min: λ=225 nm). Thus, the title compounds example 57a (41 mg) and example 57b (40 mg) were obtained as a colourless oils.

### Example 57a:

Chiral HPLC: Column Chiralcel OD 25cm x 4.6mm x 5µm, mobile phase n-hexane/ehanol 70:30, flow rate=1 mL/min, λ=225 nm, retention time: 6.69 minutes, ratio 57a/57b=100/0.
MS (ES/+): m/z=547 [MH]⁺.

### Example 57b:

Chiral HPLC: Column Chiralcel OD 25cm x 4.6mm x 5µm, mobile phase n-hexane/ehanol 70:30, flow rate=1 mL/min. λ=225 nm, retention time: 10.85 minutes, ratio **57a/57b**=0/100
MS (ES/+): m/z=547 [MH]⁺.

### Example 58

**3-(4-chlorophenyl)-*N*-[(3,5-dibromophenyl)methyl]-3-(4-fluoro-4-piperidinyl)-*N-*methylproplonamide hydrochloride (58) (enantiomer 1) The compound of example** 57a (41 mg) was dissolved in Et2O (1 mL), previously cooled to 0°C under a Nitrogen atmosphere, and treated with hydrochloric acid (1M in Et2O - 83 µL). The mixture was stirred at 0°C for 10 minutes, then the solvent was decanted and the residue was triturated with pentane (2 x 1 mL) to give the title compound (37 mg) as a white solid.
NMR (d₆-DMSO, 60°C): δ (ppm) 8.8-8.3 (b, 2H) 7.7 (m. 3H); 7.1 (m, 4H); 4.7-4.1 (dd, 2H); 3.5 (m, 1H); 3.3-2.8 (m, 4H); 2.9 (m, 2H), 2.9 (s, 3H), 2.0-1.5 (m, 4H).

### Example 59

**3-(4-chlorophenyl)-*N*-[(3,5-dibromophenyl)methyl]-3-(4-fluoro-4-piperidinyl)-*N-*methylpropionamide hydrochloride (59) (enantiomer 2**) The compound of example 57b (40 mg) was dissolved in Et2O (1 mL), previously cooled to -10°C under a Nitrogen atmosphere, and treated with hydrochloric acid (1M in Et2O - 80 µL). The mixture was stirred at 0°C for 10 minutes, then the solvent was decanted and the residue was triturated with pentane (2 × 1 mL) to give the title compound (40 mg) as a white solid.
NMR (d₆-DMSO, 60°C): δ (ppm) 8.8-8.3 (b, 2H) 7.7 (m, 3H); 7.1 (m, 4H); 4.7-4.1 (dd, 2H); 3.5 (m, 1H); 3.3-2.8 (m, 4H); 2.9 (m, 2H), 2.9 (s, 3H), 2.0-1.5 (m, 4H).

### Example 60

### N-{[3,5-bis(trifluoromethyl}phenyl]methyl}-3-(4-fluorophenyl)-N-methyl-3-(3-piperidinylidene)propionamide (isomer 1)

TFA (0.121 mL) was added to a solution of intermediate 73a (6 mg) in anhydrous DCM (0.6 mL) previously cooled to 0°C under a Nitrogen atmosphere. The resulting solution was stirred at 0°C for 1 hour, then the solution was concentrated *in vacuo.* The residue was diluted with DCM and washed with a saturated potassium carbonate solution. The aqueous layer was extracted with further DCM (2 x 2 mL). The combined organic extracts were dried and concentrated *in vacuo* to a residue which was purified by flash chromatography (DCM/MeOH from 95:5 to 9:1) to give the title compound (3 mg) as a white foam.
MS (ES/+): m/z=489 [M+H]⁺.

### Example 61

### N-{[3,5-bis(trifluoromethyl)phenyl]methyl}-3-(4-fluorophenyl)-N-methyl-3-(3-piperidinylidene)propionamide (isomer 2)

TFA (0.243 mL) was added to a solution of intermediate 73b (12 mg) in anhydrous DCM (1.2 mL) previously cooled to 0°C under a Nitrogen atmosphere. The resulting solution was stirred at 0°C for 1 hour, then the solution was concentrated *in vacuo*. The residue was diluted with DCM and washed with a saturated potassium carbonate solution. The aqueous layer was extracted with further DCM (2 x 2 mL). The combined organic extracts were dried and concentrated *in vacuo* to a residue which was purified by flash chromatography (DCM/MeOH from 95:5 to 9:1) to give the title compound (4.8 mg) as a white foam.
MS (ES/+): m/z=489 [M+H]⁺.

### Example 62

### (3E)-N-{[3,5-bis(trifluoromethyl)phenyl]methyl}-3-(4-fluorophenyl)-N-methyl-3-(3-pyrrolidinylidene)propionamide

TFA (1.0 mL) was added to a solution of intermediate 81 a (23 mg) in anhydrous DCM (4 mL) previously cooled at 0°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried, concentrated *in vacuo* and the residue was purified by flash chromatography (DCM/MeOH from 9/1 to 7/3) to give the title compound (0.015 g) as a white foam.
T.I.c.: DCM/MeOH 8:2, Rf=0.2.
MS (ES/+): m/z=475 [M+H]⁺.
NMR (d₆-DMSO): δ (ppm) 7.98 (s, 1H); 7.76 (s, 2H); 7.26 (dd, 2H); 7.08 (t, 2H); 4.58 (s, 2H); 3.75 (s, 2H); 3.07 (t, 2H); 2.96 (s, 3H); 2.56 (s, 2H); 2.40 (t, 2H).

### Example 63

### (3Z)-N-{[3,5-bis(trifluoromethyl)phenyl]methyl)-3-14-fluorophenyl)-N-methyl-3-(3-pyrrolidinylidene)propionamide

TFA (1.0 mL) was added to a solution of intermediate 81b (25 mg) in anhydrous DCM (4 mL) previously cooled at 0°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried, concentrated *in vacuo* and the residue was purified by flash chromatography (DCM/MeOH from 9/1 to 7/3) to give the title compound (0.013 g) as a white foam.
T.I.c.: DCM/MeOH 8:2, Rf=0.22.
MS (ES/+): m/z=475 [M+H]⁺.
NMR (d₆-DMSO): δ (ppm) 7.98 (s, 1 H); 7.77 (s, 2H); 7.24 (dd, 2H); 7.10 (t, 2H); 4.6 (s, 2H); 3.61 (bs, 4H); 3.22 (t, 2H); 2.99 (s, 3H); 2.60 (t, 2H).

### Example 64

### N-{(1R)-1-[3.5-bis(trifluoromethyl)phenyl]ethyl}-3-(4-fluorophenyl)-N-methyl-3-(4-piperidinylidene)propionamide

A solution of TFA (1 mL) in anhydrous DCM (2 mL) was added to a solution of intermediate 84 (40 mg) in anhydrous DCM (2 mL) previously cooled to -10°C under a Nitrogen atmosphere. The resulting solution was left in freezer overnight, then the solution was concentrated *in vacuo.* The residue was diluted with AcOEt/water and washed with a saturated potassium carbonate solution. The aqueous layer was extracted with further AcOEt. The combined organic extracts were dried and concentrated *in vacuo* to a residue which was purified by silica cartridge (from DCM/MeOH 95:5 to 7:3) to give the title compound (15 mg) as a white foam.
MS (ES/+): m/z=503 [M+H]⁺.

### Example 65

### N-{(1R)-1-[3,5-bis(trifluoromethyl)phenyl]ethyl}-3-(4-fluorophenyl)-N-methyl-3-(4-piperidinylidene)propionamide hydrochloride

Hydrochloric acid (1 M in Et₂O - 50 µL) was added to a solution of example 64 (12 mg) in anhydrous Et2O (0.3 mL) previously cooled to -10°C under a Nitrogen atmosphere. The mixture was stirred at -10°C for 15 minutes, then it was concentrated *in vacuo.* The residue was triturated with pentane (3 x 1 mL) to give the title compound (12 mg) as white solid.
MS (ES/+): m/z=503 [MH-HCl]⁺.
NMR (d₆-OMSO): δ (ppm) 7.75 (s, 1 H); 7.65 (s, 2H); 7.05 (dd, 2H); 6.95 (dd, 2H); 6.00 (q, 1 H); 3.40 (d, 2H); 2.95 (t. 2H); 2.80 (t, 2H); 2.50 (s, 3H); 2.35 (t, 2H); 2.05 (t, 2H); 1.40 (d, 3H).

### Example 66

### N-{(1S)-1-[3.5-bis(trifluoromethyl)phenyl]ethyl}-3-(4-fluorophenyl)-N-methyl-3-(4-piperidinylidene)propionamide

A solution of TFA (1 mL) in anhydrous DCM (2 mL) was added to a solution of intermediate 85 (40 mg) in anhydrous DCM (2 mL) previously cooled to -10°C under a Nitrogen atmosphere. The resulting solution was left in freezer overnight, then the solution was concentrated *in vacuo.* The residue was diluted with AcOEt/water and washed with a saturated potassium carbonate solution. The aqueous layer was extracted with further AcOEt. The combined organic extracts were dried and concentrated *in vacuo* to a residue which was purified by silica cartridge (from DCM/MeOH 95:5 to 7:3) to give the title compound (36 mg) as a white foam.
MS (ES/+): m/z=503 [M+H]⁺.
NMR (d₆-DMSO): δ (ppm) 7.75 (s, 1H); 7.65 (s, 2H); 7.05 (dd, 2H); 6.95 (dd, 2H); 6.00 (q, 1 H); 3.40 (d, 2H); 2.95 (t, 2H); 2.80 (t, 2H); 2.50 (s, 3H); 2.35 (t, 2H); 2.05 (m, 2H); 1.40 (d, 3H).

### Example 67

### N-{(1S)-1-[3.5-bis trifluoromethyl)phenyl]ethyl}-3-(4-fluorophenyl)-N-methyl-3-(4-piperldinylidene)propionamide hydrochloride

Hydrochloric acid (1M in Et20 - 50 µL) was added to a solution of example 66 (15 mg) in anhydrous Et20 (0.2 mL) previously cooled to -10°C under a Nitrogen atmosphere. The mixture was stirred at -10°C for 15 minutes, then it was concentrated *in vacuo.* The residue was triturated with pentane (3 x 1 mL) to give the title compound (13 mg) as white solid.
MS (ES/+): m/z=503 [MH-HCl]⁺.

### Example 68

### N-[(3,5-dibromophenyl)methyl]-3-(4-fluorophenyl)-N-methyl-3-(4-piperidinylidene)propionamide

A solution of TFA (1 mL) in anhydrous DCM (2 mL) was added to a solution of intermediate 86 (42 mg) in anhydrous DCM (3 mL) previously cooled to -10°C under a Nitrogen atmosphere. The resulting solution was stirres at 0°C overnight, then the solution was concentrated *in vacuo.* The residue was diluted with AcOEt/water and washed with a saturated potassium carbonate solution. The aqueous layer was extracted with further AcOEt. The combined organic extracts were dried and concentrated *in vacuo* to a residue which was purified by silica cartridge (DCM, DCM/MeOH from 95:5 to 7:3) to give the title compound (16.2 mg) as a colourless oil.
T.I.c.:CH/AcOEt 1:1, Rf=0.6 (detection with ninhydrine)
MS (ES/+): m/z=511 [M+H]⁺.

### Example 69

### N-[(3,5-dibromophenyl)methyl]-3-(4-fluorophenyl)-N-methyl-3-(4-piperidinylidene)propionamide hydrochloride

Hydrochloric acid (1M in Et2O - 40 µL) was added to a solution of example 68 (16 mg) in anhydrous Et2O (0.5 mL) previously cooled to 0°C under a Nitrogen atmosphere. The mixture was stirred at 0°C for 15 minutes, then it was concentrated *in vacuo.* The residue was triturated with pentane (3 x 1 mL) to give the title compound (13.9 mg) as whitish solid.
NMR (CDCl3): δ (ppm) 9.72 (b, 2H); 7.58 (s, 1H); 7.21 (s, 1H); 7.04 (m, 1H); 7.04 (m, 4H); 4.46 (s, 2H); 3.36 (b, 4H); 3.15 (s, 2H); 2.79 (s, 3H); 2.8 (d, 2H); 2.49 (d, 2H).
MS (ES/+): m/z=509 [MH-HCl]⁺.

### Example 70

### N-{[3,5-bis(trifluoromethyl)phenyl]methyl}-3-(4-fluoro-2-methylphenyl)-N-methyl-3 (1,2,3,6-tetrahydro-4-pyridinyl)propionamide

TFA (1 mL) was diluted in DCM (3 ml) and added to a solution of intermediate 90 (44 mg) in anhydrous DCM (1 mL) previously cooled to -10°C under a Nitrogen atmosphere. The solution was left in freezer overnight, then it was concentrated *in vacuo.* The residue was dissolved in AcOEt/H2O (10 mU2 ml) and washed with saturated potassium carbonate solution (10 mL). The organic layer was dried, concentrated *in vacuo* and the residue was purified by silica cartridge (from DCM to DCM/MeOH 80:20) to give the title compound (25.5 mg) as a white solid.
T.I.c.: DCM/MeOH 8:2, Rf=0.42 (detection with ninhydrine).
MS (ES/+): m/z=503 [M+H]⁺.

### Example 71

### N-{(1R)-1-[3,5-bis(trifluoromethyl)phenyl]ethyl}-3-(4-fluoro-2-methylphenyl)-N-methyl-3-(1,2,3,6-tetrahydro-4-pyridinyl)propionamide (diastereoisomer 1)

TFA (1 mL) was diluted in DCM (2 ml) and added to a solution of intermediate 91a (25.5 mg) in anhydrous DCM (2 mL) previously cooled to -10°C under a Nitrogen atmosphere. The solution was left in freezer overnight, then it was concentrated *in vacuo.* The residue was dissolved in AcOEt/H₂O (10 mU2 ml) and washed with saturated potassium carbonate solution (10 mL). The organic layer was dried, concentrated *in vacuo* and the residue was purified by silica cartridge (DCM, DCM/MeOH from 95:5 to 80:20) to give the title compound (15 mg) as a white solid.
T.I.c.: DCM/MeOH 9:1 containing 1% NH40H, Rf=0.58 (detection with ninhydrine).
MS (ES/+): m/z=517[M+H]⁺.

### Example 72

### N-{(1R)-1-[3,5-bis(trifluoromethyl)phenyl]ethyl}-3-(4-fluoro-2-methylphenyl}-N-methyl-3-(1,2,3,6-tetrahydro-4-pyridinyl)propionamide hydrochloride (diastereoisomer 1)

Hydrochloric acid (1M in Et2O- 50 µL) was added to a solution of example 71 (15 mg) in anhydrous Et2O (0.2 mL) previously cooled to -10°C under a Nitrogen atmosphere. The mixture was stirred at -10°C for 15 minutes, then it was concentrated *in vacuo.* The residue was triturated with pentane (3 x 1 mL) to give the title compound (8.1 mg) as white solid.
NMR (d₆-DMSO): δ (ppm) 8.62 (b, 2H); 7.97 (s, 1 H); 7.76 (s, 2H); 7.17 (dd, 1H); 6.95 (td, 1H); 6.69 (dd, 1 H); 5.71 (q, 1H); 5.45 (bs, 1 H); 3.99 (t, 1H); 3.46 (bs, 2H); 3.05/3.01 (bm, 2H); 2.83 (dd, 1H); 2.72 (dd, 1H), 2.71 (s, 3H). 2.28 (s, 3H). 2.02 (bm, 2H), 1.36 (d, 3H).

### Example 73

### N-{(1R)-1-[3,5-bis(trifluoromethyl)phenyl]ethyl-3-(4-fluoro-2-methylphenyl)-N-methyl-3-(1,2,3,6-tetrahydro-4-pyridinyl)propionamide (diastereoisomer 2)

TFA (1 mL) was diluted in DCM (2 ml) and added to a solution of intermediate 91b (25.5 mg) in anhydrous DCM (2 mL) previously cooled to -10°C under a Nitrogen atmosphere. The solution was left in freezer overnight, then it was concentrated *in vacuo.* The residue was dissolved in AcOEt/H2O (10 mU2 ml) and washed with saturated potassium carbonate solution (10 mL). The organic layer was dried, concentrated *in vacuo* and the residue was purified by silica cartridge (DCM, DCM/MeOH from 95:5 to 80:20) to give the title compound (10 mg) as a white solid.
T.I.c.: DCM/MeOH 9:1 containing 1% NH4OH, Rf=0.48 (detection with ninhydrine).
MS (ES/+): m/z=517 [M+H]⁺.

### Example 74

### N-{(1R)-1-[3,5-bis(trifluoromethyl)phenyl]ethyl}-3-(4-fluoro-2-methylphenyl)-N-methyl-3-(1,2,3,6-tetrahydro-4-pyridinyl)propionamide hydrochloride (diastereoisomer 2)

Hydrochloric acid (1M in Et2O- 50 µL) was added to a solution of example 73 (10 mg) in anhydrous Et2O (0.2 mL) previously cooled to -10°C under a Nitrogen atmosphere. The mixture was stirred at -10°C for 15 minutes, then it was concentrated *in vacuo.* The residue was triturated with pentane (4 x 1 mL) to give the title compound (10 mg) as white solid.
NMR (d₆-DMSO): δ (ppm) 8.81 (b, 2H); 7.93 (s, 1H); 7.60 (s, 2H); 7.16 (dd, 1H); 6.88 (dd, 1H); 6.82 (td, 1H); 5.74 (q, 1 H); 5.42 (bs, 1 H); 3.99 (t, 1 H); 3.48 (bs, 2H); 3.01 (m, 2H); 3.01 (dd, 1H); 2.69 (dd, 1H). 2.71 (s, 3H). 2.25 (s, 3H). 2.05 (bm, 2H). 1.43 (d, 3H).

### Example 75

### N-{[3,5-bis(trifluoromethyl)phenyl]methyl}-3-(4-fluorophenyl)-N-methyl-3-(3-pyrrolidinyl)propionamide (diastereoisomer A)

TFA (1.0 mL) was added to a solution of intermediate 94a (20 mg) in anhydrous DCM (4 mL) previously cooled at 0°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried, concentrated *in vacuo* and the residue was purified by flash chromatography (DCM/MeOH from 9/1 to 6/4) to give the title compound (0.012 g) as a white foam.
T.I.c.: DCM/MeOH 8:2, Rf=0.15.
NMR (d₆-DMSO): δ (ppm) 7.91 (s, 1H); 7.64 (s, 2H); 7.19 (dd, 2H); 6.92 (dd, 2H); 4.62 (dd, 1 H); 4.34 (dd, 1H): 2.97 (dd, 1H); 2.96 (dd, 1H): 2.86 (dd, 1H): 2.83 (s, 3H); 2.62 (m, 1H); 2.59 (dd, 1 H); 2.57 (m, 1 H); 2.42 (m, 1H): 2.18 (m, 1H). 1.29 (m, 1H); 1.02 (m, 1H).
MS (ES/+): m/z=477 [M+H]⁺.

### Example 76

### N-{[3,5-bis(trifluoromethyl)Dhenynmethy}-3-(4-fluorophenyl)-N-methyl-3-(3-pyrrolidinyl)propionamide (diastereoisomer B)

TFA (1.0 mL) was added to a solution of intermediate 94b diastereoisomer 2 (20 mg) in anhydrous DCM (4 mL) previously cooled at O°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried, concentrated *in vacuo* and the residue was purified by flash chromatography (DCM/MeOH from 9/1 to 6/4) to give the title compound (0.011 g) as a white foam.
T.I.c.: DCM/MeOH 8:2, Rf=0.12.
NMR (d₆-DMSO): δ (ppm) 7.91 (s, 1H); 7.65 (s, 2H); 7.19 (dd, 2H); 6.92 (dd, 2H); 4.61 (dd, 1H); 4.36 (dd, 1H); 2.87 (dd, 1H); 2.96 (dd, 1H); 2.86 (dd, 1H); 2.84 (s, 3H); 2.75 (m, 1 H); 2.71 (m, 1 H); 2.68 (dd, 1 H); 2.32 (m, 1 H); 2.17 (m, 1H); 1.85 (m, 1H); 1.29 (m, 1 H).
MS (ES/+): m/z=477 [M+H]⁺.

### Example 77

### N-{[3.5-bis(trifluoromethyl)phenyl]methyl}-3-(4-fluorophenyl)-3-(3-fluoro-3-piperidinyl)-N-methylpropionamide (diastereoisomer A)

TFA (0.365 mL) was added to a solution of intermediate 97a (18 mg) in anhydrous DCM (1.8 mL) previously cooled to 0°C under a Nitrogen atmosphere. The resulting solution was stirred at 0°C for 1 hour, then the solution was concentrated *in vacuo.* The residue was diluted with DCM and washed with a saturated potassium carbonate solution. The aqueous layer was extracted with further DCM (2 x 2 mL). The combined organic extracts were dried and concentrated *in vacuo* to a residue which was purified by flash chromatography (DCM/MeOH 95:5 to 9:1) to give the title compound (7.8 mg) as a white foam.
T.I.c.: DCM/MeOH 8:2, Rf=0.64.
MS (ES/+): m/z=509 [M+H]⁺.

### Example 78

### N-{[3,5-bis(trifluoromethyl)phenyl]methyl}-3-(4-fluorophenyl)-3-(3-fluoro-3-piperidinyl)-N-methylpropionamide hydrochloride (diastereoisomer A)

Hydrochloric acid (1 M in Et2O - 11 µL) was added to a solution of example 77 (7.8 mg) in anhydrous Et2O (0.5 mL) previously cooled to 0°C under a Nitrogen atmosphere. The mixture was stirred at 0°C for 15 minutes, then the liquid phase was removed. The residue was triturated with pentane (3 x 0.3 mL) to give the title compound (5 mg) as a white solid.
NMR (d₆-DMSO): δ (ppm) 9.0-8.0 (b, 2H); 7.94 (s, 1 H); 7.66 (s, 2H); 7.31 (m, 2H); 7.07 (t, 2H); 4.7-4.3 (dd, 2H); 3.55 (m, 2H); 3.2-2.8 (m, 5H); 3.00 (s, 3H); 2.5-1.7 (m, 4H).
MS (ES/+): m/z=509 [M+H-HCI]⁺.

### Example 79

### N-{[3,5-bis(trifluoromethyl)phenyl]methyl}-3-(4-fluorophenyl)-3-(3-fluoro-3-Piperidinyl)-N-methylproplonamide (diastereoisomer B)

TFA (0.750 mL) was added to a solution of intermediate 97b (37 mg) in anhydrous DCM (3.7 mL) previously cooled to 0°C under a Nitrogen atmosphere. The resulting solution was stirred at 0°C for 1 hour, then the solution was concentrated *in vacuo.* The residue was diluted with DCM and washed with a saturated potassium carbonate solution. The aqueous layer was extracted with further DCM (2 x 4 mL). The combined organic extracts were dried and concentrated *in vacuo* to a residue which was purified by flash chromatography (DCM/MeOH 95:5 to 9:1) to give the title compound (20 mg) as a colourless oil.
T.I.c.: DCM/MeOH 8:2, Rf=0.56.
NMR (CDCl3): δ (ppm) 7.79 (s, 1H); 7.54 (s, 2H); 7.27 (m, 2H); 6.99 (t, 2H); 4.61 (q, 2H); 3.5 (m, 1 H); 3.0-2.4 (m, 9H); 1.8-1.2 (m, 4H).

### Example 80

### N-{[3,5-bis(trifluoromethyl)phenyl]methyl}-3-(4-fluorophenyl)-3-(3-fluoro-3-piperidinyl)-N-methylpropionamide hydrochloride (diastereoisomer B)

Hydrochloric acid (1 M in Et2O - 9 µL) was added to a solution of example 79 (4 mg) in anhydrous Et2O (0.5 mL) previously cooled to 0°C under a Nitrogen atmosphere. The mixture was stirred at 0°C for 15 minutes, then the liquid phase was removed. The residue was triturated with pentane (3 x 0.3 mL) to give the title compound (4 mg) as a white solid.
MS (ES/+): m/z=509 [M+H-HCl]⁺.

### Example 81

### N-{(1R)-1-[3,5-bis(trifluoromethyl)phenyl]ethyl}-3-(4-fluorophenyl)-N-methyl-3-(2-morpholinyl)propionamide(diastereoisomer 1)

TFA (1 mL) was added to a solution of intermediate 104a (31 mg) in DCM (4 mL) under a Nitrogen atmosphere. The resulting solution was stirred at r.t. for 3 hours, then the solution was diluted with further DCM and washed with a saturated potassium carbonate solution. The combined organic extracts were dried, concentrated *in vacuo* and the residue was purified by flash chromatography (from DCM to DCM/MeOH 80:20) to give the title compound (7 mg) as a pale yellow oil.
MS (ES/+): m/z=507 [M+H]⁺.

### Example 82

### N-{(1R)-1-[3,5-bis(trifluoromethyl)phenyl]ethyl}-3-(4-fluorophenyl)-N-methyl-3-(2-morpholinyl)propionamide hydrochloride(diastereoisomer 1)

Hydrochloric acid (1M in Et2O- 11 µL) was added to a solution of example 81 (7 mg) in anhydrous Et2O (0.5 mL) previously cooled to 0°C under a Nitrogen atmosphere. The mixture was stirred at 0°C for 30 minutes, then the liquid phase was removed. The residue was triturated with pentane (2 x 1 mL) to give the title compound (3.5 mg) as a white solid.
NMR (d₈-DMSO): δ (ppm) 8.86 (bs, 2H), 7.98 (s, 1H), 7.76 (s, 2H), 7.27 (m, 2H), 7.07 (t, 2H), 5.72 (m, 1H), 3.93 (m, 2H), 3.57 (t, 1H), 3.36 (m, 1H), 3.19 (d, 1 H), 3.05 (d, 1H), 2.90 (m, 1H), 2.75 (m, 2H), 2.71 (s, 3H), 2.30 (bm, 1H), 1.38 (d, 3H).
MS (ES/+): m/z=507 [M+H]⁺.

### Example 83

### N-{(1R)-1-(3,5-bis(trifluoromethyl)phenyllethyl}-3-(4-fluorophenyl)-N-methyl-3-(2-morpholinyl)propionamide (diastereoisomer 2)

TFA (1 mL) was added to a solution of intermediate 104b (20 mg) in DCM (4 mL) under a Nitrogen atmosphere. The resulting solution was stirred at r.t. for 3 hours, then the solution was diluted with further DCM and washed with a saturated potassium carbonate solution. The combined organic extracts were dried, concentrated *in vacuo* and the residue was purified by flash chromatography (from DCM to DCM/MeOH 80:20) to give the title compound (15 mg) as a pale yellow oil.
MS (ES/+): m/z=507 [M+H]⁺.

### Example 84

### N-{(1R)-1-(3,5-bis(trifluoromethyl)phenyl]ethyl}-3-(4-fluorophenyl)-N-methyl-3-12-morpolinyl)propionamide hydrochloride(diastereoisomer 2)

Hydrochloric acid (1M in Et2O - 32 µL) was added to a solution of example 83 (15 mg) in anhydrous Et2O (0.5 mL) previously cooled to 0°C under a Nitrogen atmosphere. The mixture was stirred at 0°C for 30 minutes, then the liquid phase was removed. The residue was triturated with pentane (2 x 1 mL) to give the title compound (7 mg) as a white solid.
NMR (d₆-DMSO): δ (ppm) 8.66 (bs, 2H), 7.93 (s, 1H), 7.61 (s, 2H), 7.26 (m, 2H), 7.02 (t, 2H), 5.76 (m, 1H), 3.93 (m, 1H), 3.84 (m, 1 H), 3.58 (t, 1H), 3.38 (m, 1H), 3.18 (d, 1H), 3.04 (d. 1H), 2.90-2.70 (m, 3H), 2.70 (s, 3H), 2.35 (t, 1H), 1.46 (d, 3H).
MS (ES/+): m/z=507 [M+H]⁺.

### Example 85

### N-{(1R)-1-[3,5-bis(trifluoromethyl)phenyl][ethyl}-3-(4-fluorophenyl)-N-methyl-3-(2-morpholinyl)propionamide (diastereoisomer 3)

TFA (0.5 mL) was added to a solution of intermediate 105a (16 mg) in DCM (2 mL) under a Nitrogen atmosphere. The resulting solution was stirred at r.t. for 3 hours, then the solution was diluted with further DCM and washed with a saturated potassium carbonate solution. The combined organic extracts were dried, concentrated *in vacuo* and the residue was purified by flash chromatography (from DCM to DCM/MeOH 80:20) to give the title compound (8.5 mg) as a pale yellow oil.
MS (ES/+): m/z=507 [M+H]⁺.

### Example 86

***N*-{(1*R*)-1-[3,5-bis(trifluoromethyl)phenyl]ethyl}-3-(4-fluorophenyl)-*N*-methyl-3-(2-morpholinyl)propionamidehydrochloride (diastereoisomer 3)** Hydrochloric acid (1M in Et2O - 18 µL) was added to a solution of example 85 (8.5 mg) in anhydrous Et2O (0.5 mL) previously cooled to 0°C under a Nitrogen atmosphere. The mixture was stirred at 0°C for 30 minutes, then the liquid phase was removed. The residue was triturated with pentane (2 x 1 mL) to give the title compound (7.5 mg) as a white solid.
NMR (d₆-DMSO): δ (ppm) 8.81 (bs, 2H), 7.96 (s, 1H), 7.74 (s, 2H), 7.29 (m, 2H), 7.09 (t, 2H), 5.65 (m, 1H), 3.91 (m, 1H), 3.77 (m, 1H), 3.64 (t, 1H), 3.27 (m, 1H), 3.09 (d, 1H), 2.93 (d, 1H), 2.86 (m, 2H), 2.70 (s, 3H), 2.80-2.60 (m, 2H), 1.30 (d, 3H).
MS (ES/+): m/z=507 [M+H]⁺.

### Example 87

### N-((1R)-1-[3,5-bis(trifluoromethyl)phenyl]ethyl}-3-(4-fluorophenyl)-N-methyl-3-(2-morpholinyl)propionamide (diastereoisomer 4)

TFA (0.5 mL) was added to a solution of intermediate 105b (19 mg) in DCM (2 mL) under a Nitrogen atmosphere. The resulting solution was stirred at r.t. for 3 hours, then the solution was diluted with further DCM and washed with a saturated potassium carbonate solution. The combined organic extracts were dried, concentrated *in vacuo* and the residue was purified by flash chromatography (from DCM to DCM/MeOH 80:20) to give the title compound (11 mg) as a pale yellow oil.
MS (ES/+): m/z=507 [M+H]⁺.

### Example 88

### N-{(1R)-1-[3,5-bis(trifluoromethyl)phenyl]ethyl}-3-(4-fluorophenyl)-N-methyl-3-(2-morpholinyl)propionamidehydrochloride (diastereoisomer 4)

Hydrochloric acid (1M in Et2O - 23 µL) was added to a solution of example 87 (11 mg) in anhydrous Et2O (0.5 mL) previously cooled to 0°C under a Nitrogen atmosphere. The mixture was stirred at 0°C for 30 minutes, then the liquid phase was removed. The residue was triturated with pentane (2 x 1 mL) to give the title compound (7.5 mg) as a white solid.
NMR (d₆-DMSO): δ (ppm) 8.80 (bs, 2H), 7.91 (s, 1H), 7.50 (s, 2H), 7.27 (m, 2H), 7.01 (t, 2H), 5.72 (m, 1H), 3.98 (M, 1H), 3.76 (m, 1H), 3.65 (t, 1H), 3.27 (m, 1H), 3.10 (d, 1H), 3.00-2.80 (d, 3H), 2.68 (m, 2H), 2.64 (s, 3H), 1.40 (d, 3H).
MS (ES/+): m/z=507 [M+H]⁺.

### Example 89

### N-{[3,5-bis(trifluoromethyl)phenyl]methyl}-3-(4-fluorophenyl)-N-methyl-3-(3-piperidinyl)propionamide (diastereoisomer A)

TFA (0.375 mL) was added to a solution of intermediate 109a (15 mg) in anhydrous DCM (1.7 mL) previously cooled at 0°C, under a Nitrogen atmosphere. The solution was stirred at 0°C for 1 hours, then it was concentrated *in vacuo* at 0°C. The residue was purified by SCX cartridge (load with DCM and elution with MeOH, NH3 0.25M in MeOH) to give the title compound as a colourless oil:
T.I.c.: AcOEt 100%. Rf=0.15.
NMR (CDCl₃): δ (ppm) 7.76 (s, 1H); 7.52 (s, 2H); 7.12 (dd, 2H); 6.95 (t, 2H); 4.67-4.45 (dd, 2H); 3.3-2.1 (m, 7H); 2.1-0.8 (m, 5H); 2.86 (s, 3H).
MS (ES/+): m/z=491 [M+H]⁺.

### Example 90

### N-{[3,5-bis(trifluoromethyl)phenyl]methyl}-3-(4-fluorophenyl)-N-methyl-3-(3-piperidinyl)propionamide (diastereoisomer B)

TFA (0.75 mL) was added to a solution of intermediate **109b** (40 mg) in anhydrous DCM (3.7 mL) previously cooled at 0°C, under a Nitrogen atmosphere. The solution was stirred at 0°C for 1 hours, then it was concentrated *in vacuo* at 0°C. The residue was purified by SCX cartridge (load with DCM and elution with MeOH, NH3 0.25M in MeOH) to give the title compound as a colourless oil:
T.l.c.: AcOEt 100%, Rf=0.15.
NMR (CDCl3): δ (ppm) 7.76 (s, 1 H); 7.52 (s, 2H); 7.13 (dd, 2H); 6.93 (t, 2H); 4.62-4.46 (dd, 2H); 3.1-2.1 (m, 7H); 2.1-1.1 (m, 5H); 2.85 (s, 3H).
MS (ES/+): m/z=491 [M+H]⁺.

### Example 91

***N*-{[3,5-bis(trifluoromethyl)phenyl]methyl)-3-(4-fluorophenyl)-*N*-methyl-3-(4-pyridinyl]propionamideDIPEA** (85 µL) and O-(benzotriazol-1-yl)-N,N,N'N'-tetramethyluronium tetrafluoroborate (50.9 mg) were added to a solution of intermediate 112 (30 mg) in anhydrous DMF (3.5 mL) under a Nitrogen atmosphere. After stirring for 10 minutes, {[3,5-bis(trifluoromethyl)phenyl]methyl}methylamine hydrochloride (53.8 mg) was added. The mixture was stirred at r.t. for 16 hours, then it was diluted with AcOEt, washed with a 5% sodium hydrogen carbonate solution and brine. The organic layer was dried, concentrated *in vacuo* and the residue was purified by flash chromatography (CH, CH/AcOEt 1:1, then AcOEt) to give the title compound (42 mg) as a white solid.
T.I.c.: AcOEt, Rf=0.13 (detection with ninhydrine).
NMR (d₆-DMSO): δ (ppm) 8.36 (d, 2H); 7.92 (s, 1H); 7.70 (s, 2H); 7.35 (dd, 2H); 7.32 (d, 2H); 7.02 (dd, 2H); 4.58 (d, 1 H); 4.53 (d, 1 H); 4.51 (dd, 1 H); 3.28 (dd, 1 H); 3.19 (dd, 1 H); 3.02 (s, 3H).
MS (ES/+): m/z=485 [M+H]⁺.

### Pharmacy examples

**A. Capsules/ Tablets**

| | |
|---|---|
| Active ingredient | 25.0mg |
| PVP | 2.5mg |
| Microcrystalline Cellulose | 198.5mg |
| Croscarmellose Sodium | 2.5mg |
| Magnesium Stearate | 1.5mg |

The active ingredient is blended with the other excipients. The blend can be used to fill gelatin capsules or compressed to form tablets using appropriate punches. The tablets can be coated using conventional techniques and coatings.

**B. Tablets**

| | |
|---|---|
| Active ingredient | 25.0mg |
| Microcrystalline Cellulose | 264.0mg |
| Croscarmellose Sodium | 10.0mg |
| Magnesium Stearate | 1.0mg |

The active ingredient is blended with microcrystalline cellulose and croscarmellose sodium. Magnesium stearate is then added to the previous blend. The mixture thus obtained can be compressed using appropriate punches and the tablets coated using conventional techniques and coatings.

**C) Infusion**

| | |
|---|---|
| Active ingredient | 2-50 mg/ml |
| Buffer solution pH 4.5 suitable for infusion | qs to 100ml |
| (e.g. sodium citrate in NaCl 0.9% or 5% dextrose) | |

The formulation may be packed in glass vials or plastic bag.

The affinity of the compound of the invention for the NK₁ receptor was determined using the NK₁ receptor binding affinity method measuring in vitro by the compounds' ability to displace [3H] - substance P (SP) from recombinant human NK₁ receptors expressed in Chinese Hamster Ovary (CHO) cell membranes. The affinity values are expressed as negative logarithm of the inhibition constant (Ki) of displacer ligands (pKi).
The pKi values obtained as the average of at least two determinations with representative compounds of the invention are within the range of 10.44 to 7.54.

The inhibitory activity of the compound of the invention at the human serotonin transporter was determined using the hSERT uptake affinity method and measuring the compounds' ability to inhibit uptake of [3H] 5HT in hSERT-LLCPK cells. The data have been digitally processed to obtain the pIC50 values of the antagonists. The pIC50 values obtained as the average of at least two determinations with representative compounds of the invention are within the range of 7.19 to 6.24.

The inhibitory activity of the compound of the invention at the rat serotonin transporter was determined using the rSERT uptake affinity method and measuring the compounds' ability to inhibit uptake of [3H] 5HT in rSERT-LLCPK cells. The data have been digitally processed to obtain the plC50 values of the antagonists. The plC50 values obtained as the average of at least two determinations with representative compounds of the invention are within the range of 7.38 to 3.97.

Particularly preferred compounds of the invention have shown a NK1 receptor binding affinity (pki) greater than 8 and an inhibitory activity (plC50) at the hSERT greater than 7.

## Claims

1. A compound of formula(I) 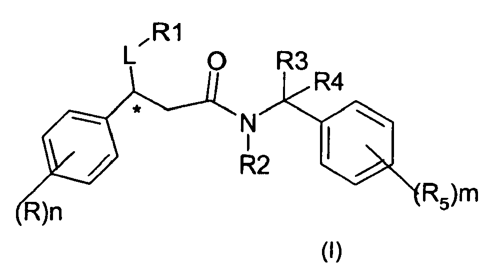 wherein
R represents halogen, C₁₋₄ alkyl, cyano, C₁₋₄ alkoxy, trifluoromethyl or trifluoromethoxy;
R₁ represents a 5 or 6 membered heteroaryl group, in which the 5-membered heteroaryl group contains at least one heteroatom selected from oxygen, sulphur or nitrogen and the 6-membered heteroaryl group contains from 1. to 3 nitrogen atoms, or R₁ represents a 4, 5 or 6 membered heterocyclic group, wherein said 5 or 6 membered heteroaryl or the 4, 5 or 6 membered heterocyclic group may optionally be substituted by one to three substituents, which may be the same or different, selected from (CH₂)ₚR₆, wherein p is zero or an integer from 1 to 4 and R₆ is selected from:
halogen,
C₁₋₄alkoxy,
C₁₋₄alkyl.
C₃₋₇cycloalkyl,
C₁₋₄ alkyl optionally substituted by halogen, cyano or C₁₋₄ alkoxy,
hydroxy,
cyano,
nitro,
trifluoromethyl,
carboxy,
NH(C₁₋₄ alkyl),
N(C₁₋₄ alkyl)₂,
NH(C₃₋₇ cycloalkyl),
N(C₁₋₄ alkyl)(C₃₋₇ cycloalkyl),
NH(C₁₋₄alkylOC₁₋₄alkoxy),
OC(O)NR₇R₈,
NR₈C(O) R₇ or
C(O)NR₇R₈;
R₂ represents hydrogen, or C₁₋₄ alkyl ;
R₃ and R₄ independently represent hydrogen, C₁₋₄ alkyl or R₃ together with R₄ represents C₃₋₇ cycloalkyl;
R₅ represents trifluoromethyl, S(O)_{q}C ₁₋₄ alkyl, C₁₋₄ alkyl, C₁₋₄ alkoxy, trifluoromethoxy, halogen or cyano;
R₇ and R₈ independently represent hydrogen, C₁₋₄ alkyl or C₃₋₇ cycloalkyl;
L is a single or a double bond;
n is an Integer from 1 to 3;
m is zero or an Integer from 1 to 3;
q is zero or an integer from 1 to 2;
provided that
a) when L is a double bond, R₁ is not an optionally substituted 5 or 6 membered heteroaryl group, in which the 5-membered heteroaryl group contains at least one heteroatom selected from oxygen, sulphur or nitrogen and the 6-membered heteroaryl group contains from 1 to 3 nitrogen atoms;
b) the group R₁ is linked to the carbon atom shown as via a carbon atom;
and
c) when the heteroatom contained in the group R₁ is substituted, p is not zero;
and pharmaceutically acceptable salts and solvates thereof.

2. A compound as claimed in claim 1 wherein R is halogen (e.g. fluorine or chlorine) and/or a C₁₋₄ alkyl (e.g. methyl) group and n is an integer from 1 to 2.

3. A compound as claimed in claim 1 or claim 2 wherein R₅ is trifluorymethyl, methyl, methoxy, bromine, chlorine or fluorine atom and m is an integer from 1 to 2.

4. A compound as claimed In any of claims 1 to 3 wherein R₁ is piperidyl, morpholinyl, 1,2,3,6-tetrahydro-4-pyridinyl, pyridyl or pyrrolidinyl.

5. A compound as claimed in any of claims 1 to 4 wherein R is halogen (e.g. fluorine or chlorine) and/or a C₁₋₄ alkyl (e.g. methyl) group and n is an integer from 1 to 2; R₁ is piperidyl, 2-morpholinyl, 1,2,3,6-tetrahydro-4-pyridinyl, pyridyl or pyrrolidinyl and wherein R₁ is optionally substituted by one or two groups selected from halogen (e.g fluorine), C₁₋₄ alkyl (e.g. methyl) or ethylC₁₋₄ alkoxy; R₂ and R₃ are independently hydrogen or methyl; R₄ is hydrogen, methyl or together with R₃ is cyclopropyl and R₅ is trifluoromethyl, methyl, methoxy, bromine, chlorine or fluorine atom and m is preferably an integer from 1 to 2.

6. A compound according to Claim 1 selected from:
*N*-(3,5-Bis-trifluoromethyl-benzyl)-3-(4-fluoro-phenyl)-*N*-methyl-3-piperidin-4-yl-prypionamide;
*N*-(3,5-Dichloro-benzyl)-3-(4-fluoro-phenyl)-*N*-methyl-3-piperidin-4-yl-propionamide;
*N*-[1-(3,5-Dichloro-phenyl)-ethyl]-3-(4-fluoro-phenyl)-*N*-methyl-3-piperidin-4-yl-propionamide;
*N-*[1-(3,5-Dichloro-phenyl)-ethyl]-3-(4-fluoro-phenyl)-*N*-methyl-3-[1-(2-methoxyethyl)-piperidin-4-yl]-propionamide;
*N*-(3,5-Dichioro-benzyl)-3-(4-fluoro-phenyl)-3-(4-fluoro-piperidin-4-yl)-*N*-methylpropionamide;
*N*-([3,5-bis(trifluoromethyl)phenyl]methyl)-3-(4-fluorophenyl)-*N*-methyl-3-(l-[2-(methyloxy)ethyl]-4-piperidinyl}propionamide; *N*-{-1-[3,5-bis(trifluoromethyl)phenyl]ethyl}-3-(4-fluorophenyl)-*N*-methyl-3-(4-piperidinyl)propionamide;
*N*-{1-[3.5-bis(trif)uoromethy))pheny)]-1-methy)ethyl}-3-(4-fluorophenyl)-3-(4-piperidinyl)propionamide;
*N*-{[3-bromo-4-(methyloxy)phenyl]methyl}-3-(4-fluorophenyl)-*N*-methyl-3-(4-piperidinyl)propionamide;
*N*-[(3,5-dimethylphenyl)methyl)-3-(4-fluorophenyl)-*N*-methyl-3-(4-piperidinyl)propionamide;
*N*-[(3,4-dibromophenyl)methyl]-3-(4-fluorophenyl)-*N*-methyl-3-(4-piperidinyl)propionamide;
*N*-[(3-fluoro-2-methylphenyl)methyl]-3-(4-fluorophenyl)-*N*-methyl-3-(4-piperidinyl)propionamide;
*N*-{[2-chloro-3-(trifluoromethyl)phenyl]methyl}-3-(4-fluorophenyl)-*N*-methyl-3-(4-piperidinyl)propionamide;
*N*-{-1-[3,5-bis(trifluoromethyl)phenyl]ethyl}-3-(4-fluorophenyl)-3-(4-fluoro-4-piperidinyl)-*N-*methylpropionamide;
*N*-[(3,5-dibromophenyl)methyl]-3-(4-fluorophenyl)-3-(4-fluoro-4-piperidinyl)-*N-*methylpropionamide;
*N*-{-1-[3,5-bis(trifluoromethyl)phenyljethyl}-3-(2,4-dichlorophenyl)-3-(4-fluoro-4-piperidinyl)-*N*-methylpropionamide;
*N*-{-1-[3,5-bis(trifluoromethyl)phenyl]ethyl}-3-(4-fluoro-2-methylphenyl)-3-(4-fluoro-4-piperidinyl)-*N*-methylpropionamide;
*N*-[(3,5-dibromophenyl)methyl]-3-(4-fluoro-2-methylphenyl)-3-(4-fluoro-4-piperidinyl)-*N-*methylpropionamide;
*N*-[(3,5-dibromophenyl)methyl]-3-(3,4-dichlorophenyl)-3-(4-fluoro-4-piperidinyl)-*N-*methylpropionamide;
*N*-{[3,5-bis(trifluoromethyl)phenyl]methyl}-3-(4-fluorophenyl)-3-(4-fluoro-4-piperidinyl)-*N-*methylpropionamide;
3-(4-chlorophenyl)-*N*-[(3,5-dibromophenyl)methyl]-3-(4-fluoro-4-piperidinyl)-*N-*methylpropionamide;
*N*-{[3,5-bis(trifluoromethyl)phenyl]methyl}-3-(4-fluorophenyl)-*N*-methyl-3-(3-piperidinylidene)propionamide;
*N*-[(3,5-dibromophenyl)methyl]-3-(4-fluorophenyl)-*N*-methyl-3-(4-piperidinylidene)propionamide;
*N*-{[3,5-bis(trifluoromethyl)phenyl]methyl}-3-(4-fluoro-2-methylphenyl)-*N*-methyl-3-(1,2,3,6-tetrahydro-4-pyridinyl)propionamide;
*N-*{(1*R*)-1-[3,5-bis(trifluoromethyl)phenyl]ethyl}-3-(4-fluoro-2-methylphenyl)-*N*-methyl-3-(1,2,3,6-tetrahydro-4-pyridinyl)propionamide;
*N*-{[3,5-bis(trifluoromethyl)phenyl]methyl}-3-(4-fluorophenyl)-*N*-methyl-3-(3-pyrrolidinyl)propionamide;
*N*-{[3,5-bis(trifluoromethyl)phenyl]methyl}-3-(4-fluorophenyl)-3-(3-fluoro-3-piperidinyl)-*N-*methylpropionamide;
*N*-{-1-[3,5-bis(trifluoromethyl)phenyl]ethyl}-3-(4-fluorophenyl)-*N*-methyl-3-(2-morpholinyl)propionamide;
*N*-{[3,5-bis(trifluoromethyl)phenyl]methyl}-3-(4-fluorophenyl)-*N*-methyl-3-(3-piperidinyl)propionamide;
*N*-{[3,5-bis(trifluoromethyl)phenyl]methyl}-3-(4-fluorophenyl)-*N*-methyl-3-(4-pyridinyl)propionamide;
and enantiomers, diastereoisomers, pharmaceutically acceptable salts(e.g hydrochloride) and solvates thereof.

7. A compound selected from
*N*-{(1*R*)-1-[3,5-bis(trifluoromethyl)phenyl]ethyl}-3-(4-fluorophenyl)-*N*-methyl-3-(4-piperidinyl)propionamide(diastereoisomer 1);
*N*-{(1*S*)-1-[3,5-bis(trifluoromethyl)phenyl]ethyl}-3-(4-fluorophenyl)-*N*-methyl-3-(4-piperidinyl)propionamide (diastereoisomer 2);
*N*-{(1*R*)-1-[3,5-bis(trifluoromethyl)phenyl]ethyl}-3-(4-fluorophenyl)-3-(4-fluoro-4-piperidinyl)-*N*-methylpropionamide (diastereoisomer 1);
*N*-[(3,5-dibromophenyl)methyl]-3-(4-fluorophenyl)-3-(4-fluoro-4-piperidinyl)-*N-*methylpropionamide (enantiomer 2);
*N*-{[3,5-bis(trifluoromethyl)phenyl]methyl}-3-(4-fluorophenyl)-3-(3-fluoro-3-piperidinyl)-*N-*methylpropionamide (diastereoisomer A);
and pharmaceutically acceptable salts(e.g hydrochloride) and solvates thereof.

8. A process for the preparation of a compound as claimed in claim 1 which comprises reacting an activated derivative of the carboxylic acid of formula (II) wherein R₁ has the meaning previously defined or is a protected group thereof, with amine (III) 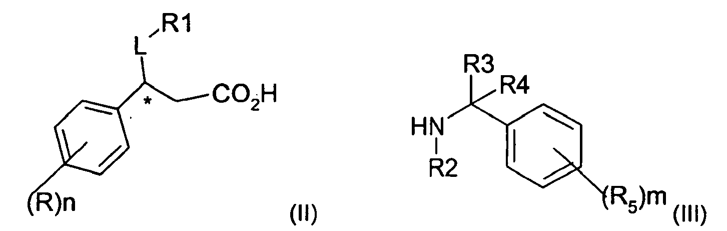 wherein R₂ is C₁₋₄ alkyl or a nitrogen protecting group, followed where necessary by removal of any protecting group.

9. A compound as claimed in any claims 1 to 7 for use in therapy.

10. The use of a compound as claimed in any claims 1 to 7 in the preparation of a medicament for use in the treatment of conditions mediated by tachykinins (including substance P and other neurokinins) and/or by selective inhibition of the serotonin reuptake transporter protein.

11. A pharmaceutical composition comprising a compound as claimed in any claims 1 to 7 in admixture with one or more pharmaceutically acceptable carriers or excipients.

## Patentansprüche

1. Verbindung der Formel (I): 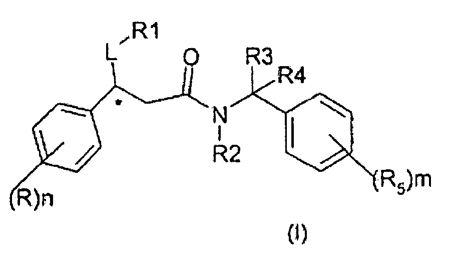 wobei
R Halogen, C₁₋₄-Alkyl, Cyano, C₁₋₄-Alkoxy, Trifluormethyl oder Trifluormethoxy darstellt;
R₁ einen 5- oder 6-gliedrigen Heteroarylrest darstellt, wobei der 5-gliedrige Heteroarylrest mindestens ein Heteroatom enthält, ausgewählt aus Sauerstoff, Schwefel oder Stickstoff, und der 6-gliedrige Heteroarylrest 1 bis 3 Stickstoffatome enthält, oder R₁ einen 4-, 5- oder 6-gliedrigen heterocyclischen Rest darstellt, wobei der 5- oder 6-gliedrige Heteroarylrest oder der 4-, 5-, oder 6-gliedrige heterocyclische Rest gegebenenfalls mit 1 bis 3 Substituenten substituiert sein kann, welche gleich oder voneinander verschieden sein können, ausgewählt aus (CH₂)ₚR₆, wobei p null oder eine ganze Zahl von 1 bis 4 ist und R₆ ausgewählt ist aus:
Halogen,
C₁₋₄-Alkoxy,
C₁₋₄-Alkyl,
C₃₋₇-Cycloalkyl,
C₁₋₄-Alkyl, gegebenenfalls substituiert mit Halogen, Cyano oder C₁₋₄-Alkoxy,
Hydroxy,
Cyano,
Nitro,
Trifluormethyl,
Carboxy,
NH(C₁₋₄-Alkyl),
N(C₁₋₄-Alkyl)₂,
NH(C₃₋₇-Cycloalkyl),
N(C₁₋₄-Alkyl)(C₃₋₇-cycloalkyl),
NH(C₁₋₄-AlkylOC₁₋₄-alkoxy),
OC(O)NR₇R₈,
NR₈C(O)R₇ oder
C(O)NR₇R₈;
R₂ Wasserstoff oder C₁₋₄-Alkyl darstellt;
R₃ und R₄ unabhängig voneinander Wasserstoff, C₁₋₄-Alkyl darstellen oder R₃ zusammen mit R₄ C₃₋₇-Cycloalkyl darstellt;
R₅ Trifluormethyl, S(O)_{q}C₁₋₄-Alkyl, C₁₋₄-Alkyl, C₁₋₄-Alkoxy, Trifluormethoxy, Halogen oder Cyano darstellt;
R₇ und R₈ unabhängig voneinander Wasserstoff, C₁₋₄-Alkyl oder C₃₋₇-Cycloalkyl darstellen;
L eine Einfach- oder eine Doppelbindung ist;
n eine ganze Zahl von 1 bis 3 ist;
m null oder eine ganze Zahl von 1 bis 3 ist;
q null oder eine ganze Zahl von 1 bis 2 ist;
mit der Maßgabe, dass
(a) wenn L eine Doppelbindung ist, R₁ kein gegebenenfalls substituierter 5- oder 6-gliedriger Heteroarylrest ist, wobei der 5-gliedrige Heteroarylrest mindestens ein Heteroatom enthält, ausgewählt aus Sauerstoff, Schwefel oder Stickstoff, und der 6-gliedrige Heteroarylrest 1 bis 3 Stickstoffatome enthält;
(b) der Rest R₁ an das als * gezeigte Kohlenstoffatom durch ein Kohlenstoffatom gebunden ist;
und
(c) wenn das im Rest R₁ enthaltene Heteroatom substituiert ist, p nicht null ist;
und pharmazeutisch verträgliche Salze und Solvate davon.

2. Verbindung gemäß Anspruch 1, wobei R ein Halogenatom (z.B. Fluor oder Chlor) ist und/oder ein C₁₋₄-Alkylrest (z.B. Methyl) ist und n eine ganze Zahl von 1 bis 2 ist.

3. Verbindung gemäß Anspruch 1 oder Anspruch 2, wobei R₅ ein Trifluormethylrest, eine Methyl-, Methoxygruppe, ein Brom-, Chlor- oder Fluoratom ist und m eine ganze Zahl von 1 bis 2 ist.

4. Verbindung gemäß einem der Ansprüche 1 bis 3, wobei R₁ Piperidyl, Morpholinyl, 1,2,3,6-Tetrahydro-4-pyridinyl, Pyridyl oder Pyrrolidinyl ist.

5. Verbindung gemäß einem der Ansprüche 1 bis 4, wobei R ein Halogenatom (z.B. Fluor oder Chlor) ist und/oder ein C₁₋₄-Alkylrest (z.B. Methyl) ist und n eine ganze Zahl von 1 bis 2 ist; R₁ Piperidyl, 2-Morpholinyl, 1,2,3,6-Tetrahydro-4-pyridinyl, Pyridyl oder Pyrrolidinyl ist und wobei R₁gegebenenfalls mit einem oder zwei Resten substituiert ist, ausgewählt aus einem Halogenatom (z.B. Fluor), einem C₁₋₄-Alkylrest (z.B. Methyl) oder Ethyl-C₁₋₄-alkoxy; R₂ und R₃ unabhängig voneinander Wasserstoff oder Methyl sind; R₄ Wasserstoff, Methyl oder zusammen mit R₃ Cyclopropyl ist und R₅ ein Trifluormethylrest, eine Methyl-, Methoxygruppe, ein Brom-, Chlor- oder Fluoratom ist und m vorzugsweise eine ganze Zahl von 1 bis 2 ist.

6. Verbindung gemäß Anspruch 1, ausgewählt aus:
*N*-(3,5-Bistrifluormethylbenzyl)-3-(4-fluorphenyl)-*N*-methyl-3-piperidin-4-yl-propionamid;
*N*-(3,5-Dichlorbenzyl)-3-(4-fluorphenyl)-*N*-methyl-3-piperidin-4-yl-propionamid;
*N*-[1-(3,5-Dichlorphenyl)ethyl]-3-(4-fluorphenyl)-*N*-methyl-3-piperidin-4-yl-propionamid;
*N*-[1-(3,5-Dichlorphenyl)ethyl]-3-(4-fluorphenyl)-*N*-methyl-3-[1-(2-methoxyethyl)-piperidin-4-yl]propionamid;
*N*-(3,5-Dichlorbenzyl)-3-(4-fluorphenyl)-3-(4-fluorpiperidin-4-yl)-*N*-methylpropionamid;
*N*-{ ([3,5-Bis(trifluormethyl)phenyl]methyl)}-3-(4-fluorphenyl)-*N*-methyl-3-{1-[2-(methyloxy)ethyl]-4-piperidinyl}propionamid-*N*-{1-[3,5-bis(trifluormethyl)-phenyl]ethyl}-3-(4-fluorphenyl)-*N*-methyl-3-(4-piperidinyl)propanamid;
*N*-{1-[3,5-Bis(trifluormethyl)phenyl]-1-methylethyl}-3-(4-fluorphenyl)-3-(4-piperidinyl)-propionamid;
*N*-{[3-Brom-4-(methyloxy)phenyl]methyl}-3-(4-fluorphenyl)-*N-*methyl-3-(4-piperidinyl)propionamid;
*N*-[(3,5-Dimethylphenyl)methyl]-3-(4-fluorphenyl)-*N*-methyl-3-(4-piperidinyl)-propionamid;
*N*-[(3,4-Dibromphenyl)methyl]-3-(4-fluorphenyl)-*N*-methyl-3-(4-piperidinyl)-propionamid;
*N*-[(3-Fluor-2-methylphenyl)methyl]-3-(4-fluorphenyl)-*N*-methyl-3-(4-piperidinyl)-propionamid;
*N*-{[2-Chlor-3-(trifluormethyl)phenyl]methyl}-3-(4-fluorphenyl)-*N*-methyl-3-(4-piperidinyl)propionamid;
*N*-{1-[3,5-Bis(trifluormethyl)phenyl]ethyl}-3-(4-fluorphenyl)-3-(4-fluor-4-piperidinyl)-*N*-methylpropionamid;
*N*-[(3,5-Dibromphenyl)methyl]-3-(4-fluorphenyl)-3-(4-fluor-4-piperidinyl)-*N*-methylpropionamid;
*N*-{1-[3,5-Bis(trifluormethyl)phenyl]ethyl}-3-(2,4-dichlorphenyl)-3-(4-fluor-4-piperidinyl)-*N*-methylpropionamid;
*N*-{1-[3,5-Bis(trifluormethyl)phenyl]ethyl}-3-(4-fluor-2-methylphenyl)-3-(4-fluor-4-piperidinyl)-*N*-methylpropionamid;
*N*-[(3,5-Dibromphenyl)methyl]-3-(4-fluor-2-methylphenyl)-3-(4-fluor-4-piperidinyl)-*N-*methylpropionamid;
*N*-[(3,5-Dibromphenyl)methyl]-3-(3,4-dichlorphenyl)-3-(4-fluor-4-piperidinyl)-*N-*methylpropionamid;
*N*-{[3,5-Bis(trifluormethyl)phenyl]methyl}-3-(4-fluorphenyl)-3-(4-fluor-4-piperidinyl)-*N*-methylpropionamid;
3-(4-Chlorphenyl)-*N*-[(3,5-dibromphenyl)methyl]-3-(4-fluor-4-piperidinyl)-*N*-methylpropionamid;
*N*-{[3,5-Bis(trifluormethyl)phenyl]methyl}-3-(4-fluorphenyl)-*N*-methyl-3-(3-piperidinyliden)propionamid;
*N*-[(3,5-Dibromphenyl)methyl]-3-(4-fluorphenyl)-*N*-methyl-3-(4-piperidinyliden)propionamid;
*N*-{[3,5-Bis(trifluormethyl)phenyl]methyl}-3-(4-fluor-2-methylphenyl)-*N*-methyl-3-(1,2,3,6-tetrahydro-4-pyridinyl)propionamid;
*N*-{(1*R*)-1-[3,5-Bis(trifluormethyl)phenyl]ethyl}-3-(4-fluor-2-methylphenyl)-*N*-methyl-3-(1,2,3,6-tetrahydro-4-pyuidinyl)propionamid;
*N*-{[3,5-Bis(trifluormethyl)phenyl]methyl-3-(4-fluorphenyl)-*N*-methyl-3-(3-pyrrolidinyl)propionamid;
*N*-{[3,5-Bis(trifluormethyl)phenyl]methyl}-3-(4-fluorphenyl)-3-(3-fluor-3-piperidinyl)-*N*-methylpropionamid;
*N*-{1-[3,5-Bis(trifluormethyl)phenyl]ethyl}-3-(4-fluorphenyl)-*N*-methyl-3-(2-morpholinyl)propionamid;
*N-*{[3,5-Bis(trifluormethyl)phenyl]methyl}-3-(4-fluorphenyl)-*N*-methyl-3-(3-piperidinyl)propionamid;
*N*-{[3,5-Bis(trifluormethyl)phenyl]methyl}-3-(4-fluorphenyl)-*N*-methyl-3-(4-pyridinyl)-propionamid;
und Enantiomere, Diastereosisomere, pharmazeutisch verträgliche Salze (z.B. Hydrochlorid) und Solvate davon.

7. Verbindung gemäß Anspruch 1, ausgewählt aus:
*N*-{(1*R*)-1-[3,5-Bis(trifluormethyl)phenyl]ethyl}-3-(4-fluorphenyl)-*N*-methyl-3-(4-piperidinyl)propionamid (Diastereoisomer 1);
*N*-{(1*S*)-1-[3,5-Bis(trifluormethyl)phenyl]ethyl-3-(4-fluorphenyl)-*N*-methyl-3-(4-piperidinyl)propionamid (Diastereoisomer 2);
*N*-{(1*R*)-1-[3,5-Bis(trifluormethyl)phenyl]ethyl}-3-(4-fluorphenyl)-3-(4-fluor-4-piperidinyl)-*N*-methylpropionamid (Diastereoisomer 1);
*N*-[(3,5-Dibromphenyl)methyl]-3-(4-fluorphenyl)-3-(4-fluor-4-piperidinyl)-*N*-methylpropionamid (Enantiomer 2);
*N*-{[3,5-Bis(trifluormethyl)phenyl]methyl }-3-(4-fluorphenyl)-3-(3-fluor-3-piperidinyl)-*N*-methylpropionamid (Diastereoisomer A);
und pharmazeutisch verträgliche Salze (z.B. Hydrochlorid) und Solvate davon.

8. Verfahren zur Herstellung einer Verbindung gemäß Anspruch 1, welches das Umsetzen eines aktivierten Derivats der Carbonsäure der Formel (II), wobei R₁ die vorstehend definierte Bedeutung hat oder eine geschützte Gruppe davon ist, mit Amin (III) 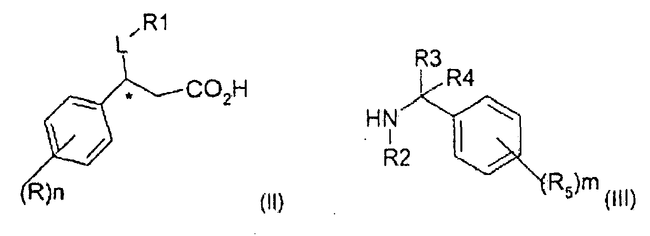 wobei R₂ C₁₋₄-Alkyl ist oder eine Stickstoff-Schutzgruppe ist, wennn nötig gefolgt vom Entfernen jeglicher Schutzgruppen, umfasst.

9. Verbindung gemäß einem der Ansprüche 1 bis 7 zur Verwendung in der Therapie.

10. Verwendung einer Verbindung gemäß einem der Ansprüche 1 bis 7 zur Herstellung eines Medikaments zur Behandlung von durch Tachykinine (einschließlich Substanz P und anderer Neurokinine) und/oder durch selektive Hemmung des Serotonin-Wiederaufnahme-Transportproteins vermittelten Zuständen.

11. Arzneimittel, umfassend eine Verbindung gemäß einem der Ansprüche 1 bis 7, als Gemisch mit einem oder mehreren pharmazeutisch verträglichen Trägern oder Exzipienten.

## Revendications

1. Composé de formule (I) 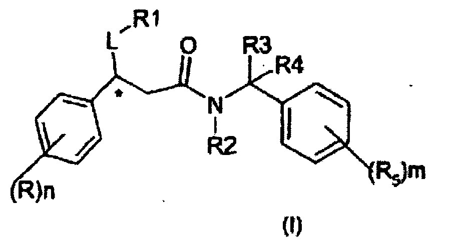 dans laquelle
R représente un groupe halogéno, alkyle en C₁ à C₄, cyano, alkoxy en C₁ à C₄, trifluorométhyle ou trifluorométhoxy ;
R₁ représente un groupe hétéroaryle penta- ou hexagonal, le groupe hétéroaryle pentagonal contenant au moins un hétéroatome choisi entre l'oxygène, le soufre et l'azote et le groupe hétéroaryle hexagonal contenant 1 à 3 atomes d'azote, ou bien R₁ représente un groupe hétérocyclique tétra-, penta- ou hexagonal, ledit groupe hétéroaryle penta- ou hexagonal ou le groupe hétérocyclique tétra-, penta- ou hexagonal pouvant être facultativement substitué avec 1 à 3 substituants, qui peuvent être identiques ou différents, choisis parmi des substituants (CH₂)ₚR₆, dans lesquels p est égal à 0 ou un nombre entier de 1 à 4 et R₆ est choisi entre des groupes :
halogéno,
alkoxy en C₁ à C₄,
alkyle en C₁ à C₄,
cycloalkyle en C₃ à C₇,
alkyle en C₁ à C₄ facultativement substitué avec un substituant halogéno, cyano ou alkoxy en C₁ à C₄,
hydroxy,
cyano,
nitro,
trifluorométhyle,
carboxy,
NH(alkyle en C₁ à C₄),
N(alkyle en C₁ à C₄)₂
NH (cycloalkyle en C₃ à C₇),
N(alkyle en C₁ à C₄) (cycloalkyle en C₃ à C₇),
NH((alkyle en C₁ à C₄) O (alkoxy en C₁ à C₄)),
OC(O)NR₇R₈,
NR₈C (O) R₇ ou
C (O) NR₇R₈;
R₂ représente un atome d'hydrogène ou un groupe alkyle en C₁ à C₄ ;
R₃ et R₄ représentent indépendamment un atome d'hydrogène, un groupe alkyle en C₁ à C₄ ou bien R₃, conjointement avec R₄ représente un groupe cycloalkyle en C₃ à C₇ ;
R₅ représente un groupe trifluorométhyle, S(O)_{q}(alkyle en C₁ à C₄), alkyle en C₁ à C₄, alkoxy en C₁ à C₄, trifluorométhoxy, halogéno ou cyano ;
R₇ et R₈ représentent indépendamment un atome d'hydrogène, un groupe alkyle en C₁ à C₄ ou cycloalkyle en C₃ à C7 ;
L représente une liaison simple ou une double liaison ;
n représente un nombre entier de 1 à 3;
m est égal à 0 ou à un nombre entier de 1 à 3 ;
q est égal à 0 ou à un nombre entier 1 ou 2 ;
sous réserve que
(a) lorsque L représente une double liaison, R₁ ne représente pas un groupe hétéroaryle penta- ou hexagonal facultativement substitué, le groupe hétéroaryle pentagonal contenant au moins un hétéroatome choisi entre l'oxygène, le soufre et l'azote et le groupe hétéroaryle hexagonal contenant 1 à 3 atomes d'azote ;
(b) le groupe R₁ est lié à l'atome de carbone désigné par le signe * par l'intermédiaire d'un atome de carbone ; et
(c) lorsque l'hétéroatome présent dans le groupe R₁ est substitué, p n'est pas égale à 0 ;
et ses sels et produits de solvatation pharmaceutiquement acceptables.

2. Composé suivant la revendication 1, dans lequel R représente un halogène (par exemple le fluor ou le chlore) et/ou un groupe alkyle en C₁ à C₄ (par exemple méthyle) et n représente le nombre entier 1 ou 2.

3. Composé suivant la revendication 1 ou la revendication 2, dans lequel dans lequel R₅ représente un groupe trifluorométhyle, méthyle, méthoxy ou un atome de brome, de chlore ou de fluor et m représente le nombre entier 1 ou 2.

4. Composé suivant l'une quelconque des revendications 1 à 3, dans lequel R₁ représente un groupe pipéridyle, morpholinyle, 1,2,3,6-tétrahydro-4-pyridinyle, pyridyle ou pyrrolidinyle.

5. Composé suivant l'une quelconque des revendications 1 à 4, dans lequel R représente un atome d'halogène (par exemple de fluor ou de chlore) et/ou un groupe alkyle en C₁ à C₄ (par exemple méthyle) et n représente le nombre entier 1 ou 2 ; R₁ représente un groupe pipéridyle, 2-morpholinyle, 1,2,3,6-tétrahydro-4-pyridinyle, pyridyle ou pyrrolidinyle, R₁ étant facultativement substitué avec un ou deux groupes choisis entre des groupes halogéno (par exemple fluoro), alkyle en C₁ à C₄ (par exemple méthyle) et éthyl (alkoxy en C₁ à C₄) ; R₂ et R₃ représentent indépendamment un atome d'hydrogène ou un groupe méthyle ; R₄ représente un atome d'hydrogène ou un groupe méthyle ou bien, conjointement avec R₃, représente un groupe cyclopropyle et R₅ représente un groupe trifluorométhyle, méthyle ou méthoxy ou un atome de brome, de chlore ou de fluor et m représente de préférence le nombre entier 1 ou 2.

6. Composé suivant la revendication 1, choisi entre :
*N*-(3,5-bis-trifluorométhyl-benzyl)-3-(4-fluoro-phényl)-*N*-méthyl-3-pipéridine-4-yl-propionamide ;
*N*-(3,5-dichloro-benzyl)-3-(4-fluoro-phényl)-*N-*méthyl-3-pipéridine-4-yl-propionamide ;
*N*-[1-(3,5-dichloro-phényl)-éthyl]-3-(4-fluoro-phényl)-*N-*méthyl-3-pipéridine-4-yl-propionamide ;
*N-*[1-(3,5-dichloro-phényl)-éthyl]-3-(4-fluoro-phényl)-*N-*méthyl-3-[1-(2-méthoxyéthyl)-pipéridine-4-yl]-propionamide;
*N-*(3,5-dichloro-benzyl)-3-(4-fluoro-phényl)-3-(4-fluoropipéridine-4-yl)-*N*-méthyl-propioanmide ;
*N*-{[3,5-bis(trifluorométhyl)phényl]méthyl}-3-(4-fluorophényl)-*N*-méthyl-3-{1-[2-(rnéthyloxy)éthyl]-4-pipéridinyl}propionamide-*N*-{-1-[3,5-bis(trifluorométhyl)phényl]éthyl}-3-(4-fluorophényl)-*N*-méthyl-3-(4-pipéridinyl)propanamide ;
*N*-{1-[3,5-bis(trifluorométhyl)phényl]-1-méthyléthyl}-3-(4-fluorophényl)-3-(4-pipéridinyl)propionamide ;
*N*-{[3-bromo-4-(méthyloxy)phényl]méthyl)-3-(4-fluorophényl)-*N*-méthyl-3-(4-pipéridinyl)propionamide ;
*N*-[(3,5-diméthylphényl)méthyl]-3-(4-fluorophényl)-*N*-méthyl-3-(4-pipéridinyl)propionamide ;
*N*-[(3,4-dibromophényl)méthyl]-3-(4-fluorophényl)-*N*-méthyl-3-(4-pipéridinyl)propionamide ;
*N*-[(3-fluoro-2-méthylphényl)méthyl]-3-(4-fluorophényl)-*N-*méthyl-3-(4-pipéridinyl)propionamide ;
*N*-([2-chloro-3-(trifluorométhyl)phényl]méthyl)-3-(4-fluorophényl)-*N*-méthyl-3-(4-pipéridinyl)propionamide ;
*N*-(1-[3,5-bis(trifluorométhyl)phényl]éthyl)-3-(4-fluorophényl)-3-(4-fluoro-4-pipéridinyl)-*N*-méthylpropionamide ;
*N*-[(3,5-dibromophényl)méthyl]-3-(4-fluorophényl)-3-(4-fluoro-4-pipéridinyl)-*N*-méthylpropionamide ;
*N*-(-1-[3,5-bis(trifluorométhyl)phényl]éthyl}-3-(2,4-dichlorophényl)-3-(4-fluoro-4-pipéridinyl)-*N*-méthylpropionamide ;
*N*-{-1-[3,5-bis(trifluorométhyl)phényl]éthyl}-3-(4-fluoro-2-methylphényl)-3-(4-fluoro-4-pipéridinyl)-*N*-méthylpropionamide ;
*N*-[(3,5-dibromophényl)méthyl]-3-(4-fluoro-2-méthylphényl)-3-(4-fluoro-4-pipéridinyl)-*N*-méthylpropionamide ;
*N*-[(3,5-dibromophényl)méthyl]-3-(3,4-dichlorophényl)-3-(4-fluoro-4-pipéridinyl)-*N*-méthylpropionamide ;
*N*-{[3,5-bis(trifluorométhyl)phényl]méthyl}-3-(4-fluorophényl)-3-(4-fluoro-4-pipéridinyl)-*N*-méthylpropionamide ;
3-(4-chlorophényl)-*N*-[(3,5-dibromophényl)méthyl]-3-(4-fluoro-4-pipéridinyl)-*N*-méthylpropionamide ;
*N*-{[3,5-bis(trifluorométhyl)phényl]méthyl}-3-(4-fluorophényl)-*N*-méthyl-3-(3-pipéridinylidène)propionamide ;
*N*-[(3,5-dibromophényl)méthyl]-3-(4-fluorophényl)-*N*-méthyl-3-(4-pipéridinylidène)propionamide ;
*N*-{[3,5-bis(trifluorométhyl)phényl]méthyl}-3-(4-fluoro-2-méthylphényl)-*N*-méthyl-3-(1,2,3,6-tétrahydro-4-pyridinyl)-propionamide ;
*N*-{(1*R*)-1-[3,5-bis(trifluorométhyl)phényl]éthyl}-3-(4-fluoro-2-méthylphényl)-*N*-méthyl-3-(1,2,3,6-tétrahydro-4-pyridinyl)-propionamide ;
*N*-{[3,5-bis(trifluorométhyl)phényl]méthyl}-3-(4-fluorophényl)-*N*-méthyl-3-(3-pyrrolidinyl)propionamide ;
*N*-([3,5-bis(trifluorométhyl)phényl]méthyl}-3-(4-fluorophényl)-3-(3-fluoro-3-pipéridinyl)-*N*-méthylpropionamide ;
*N*-{-1-[3,5-bis(trifluorométhyl)phényl]éthyl}-3-(4-fluorophényl)-3-(4-fluorophényl)-*N*-méthyl-3-(2-morpholinyl)propionamide ;
*N*-{[3,5-bis(trifluorométhyl)phényl]méthyl}-3-(4-fluorophényl)-*N*-méthyl-3-(3-pipéridinyl)propionamide ;
*N*-{[3,5-bis(trifluorométhyl)phényl]méthyl}-3-(4-fluorophényl)-*N*-méthyl-3-(4-pyridinyl)propionamide ;
et leurs énantiomères, diastéréoisomères, sels pharmaceutiquement acceptables (par exemple le chlorhydrate) et produits de solvatation.

7. Composé suivant la revendication 1, choisi entre :
*N*-{(1*R*)-1-[3,5-bis(trifluorométhyl)phényl]éthyl}-3-(4-fluorophényl)-*N*-méthyl-3-(4-pipéridinyl)propionamide (diastéréoisomère 1) ;
*N*-{(1*S*)-1-[3,5-bis (trifluorométhyl)phényl] éthyl}-3-(4-fluorophényl)-*N*-méthyl-3-(4-pipéridinyl)propionamide (diastéréoisomère 2) ;
*N*-{(1*R*)-1-[3,5-bis(trifluorométhyl)phényl]éthyl}-3-(4-fluorophényl)-3-(4-fluoro-4-pipéridinyl))-*N*-méthylpropionamide (diastéréoisomère 1);
*N*-[(3,5-dibromophényl)méthyl]-3-(4-fluorophényl)-3-(4-fluoro-4-pipéridinyl))-*N*-méthylpropionamide (énéntiomère 2) ;
*N*-{[3,5-bis (trifluorométhyl)phényl]méthyl}-3-(4-fluorophényl)-3-(3-fluoro-3-pipéridinyl))-*N*-méthylpropionamide (diastéréoisomère A) ;
et leurs sels pharmaceutiquement acceptables (par exemple le chlorhydrate) et leurs produits de solvatation.

8. Procédé pour la préparation d'un composé suivant la revendication 1, qui comprend la réaction d'un dérivé activé de l'acide carboxylique de formule (II) dans lequel R₁ répond à la définition précitée ou représente un groupe protégé correspondant, avec l'amine (III) 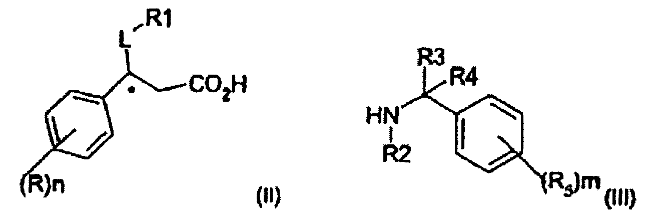 dans laquelle R₂ représente un groupe alkyle en C₁ à C₄ ou un groupe protecteur de l'atome d'azote, avec ensuite si nécessaire l'élimination de n'importe quel groupe protecteur.

9. Composé suivant l'une quelconque des revendications 1 à 7, destiné à être utilisé en thérapeutique.

10. Utilisation d'un composé suivant l'une quelconque des revendications 1 à 7 dans la préparation d'un médicament destiné au traitement d'affections à médiation par des tachykinines (comprenant la substance P et d'autres neurokinines) et/ou par l'inhibition sélective de la protéine transporteur de réabsorption de sérotonine.

11. Composition pharmaceutique comprenant un composé suivant l'une quelconque des revendications 1 à 7 en mélange avec un ou plusieurs supports ou excipients pharmaceutiquement acceptables.
